# EUROPEAN PATENT APPLICATION

(11) **EP 2 345 328 A1**
(43) Date of publication of application: **20.07.2011**
(21) Application number: 09814713.5
(22) Date of filing: 17.09.2009
(51) Int. Cl.: A01N 43/80, A01N 43/56, A01N 43/647, A01N 43/713, A01N 43/836, A01P 3/00, C07D 401/04, C07D 413/04

(54) **COMPOSITION FOR AGRICULTURAL USE**

(30) Priority: 19.09.2008 JP 2008240755; 03.10.2008 JP 2008258229
(71) Applicant: Sumitomo Chemical Company, Limited, Tokyo 104-8260 (JP)
(72) Inventor: MATSUZAKI, Yuichi, Toyonaka-shi Osaka 560-0021 (JP); SAKAGUCHI, Hiroshi, Toyonaka-shi Osaka 561-0802 (JP); UJITA, Satoru, Itami-shi Hyogo 664-0874 (JP)
(74) Representative: UEXKÜLL & STOLBERG
(86) International application number: PCT/JP2009/066838
(87) International publication number: WO 2010/032874

(57) **Abstract**

Provided is an agricultural composition comprising a compound represented by the formula (I) or a salt thereof or a hydrate thereof, for controlling a plant disease due to a plant pathogen: 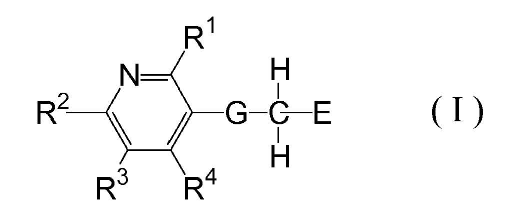 [wherein, G represents a di-valent aromatic hetero 5-membered ring optionally having a halogen atom or C1 to 3 alkyl group (with the proviso that a hetero atom of the aromatic hetero 5-membered ring is a nitrogen atom, oxygen atom or sulfur atom), R¹ represents a hydrogen atom or amino group, R², R³ and R⁴ represent each independently a hydrogen atom or the like, and E represents an optionally substituted 5 to 10-membered heterocyclic group or C6-10 aryl group.].

## Description

### Technical Field

The present invention relates to an agricultural composition comprising a compound for controlling or preventing a plant disease due to a plant pathogen or a salt of the compound or a hydrate thereof, a method for controlling or preventing a plant disease due to a plant pathogen comprising applying an effective amount of the agricultural composition to a useful crop, and so on.

### Background Art

Conventionally, agricultural compositions for controlling plant diseases are known, and there is a constant desire for a more highly active agricultural composition for plant disease control. For example, amide compounds are known described in international publication WO 2007/052615 and international publication WO 2008/035726.

### Disclosure of the Invention

The present invention has an object of providing an agricultural composition for controlling or preventing a plant disease due to a plant pathogen, a method for controlling or preventing a plant disease due to a plant pathogen comprising applying an effective amount of the agricultural composition to a useful crop, and so on.

The present invention discloses that a compound represented by the following formula (I) has an excellent effect for controlling or preventing a plant disease due to a plant pathogen. 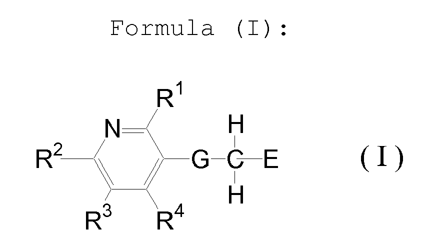 [wherein,
G represents a di-valent aromatic hetero 5-membered ring optionally having a halogen atom or a C1-3 alkyl group (with the proviso that a hetero atom of the aromatic hetero 5-membered ring is a nitrogen atom, an oxygen atom or a sulfur atom);
R¹ represents a hydrogen atom or an amino group;
R², R³ and R⁴ represent each independently a hydrogen atom or a member selected from the following substituent group a-1 and substituent group a-2;
E represents a 5 to 10-membered heterocyclic group optionally having 1 to 5 members selected from the following substituent group a-1 and substituent group a-2 or a C6 to 10 aryl group optionally having 1 to 5 members selected from the following substituent group a-1 and substituent group a-2.]
[Substituent Group a-1]
   halogen atom, amino group, hydroxyl group, mercapto group, cyano group, formyl group, carboxyl group, C1-8 alkyl group, C2-9 alkenyl group, C2-9 alkynyl group, C3-8 cycloalkyl group, C6-10 aryl group, 5 to 10-membered heterocyclic group, C3-8 cycloalkyl C1-6 alkyl group, C3-8 cycloalkylidene C1-6 alkyl group, C6-10 aryl C1-6 alkyl group, C6-10 aryl C2-6 alkenyl group, 5 to 10-membered heterocyclic C1-6 alkyl group, C1-8 alkoxy group, C3-9 alkenyloxy group, C3-9 alkynyloxy group, C3-8 cycloalkoxy group, C6-10 aryloxy group, 5 to 10-membered heterocyclic oxy group, C3-8 cycloalkyl C1-6 alkoxy group, C6-10 aryl C1-6 alkoxy group, 5 to 10-membered heterocyclic C1-6 alkoxy group, C1-8 alkylthio group, C3-9 alkenylthio group, C3-9 alkynylthio group, C3-8 cycloalkylthio group, C6-10 arylthio group, C3-8 cycloalkyl C1-6 alkylthio group, C6-10 aryl C1-6 alkylthio group, 5 to 10-membered heterocyclic C1-6 alkylthio group, mono-C1-8 alkylamino group, mono-C3-9 alkenylamino group, mono-C3-9 alkynylamino group, mono-C3-8 cycloalkylamino group, mono-C6-10 arylamino group, mono-C3-8 cycloalkyl C1-6 alkylamino group, mono-C6-10 aryl C1-6 alkylamino group, mono-5 to 10-membered heterocyclic C1-6 alkylamino group, di-C1-6 alkylamino group, N-C3-9 alkenyl-N-C1-8 alkylamino group, N-C3-9 alkynyl-N-C1-8 alkylamino group, N-C3-8 cycloalkyl-N-C1-8 alkylamino group, N-C6-10 aryl-N-C1-8 alkylamino group, N-C3-8 cycloalkyl C1-6 alkyl-N-C1-8 alkylamino group, N-C6-10 aryl C1-6 alkyl-N-C1-8 alkylamino group, N-5 to 10-membered heterocyclic C1-6 alkyl-N-C1-8 alkylamino group, C1-8 alkylcarbonyl group, C6-10 arylcarbonyl group, C1-8 alkylsulfonyl group, C1-8 alkylsulfinyl group, C6-10 aryloxy C1-6 alkyl group, 5 to 10-membered heterocyclic oxy C1-6 alkyl group and C1-3 trialkylsilyl C2-9 alkynyl group; and
[Substituent Group a-2]
   C1-8 alkyl group, C2-9 alkenyl group, C2-9 alkynyl group, C3-8 cycloalkyl group, C6-10 aryl group, 5 to 10-membered heterocyclic group, C3-8 cycloalkyl C1-6 alkyl group, C6-10 aryl C1-6 alkyl group, 5 to 10-membered heterocyclic C1-6 alkyl group, C1-8 alkoxy group, C3-9 alkenyloxy group, C3-9 alkynyloxy group, C3-8 cycloalkoxy group, C6-10 aryloxy group, 5 to 10-membered heterocyclic oxy group, C3-8 cycloalkyl C1-6 alkoxy group, C6-10 aryl C1-6 alkoxy group, 5 to 10-membered heterocyclic C1-6 alkoxy group, C1-8 alkylthio group, C3-9 alkenylthio group, C3-9 alkynylthio group, C3-8 cycloalkylthio group, C6-10 arylthio group, C3-8 cycloalkyl C1-6 alkylthio group, C6-10 aryl C1-6 alkylthio group, 5 to 10-membered heterocyclic C1-6 alkylthio group, mono-C1-8 alkylamino group, mono-C3-9 alkenylamino group, mono-C3-9 alkynylamino group, mono-C3-8 cycloalkylamino group, mono-C6-10 arylamino group, mono-C3-8 cycloalkyl C1-6 alkylamino group, mono-C6-10 aryl C1-6 alkylamino group, mono-5 to 10-membered heterocyclic C1-6 alkylamino group, di-C1-6 alkylamino group, N-C3-9 alkenyl-N-C1-8 alkylamino group, N-C3-9 alkynyl-N-C1-8 alkylamino group, N-C3-8 cycloalkyl-N-C1-8 alkylamino group, N-C6-10 aryl-N-C1-8 alkylamino group, N-C3-8 cycloalkyl C1-6 alkyl-N-C1-8 alkylamino group, N-C6-10 aryl C1-6 alkyl-N-C1-8 alkylamino group, N-5 to 10-membered heterocyclic C1-6 alkyl-N-C1-8 alkylamino group, C6-10 aryloxy C1-6 alkyl group and 5 to 10-membered heterocyclic oxy C1-6 alkyl group (with the proviso that each group in the substituent group a-2 has 1 to 3 members selected from the following substituent group b-1) :
[Substituent Group b-1]
   halogen atom, hydroxyl group, mercapto group, cyano group, amino group, nitro group, C1-6 alkyl group, C3-8 cycloalkyl group, C6-10 aryl group, 5 to 10-membered heterocyclic group, C1-6 alkoxy group, C3-8 cycloalkoxy group, C6-10 aryloxy group, C6-10 arylthio group, C6-10 arylamino group, 5 to 10-membered heterocyclic oxy group, C1-6 alkylthio group, C1-6 alkylsulfonyl group, monofluoromethyl group, difluoromethyl group, trifluoromethyl group, monofluoromethoxy group, difluoromethoxy group, trifluoromethoxy group, mono-C1-6 alkylamino group, di-C1-6 alkylamino group, mono-C6-10 arylamino group optionally having one amino group or aminosulfonyl group, and N-C6-10 aryl C1-6 alkyl-N-C1-6 alkylamino group optionally having one amino group.

Specifically, the present invention provides:
(1) an agricultural composition (hereinafter, referred to as the agricultural composition of the present invention in some cases) comprising a compound represented by the above formula (I) or a salt of the compound or a hydrate thereof (hereinafter, referred to as present compound in some cases), for controlling or preventing a plant disease due to a plant pathogen excluding Aspergillus;
(2) a method for controlling or preventing a plant disease due to a plant pathogen excluding Aspergillus comprising applying an effective amount of the agricultural composition of the present invention to a useful crop; and
(3) use of a compound represented by the formula (I) or a salt of the compound or a hydrate thereof, for producing the agricultural composition of the present invention;
and so on.

According to the present invention, an agricultural composition for controlling or preventing a plant disease due to a plant pathogen excluding Aspergillus; a method of controlling or preventing a plant disease due to a plant pathogen, comprising applying an effective amount of the agricultural composition to a useful crop; and so on can be provided.

### Modes for Carrying Out the Invention

G in the formula (I) representing the present compound is a di-valent aromatic hetero 5-membered ring optionally having a halogen atom or a C1-3 alkyl group (with the proviso that a hetero atom of the aromatic hetero 5-membered ring is a nitrogen atom, an oxygen atom or a sulfur atom), and examples thereof include an isoxazolediyl group optionally having one or two halogen atoms or C1-3 alkyl groups, an isothiazolediyl group optionally having one or two halogen atoms or C1-3 alkyl groups, a pyrazolediyl group optionally having one or two halogen atoms or C1-3 alkyl groups, a 1,2,3-triazolediyl group optionally having one or two halogen atoms or C1-3 alkyl groups, a 1,2,4- triazolediyl group optionally having one or two halogen atoms or C1-3 alkyl groups, a tetrazolediyl group optionally having one or two halogen atoms or C1-3 alkyl groups, a 1,3,4-oxadiazolediyl group optionally having one or two halogen atoms or C1-3 alkyl groups, a 1,3,4-thiadiazolediyl group optionally having one or two halogen atoms or C1-3 alkyl groups, a 1,2,4-oxadiazolediyl group optionally having one or two halogen atoms or C1-3 alkyl groups, or a 1,2,4-thiadiazolediyl group or optionally having one or two halogen atoms or C1-3 alkyl groups, and preferably, G is any of G¹ to G¹² (for G¹ to G¹¹ and G¹², its left connecting bond is connected to a pyridine ring and its right connecting bond is connected to a methylene group.).

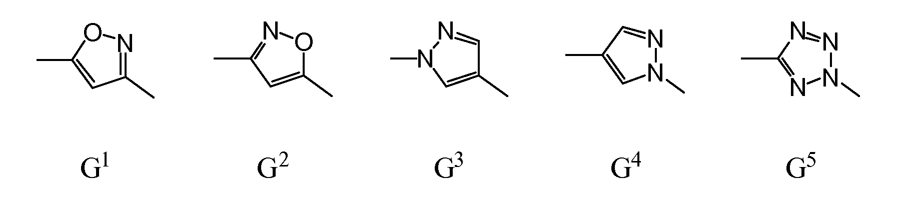

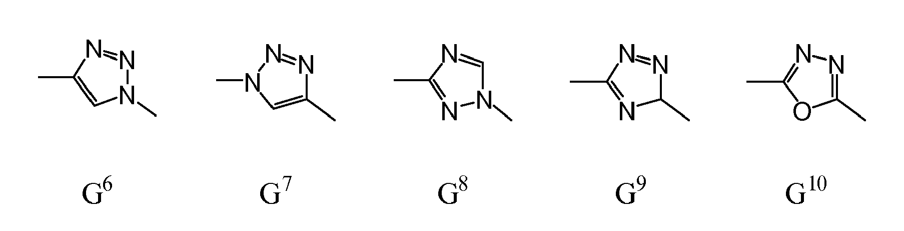

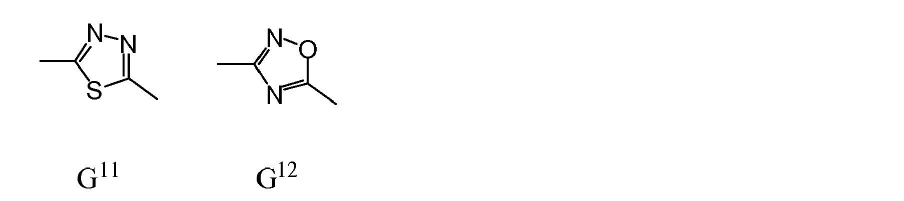

Each of R² , R³ and R⁴ in the formula (I) representing the present compound independently is a hydrogen atom, or a member selected from the above substituent group a-1 and substituent group a-2, and preferably, a hydrogen atom, or a member selected from the following substituent group c-1 and substituent group c-2 is listed.
[[Substituent Group c-1]
   halogen atom, amino group, C1-6 alkyl group, C2-6 alkenyl group, C2-6 alkynyl group, C3-8 cycloalkyl group, C6-10 aryl group, 5 to 10-membered heterocyclic group, C3-8 cycloalkyl C1-6 alkyl group, C6-10 aryl C1-6 alkyl group, C6-10 aryl C2-6 alkenyl group, 5 to 10-membered heterocyclic C1-6 alkyl group, C1-6 alkoxy group, C3-6 alkenyloxy group, C3-6 alkynyloxy group, C3-8 cycloalkyl C1-6 alkoxy group, C6-10 aryl C1-6 alkoxy group, 5 to 10-membered heterocyclic C1-6 alkoxy group, mono-C1-6 alkylamino group, mono-C3-6 alkenylamino group, mono-C3-6 alkynylamino group, mono-C3-8 cycloalkylamino group, mono-C1-6 arylamino group, mono-C6-10 cycloalkyl C1-6 alkylamino group, mono-C1-6 aryl C1-6 alkylamino group, mono-5 to 10-membered heterocyclic C1-6 alkylamino group, and C2-6 alkylcarbonyl group; and
[Substituent Group c-2]
   C1-6 alkyl group, C2-6 alkenyl group, C2-6 alkynyl group, C3-8 cycloalkyl group, C6-10 aryl group, 5 to 10-membered heterocyclic group, C3-8 cycloalkyl C1-6 alkyl group, C6-10 aryl C1-6 alkyl group, 5 to 10-membered heterocyclic C1-6 alkyl group, C1-6 alkoxy group, C3-6 alkenyloxy group, C3-6 alkynyloxy group, C3-8 cycloalkyl C1-6 alkoxy group, C6-10 aryl C1-6 alkoxy group, 5 to 10-membered heterocyclic C1-6 alkoxy group, mono-C1-6 alkylamino group, mono-C3-6 alkenylamino group, mono-C3-6 alkynylamino group, mono-C3-8 cycloalkylamino group, mono-C6-10 arylamino group, mono-C3-8 cycloalkyl C1-6 alkylamino group, mono-C6-10 aryl C1-6 alkylamino group and mono-5 to 10-membered heterocyclic C1-6 alkylamino group (with the proviso that each group in the substituent group c-2 has 1 to 3 members selected from the following substituent group d-1.):
[Substituent Group d-1]
   halogen atom, hydroxyl group, carboxyl group, amino group, carbamoyl group, C1-6 alkyl group, C1-6 alkoxy group, C1-6 alkylthio group, mono-C1-6 alkylamino group, di-C1-6 alkylamino group, mono-C6-10 arylamino group optionally having one amino group or aminosulfonyl group, N-C6-10 aryl C1-6 alkyl-N-C1-6 alkylamino group optionally having one amino group; cyano group, C6-10 aryl group and 5 to 10-membered heterocyclic group] .

Further preferably, a hydrogen atom, or a member selected from the following substituent group c-3 and substituent group c-4 is listed.
[[Substituent Group c-3]
   halogen atom, amino group, C1-6 alkyl group, C2-6 alkenyl group, C2-6 alkynyl group, C3-8 cycloalkyl group, C3-8 cycloalkyl C1-6 alkyl group, C1-6 alkoxy group, C3-6 alkenyloxy group, C3-6 alkynyloxy group, C3-8 cycloalkyl C1-6 alkoxy group, mono-C1-6 alkylamino group, mono-C3-6 alkenylamino group, mono-C3-6 alkynylamino group, mono-C3-8 cycloalkylamino group, mono-C3-8 cycloalkyl C1-6 alkylamino group; and
[Substituent Group c-4]
   C1-6 alkyl group, C2-6 alkenyl group, C2-6 alkynyl group, C3-8 cycloalkyl group, C3-8 cycloalkyl C1-6 alkyl group, C1-6 alkoxy group, C3-6 alkenyloxy group, C3-6 alkynyloxy group, C3-8 cycloalkyl C1-6 alkoxy group, mono-C1-6 alkylamino group, mono-C3-6 alkenylamino group, mono-C3-6 alkynylamino group, mono-C3-8 cycloalkylamino group, mono-C3-8 cycloalkyl C1-6 alkylamino group (with the proviso that each group in the substituent group c-4 has 1 to 3 members selected from the following substituent group d-2):
[Substituent Group d-2]
   halogen atom, hydroxyl group, carboxyl group, amino group, carbamoyl group, C1-6 alkyl group, C1-6 alkoxy group, C1-6 alkylthio group, mono-C1-6 alkylamino group, di-C1-6 alkylamino group and cyano group].

Further preferably, a hydrogen atom, or a member selected from the following substituent group c-5 and substituent group c-6 is listed.
[[Substituent Group c-5]
   halogen atom, amino group, C1-6 alkyl group, C1-6 alkoxy group, mono-C1-6 alkylamino group, and di-C1-6 alkylamino group; and
[Substituent Group c-6]
   C1-6 alkyl group, C1-6 alkoxy group, and mono-C1-6 alkylamino group (with the proviso that each group in the substituent group C-6 has 1 to 3 members selected from the following substituent group d-3):
[Substituent Group d-3]
   halogen atom, C1-6 alkoxy group, C1-6 alkylthio group].

Further preferably, examples of R² include a hydrogen atom, halogen atom (particularly, fluorine atom, chlorine atom), amino group, mono-C1-3 alkylamino group, C1-3 alkyl group, C1-3 alkoxy group and C1-3 alkoxy C1-3 alkyl group, examples of R³ include a hydrogen atom and halogen atom (particularly, fluorine atom, chlorine atom), and examples of R⁴ include a hydrogen atom.

Combinations of R¹ , R² , R³ and R⁴ in the compound represented by the formula (I) include preferably combinations in which R¹ is a hydrogen atom or amino group, R² is a hydrogen atom, or a group selected from the substituent group c-21 and substituent group c-22, R³ is a hydrogen atom or halogen atom, and R⁴ is a hydrogen atom, further preferably a combination in which R¹ is an amino group, and all of R², R³ and R⁴ represent a hydrogen atom.

E in the formula (I) representing the present compound is a 5 to 10-membered heterocyclic group or C6-10 aryl group optionally having 1 to 5 members selected from the following substituent group e-1 and substituent group e-2.
[Substituent Group e-1]
   halogen atom, amino group, hydroxyl group, mercapto group, cyano group, formyl group, carboxyl group, C1-8 alkyl group, C2-9 alkenyl group, C2-9 alkynyl group, C3-8 cycloalkyl group, C6-10 aryl group, 5 to 10-membered heterocyclic group, C3-8 cycloalkyl C1-6 alkyl group, C3-8 cycloalkylidene C1-6 alkyl group, C6-10 aryl C1-6 alkyl group, C6-10 aryl C2-6 alkenyl group, 5 to 10-membered heterocyclic C1-6 alkyl group, C1-8 alkoxy group, C3-9 alkenyloxy group, C3-9 alkynyloxy group, C3-8 cycloalkoxy group, C6-10 aryloxy group, 5 to 10-membered heterocyclic oxy group, C3-8 cycloalkyl C1-6 alkoxy group, C6-10 aryl C1-6 alkoxy group, 5 to 10-membered heterocyclic C1-6 alkoxy group, C1-8 alkylthio group, C3-9 alkenylthio group, C3-9 alkynylthio group, C3-8 cycloalkylthio group, C6-10 arylthio group, C3-8 cycloalkyl C1-6 alkylthio group, C6-10 aryl C1-6 alkylthio group, 5 to 10-membered heterocyclic C1-6 alkylthio group, mono-C1-8 alkylamino group, mono-C3-9 alkenylamino group, mono-C3-9 alkynylamino group, mono-C3-8 cycloalkylamino group, mono-C6-10 arylamino group, mono-C3-8 cycloalkyl C1-6 alkylamino group, mono-C6-10 aryl C1-6 alkylamino group, mono-5 to 10-membered heterocyclic C1-6 alkylamino group, di-C1-6 alkylamino group, N-C3-9 alkenyl-N-C1-8 alkylamino group, N-C3-9 alkynyl-N-C1-8 alkylamino group, N-C3-8 cycloalkyl-N-C1-8 alkylamino group, N-C6-10 aryl-N-C1-8 alkylamino group, N-C3-8 cycloalkyl C1-6 alkyl-N-C1-8 alkylamino group, N-C6-10 aryl C1-6 alkyl-N-C1-8 alkylamino group, N-5 to 10-membered heterocyclic C1-6 alkyl-N-C1-8 alkylamino group, C1-8 alkylcarbonyl group, C6-10 aryl Carbonyl group, C1-8 alkylsulfonyl group, C1-8 alkylsulfinyl group, C6-10 aryloxy C1-6 alkyl group, 5 to 10-membered heterocyclic oxy C1-6 alkyl group and C1-3 trialkylsilyl C2-9 alkynyl group; and
[Substituent Group e-2]
   C1-8 alkyl group, C2-9 alkenyl group, C2-9 alkynyl group, C3-8 cycloalkyl group, C6-10 aryl group, 5 to 10-membered heterocyclic group, C3-8 cycloalkyl C1-6 alkyl group, C6-10 aryl C1-6 alkyl group, 5 to 10-membered heterocyclic C1-6 alkyl group, C1-8 alkoxy group, C3-9 alkenyloxy group, C3-9 alkynyloxy group, C3-8 cycloalkoxy group, C6-10 aryloxy group, 5 to 10-membered heterocyclic oxy group, C3-8 cycloalkyl C1-6 alkoxy group, C6-10 aryl C1-6 alkoxy group, 5 to 10-membered heterocyclic C1-6 alkoxy group, C1-8 alkylthio group, C3-9 alkenylthio group, C3-9 alkynylthio group, C3-8 cycloalkylthio group, C6-10 arylthio group, C3-8 cycloalkyl C1-6 alkylthio group, C6-10 aryl C1-6 alkylthio group, 5 to 10-membered heterocyclic C1-6 alkylthio group, mono-C1-8 alkylamino group, mono-C3-9 alkenylamino group, mono-C3-9 alkynylamino group, mono-C3-8 cycloalkylamino group, mono-C6-10 arylamino group, mono-C3-8 cycloalkyl C1-6 alkylamino group, mono-C6-10 aryl C1-6 alkylamino group, mono-5 to 10-membered heterocyclic C1-6 alkylamino group, di-C1-6 alkylamino group, N-C3-9 alkenyl-N-C1-8 alkylamino group, N-C3-9 alkynyl-N-C1-8 alkylamino group, N-C3-8 cycloalkyl-N-C1-8 alkylamino group, N-C6-10 aryl-N-C1-8 alkylamino group, N-C3-8 cycloalkyl C1-6 alkyl-N-C1-8 alkylamino group, N-C6-10 aryl C1-6 alkyl-N-C1-8 alkylamino group, N-5 to 10-membered heterocyclic C1-6 alkyl-N-C1-8 alkylamino group, C6-10 aryloxy C1-6 alkyl group and 5 to 10-membered heterocyclic oxy C1-6 alkyl group (with the proviso that each group in the substituent group e-2 has 1 to 3 members selected from the following substituent group f-1):
[Substituent Group f-1]
   halogen atom, hydroxyl group, mercapto group, cyano group, amino group, nitro group, C1-6 alkyl group, C3-8 cycloalkyl group, C6-10 aryl group, 5 to 10-membered heterocyclic group, C1-6 alkoxy group, C6-10 aryloxy group, 5 to 10-membered heterocyclic oxy group, C1-6 alkylthio group, C1-6 alkylsulfonyl group, monofluoromethyl group, difluoromethyl group, trifluoromethyl group, monofluoromethoxy group, difluoromethoxy group, trifluoromethoxy group, mono-C1-6 alkylamino group, di-C1-6 alkylamino group, mono-C6-10 arylamino group optionally having one amino group or aminosulfonyl group, and N-C6-10 aryl C1-6 alkyl-N-C1-6 alkylamino group optionally having one amino group].

Examples of E include preferably a furyl group optionally having 1 or 2 identical or mutually different members selected from the following substituent group e-3 and substituent group e-4, a thienyl group optionally having 1 or 2 identical or mutually different members selected from the substituent group e-3 and substituent group e-4, a pyrrolyl group optionally having 1 or 2 identical or mutually different members selected from the substituent group e-3 and substituent group e-4, a tetrazoyl group optionally having one identical or mutually different member selected from the substituent group e-3 and substituent group e-4, a thiazolyl group optionally having 1 or 2 identical or mutually different members selected from the substituent group e-3 and substituent group e-4, a pyrazolyl group optionally having 1 or 2 identical or mutually different members selected from the substituent group e-3 and substituent group e-4, a pyridyl group optionally having 1 or 4 identical or mutually different members selected from the substituent group e-3 and substituent group e-4 and a phenyl group optionally having 1 or 5 identical or mutually different members selected from the substituent group e-3 and substituent group e-4.
[Substituent Group e-3]
   halogen atom, amino group, hydroxyl group, mercapto group, cyano group, formyl group, carboxyl group, C1-8 alkyl group, C2-9 alkenyl group, C2-9 alkynyl group, C3-8 cycloalkyl group, C6-10 aryl group, 5 to 10-membered heterocyclic group, C3-8 cycloalkyl C1-6 alkyl group, C3-8 cycloalkylidene C1-6 alkyl group, C6-10 aryl C1-6 alkyl group, C6-10 aryl C2-6 alkenyl group, 5 to 10-membered heterocyclic C1-6 alkyl group, C1-8 alkoxy group, C3-9 alkenyloxy group, C3-9 alkynyloxy group, C3-8 cycloalkoxy group, C6-10 aryloxy group, 5 to 10-membered heterocyclic oxy group, C3-8 cycloalkyl C1-6 alkoxy group, C6-10 aryl C1-6 alkoxy group, 5 to 10-membered heterocyclic C1-6 alkoxy group, C1-8 alkylthio group, C3-9 alkenylthio group, C3-9 alkynylthio group, C3-8 cycloalkylthio group, C6-10 arylthio group, C3-8 cycloalkyl C1-6 alkylthio group, C6-10 aryl C1-6 alkylthio group, 5 to 10-membered heterocyclic C1-6 alkylthio group, mono-C1-8 alkylamino group, mono-C3-9 alkenylamino group, mono-C3-9 alkynylamino group, mono-C3-8 cycloalkylamino group, mono-C6-10 arylamino group, mono-C3-8 cycloalkyl C1-6 alkylamino group, mono-C6-10 aryl C1-6 alkylamino group, mono-5 to 10-membered heterocyclic C1-6 alkylamino group, di-C1-6 alkylamino group, N-C3-9 alkenyl-N-C1-8 alkylamino group, N-C3-9 alkynyl-N-C1-8 alkylamino group, N-C3-8 cycloalkyl-N-C1-8 alkylamino group, N-C6-10 aryl-N-C1-8 alkylamino group, N-C3-8 cycloalkyl C1-6 alkyl-N-C1-8 alkylamino group, N-C6-10 aryl C1-6 alkyl-N-C1-8 alkylamino group, N-5 to 10-membered heterocyclic C1-6 alkyl-N-C1-8 alkylamino group, C1-8 alkylcarbonyl group, C6-10 arylcarbonyl group, C1-8 alkylsulfonyl group, C1-8 alkylsulfinyl group, C6-10 aryloxy C1-6 alkyl group, 5 to 10-membered heterocyclic oxy C1-6 alkyl group and C1-3 trialkylsilyl C2-9 alkynyl group; and
[Substituent Group e-4]
   C1-8 alkyl group, C2-9 alkenyl group, C2-9 alkynyl group, C3-8 cycloalkyl group, C6-10 aryl group, 5 to 10-membered heterocyclic group, C3-8 cycloalkyl C1-6 alkyl group, C6-10 aryl C1-6 alkyl group, 5 to 10-membered heterocyclic C1-6 alkyl group, C1-8 alkoxy group, C3-9 alkenyloxy group, C3-9 alkynyloxy group, C3-8 cycloalkoxy group, C6-10 aryloxy group, 5 to 10-membered heterocyclic oxy group, C3-8 cycloalkyl C1-6 alkoxy group, C6-10 aryl C1-6 alkoxy group, 5 to 10-membered heterocyclic C1-6 alkoxy group, C1-8 alkylthio group, C3-9 alkenylthio group, C3-9 alkynylthio group, C3-8 cycloalkylthio group, C6-10 arylthio group, C3-8 cycloalkyl C1-6 alkylthio group , C6-10 aryl C1-6 alkylthio group, 5 to 10-membered heterocyclic C1-6 alkylthio group, mono-C1-8 alkylamino group, mono-C3-9 alkenylamino group, mono-C3-9 alkynylamino group, mono-C3-8 cycloalkylamino group, mono-C6-10 arylamino group, mono-C3-8 cycloalkyl C1-6 alkylamino group, mono-C6-10 aryl C1-6 alkylamino group, mono-5 to 10-membered heterocyclic C1-6 alkylamino group, di-C1-6 alkylamino group, N-C3-9 alkenyl-N-C1-8 alkylamino group, N-C3-9 alkynyl-N-C1-8 alkylamino group, N-C3-8 cycloalkyl-N-C1-8 alkylamino group, N-C6-10 aryl-N-C1-8 alkylamino group, N-C3-8 cycloalkyl C1-6 alkyl-N-C1-8 alkylamino group, N-C6-10 aryl C1-6 alkyl-N-C1-8 alkylamino group, N-5 to 10-membered heterocyclic C1-6 alkyl-N-C1-8 alkylamino group, C6-10 aryloxy C1-6 alkyl group and 5 to 10-membered heterocyclic oxy C1-6 alkyl group (with the proviso that each group in the substituent group e-4 has 1 to 3 members selected from the following substituent group f-2):
[Substituent Group f-2]
   halogen atom, hydroxyl group, mercapto group, cyano group, amino group, nitro group, C1-6 alkyl group, C3-8 cycloalkyl group, C6-10 aryl group, 5 to 10-membered heterocyclic group, C1-6 alkoxy group, C6-10 aryloxy group, 5 to 10-membered heterocyclic oxy group, C1-6 alkylthio group, C1-6 alkylsulfonyl group, monofluoromethyl group, difluoromethyl group, trifluoromethyl group, monofluoromethoxy group, difluoromethoxy group, trifluoromethoxy group, mono-C1-6 alkylamino group, di-C1-6 alkylamino group, mono-C6-10 arylamino group optionally having one amino group or aminosulfonyl group and N-C6-10 aryl C1-6 alkyl-N-C1-6 alkylamino group optionally having one amino group.

Examples of E include, more preferably, a furyl group optionally having 1 or 2 identical or mutually different members selected from the following substituent group a-5, a thienyl group optionally having 1 or 2 identical or mutually different members selected from the substituent group a-5, a pyridyl group optionally having 1 or 4 identical or mutually different members selected from the substituent group a-5 and a phenyl group optionally having 1 or 5 identical or mutually different members selected from the substituent group a-5.
[Substituent Group a-5]
   halogen atom; cyano group; C1-8 alkyl group; C2-9 alkenyl group; C2-9 alkynyl group; C3-8 cycloalkyl group; phenyl group; 5 to 10-membered heterocyclic group; C3-8 cycloalkyl C1-6 alkyl group; C3-8 cycloalkylidene C1-6 alkyl group; phenyl C1-6 alkyl group; 5 to 10-membered heterocyclic C1-6 alkyl group; phenyl C2-6 alkenyl group; 5 to 10-membered heterocyclic C2-6 alkenyl group; C1-8 alkoxy group; C3-9 alkenyloxy group; C3-9 alkynyloxy group; C3-8 cycloalkoxy group; phenoxy group; 5 to 10-membered heterocyclic oxy group; C3-8 cycloalkyl C1-6 alkoxy group; C3-8 cycloalkylidene C1-6 alkoxy group; phenyl C1-6 alkoxy group; 5 to 10-membered heterocyclic C1-6 alkoxy group; phenoxy C1-6 alkyl group; 5 to 10-membered heterocyclic oxy C1-6 alkyl group; C1-3 trialkylsilyl C2-9 alkynyl group; C1-8 alkyl group substituted by a member selected from the group consisting of halogen atom, cyano group, C1-6 alkoxy group, monofluoromethyl group, difluoromethyl group, trifluoromethyl group, monofluoromethoxy group, difluoromethoxy group and trifluoromethoxy group; C2-9 alkenyl group substituted by a member selected from the group consisting of halogen atom, cyano group, C1-6 alkoxy group, monofluoromethyl group, difluoromethyl group, trifluoromethyl group, monofluoromethoxy group, difluoromethoxy group and trifluoromethoxy group; C2-9 alkynyl group substituted by a member selected from the group consisting of halogen atom, cyano group, C1-6 alkoxy group, monofluoromethyl group, difluoromethyl group, trifluoromethyl group, monofluoromethoxy group, difluoromethoxy group and trifluoromethoxy group; C3-8 cycloalkyl group substituted by a member selected from the group consisting of halogen atom, cyano group, C1-6 alkoxy group, monofluoromethyl group, difluoromethyl group, trifluoromethyl group, monofluoromethoxy group, difluoromethoxy group and trifluoromethoxy group; phenyl group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; 5 to 10-membered heterocyclic group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; C3-8 cycloalkyl C1-6 alkyl group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; C3-8 cycloalkylidene C1-6 alkyl group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; phenyl C1-6 alkyl group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; 5 to 10-membered heterocyclic C1-6 alkyl group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; phenyl C2-6 alkenyl group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; 5 to 10-membered heterocyclic C2-6 alkenyl group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; C1-8 alkoxy group substituted by a member selected from the group consisting of halogen atom, cyano group, C1-6 alkoxy group, monofluoromethyl group, difluoromethyl group, trifluoromethyl group, monofluoromethoxy group, difluoromethoxy group and trifluoromethoxy group; C3-9 alkenyloxy group substituted by a member selected from the group consisting of halogen atom, cyano group, C1-6 alkoxy group, monofluoromethyl group, difluoromethyl group, trifluoromethyl group, monofluoromethoxy group, difluoromethoxy group and trifluoromethoxy group; C3-9 alkynyloxy group substituted by a member selected from the group consisting of halogen atom, cyano group, C1-6 alkoxy group, monofluoromethyl group, difluoromethyl group, trifluoromethyl group, monofluoromethoxy group, difluoromethoxy group and trifluoromethoxy group; C3-8 cycloalkoxy group substituted by a member selected from the group consisting of halogen atom, cyano group, C1-6 alkoxy group, monofluoromethyl group, difluoromethyl group, trifluoromethyl group, monofluoromethoxy group, difluoromethoxy group and trifluoromethoxy group; phenoxy group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; 5 to 10-membered heterocyclic oxy group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; C3-8 cycloalkyl C1-6 alkoxy group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; C3-8 cycloalkylidene C1-6 alkoxy group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; phenyl C1-6 alkoxy group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; 5 to 10-membered heterocyclic C1-6 alkoxy group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; phenoxy C1-6 alkyl group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; and 5 to 10-membered heterocyclic oxy C1-6 alkyl group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group.

Further preferably, examples of E include a furyl group optionally having 1 or 2 members selected from the following substituent group e-7 and substituent group e-8, a thienyl group optionally having 1 or 2 members selected from the following substituent group e-7 and substituent group e-8, a pyridyl group optionally having 1 or 2 members selected from the following substituent group e-7 and substituent group e-8 and a phenyl group optionally having 1 or 2 members selected from the following substituent group e-7 and substituent group e-8.

Further preferably, examples of E include a furyl group optionally having one member selected from the following substituent group e-7 and substituent group e-8, a thienyl group optionally having one member selected from the following substituent group e-7 and substituent group e-8, a pyridyl group optionally having one member selected from the following substituent group e-7 and substituent group e-8 and a phenyl group optionally having one member selected from the following substituent group e-7 and substituent group e-8.
[[Substituent Group e-7]
   C1-6 alkyl group, C2-6 alkenyl group, C2-6 alkynyl group, C3-8 cycloalkyl C1-6 alkyl group, phenyl C1-6 alkyl group, furyl C1-6 alkyl group, thienyl C1-6 alkyl group, benzofuryl C1-6 alkyl group, benzothienyl C1-6 alkyl group, C1-6 alkoxy group, C3-6 alkenyloxy group, C3-6 alkynyloxy group, phenoxy group, C3-8 cycloalkyl C1-6 alkoxy group, phenyl C1-6 alkoxy group, furyl C1-6 alkoxy group, thienyl C1-6 alkoxy group, pyridyl C1-6 alkoxy group, phenoxy C1-6 alkyl group and pyridyloxy C1-6 alkyl group; and
[Substituent Group e-8]
   C1-6 alkyl group, C2-6 alkenyl group, C2-6 alkynyl group, C3-8 cycloalkyl C1-6 alkyl group, phenyl C1-6 alkyl group, furyl C1-6 alkyl group, thienyl C1-6 alkyl group, benzofuryl C1-6 alkyl group, benzothienyl C1-6 alkyl group, C1-6 alkoxy group, C1-6 alkoxy group, C3-6 alkenyloxy group, C3-6 alkynyloxy group, phenoxy group, C3-8 cycloalkyl C1-6 alkoxy group, phenyl C1-6 alkoxy group, furyl C1-6 alkoxy group, thienyl C1-6 alkoxy group, pyridyl C1-6 alkoxy group, phenoxy C1-6 alkyl group and pyridyloxy C1-6 alkyl group (with the proviso that each group in the substituent group e-8 has 1 to 3 members selected from the following substituent group f-4):
[Substituent Group f-4]
   halogen atom, hydroxyl group, cyano group, monofluoromethyl group, difluoromethyl group, trifluoromethyl group, monofluoromethoxy group, difluoromethoxy group, trifluoromethoxy group, C1-6 alkyl group, C1-6 alkoxy group and C1-6 alkylthio group].

Further preferably, examples of E include a 2-furyl group, 2-thienyl group, pyridyl group and phenyl group of which hydrogen atom is optionally mono- to di-substituted (especially preferably, of which hydrogen atom is optionally mono-substituted) by
[fluorine atom, chlorine atom, C1-6 alkyl group (optionally mono- to tri-substituted by halogen atom, C3-5 cycloalkyl group, C1-6 alkoxy group, phenoxy group, 5 to 6-membered heterocyclic oxy group or C1-6 alkyl group), C2-6 alkenyl group (optionally mono- to tri-substituted by halogen atom, C3-5 cycloalkyl group, C1-6 alkoxy group, phenoxy group, 5 to 6-membered heterocyclic oxy group or C1-6 alkyl group), C2-6 alkynyl group (optionally mono- to tri-substituted by halogen atom, C3-5 cycloalkyl group, C1-6 alkoxy group, phenoxy group, 5 to 6-membered heterocyclic oxy group or C1-6 alkyl group), phenyl group (optionally mono- to tri-substituted by halogen atom, C3-5 cycloalkyl group, C1-6 alkoxy group or C1-6 alkyl group), C3-5 cycloalkyl C1-6 alkyl group (optionally mono- to tri-substituted by halogen atom or C1-6 alkyl group), C3-5 cycloalkylidene C1-6 alkyl group (optionally mono- to tri-substituted by halogen atom or C1-6 alkyl group), phenyl C1-6 alkyl group (optionally mono- to tri-substituted by halogen atom, C3-5 cycloalkyl group, C1-6 alkoxy group, phenoxy group, 5 to 6-membered heterocyclic oxy group or C1-6 alkyl group), phenyl C2-6 alkenyl group (optionally mono- to tri-substituted by halogen atom, C3-5 cycloalkyl group, C1-6 alkoxy group, phenoxy group, 5 to 6-membered heterocyclic oxy group or C1-6 alkyl group), 5 to 6-membered heterocyclic C1-6 alkyl group (optionally mono- to tri-substituted by halogen atom, C3-5 cycloalkyl group, C1-6 alkoxy group, phenoxy group, 5 to 6-membered heterocyclic oxy group or C1-6 alkyl group), C1-6 alkoxy group (optionally mono- to tri-substituted by halogen atom or C1-6 alkyl group), C3-6 alkenyloxy group (optionally mono- to tri-substituted by halogen atom or C1-6 alkyl group), C3-6 alkynyloxy group (optionally mono- to tri-substituted by halogen atom or C1-6 alkyl group), phenoxy group (optionally mono- to tri-substituted by halogen atom, C3-5 cycloalkyl group, C1-6 alkoxy group, phenoxy group, 5 to 6-membered heterocyclic oxy group or C1-6 alkyl group), C3-6 cycloalkyl C1-6 alkoxy group optionally mono- to tri-substituted by halogen atom, phenyl C1-6 alkoxy group (optionally mono- to tri-substituted by halogen atom, C3-5 cycloalkyl group, C1-6 alkoxy group, phenoxy group, 5 to 6-membered heterocyclic oxy group or C1-6 alkyl group), 5 to 6-membered heterocyclic C1-6 alkoxy group (optionally mono- to tri-substituted by halogen atom, C3-5 cycloalkyl group, C1-6 alkoxy group, phenoxy group, 5 to 6-membered heterocyclic oxy group or C1-6 alkyl group), phenoxy C1-6 alkyl group (optionally mono- to tri-substituted by halogen atom, C3-5 cycloalkyl group, C1-6 alkoxy group, phenoxy group or 5 to 6-membered heterocyclic oxy group) and 5 to 6-membered heterocyclic oxy C1-6 alkyl group (optionally mono- to tri-substituted by halogen atom, C3-5 cycloalkyl group, or C1-6 alkoxy group)].

Particularly preferably, examples of E include a 2-furyl group, 2-thienyl group, pyridyl group and phenyl group of which hydrogen atom is optionally mono- to di-substituted (especially preferably, of which hydrogen atom is optionally mono-substituted) by [fluorine atom, chlorine atom, C1-6 alkyl group (optionally mono- to tri-substituted by halogen atom, C3-5 cycloalkyl group, C1-6 alkoxy group, phenoxy group, 5 to 6-membered heterocyclic oxy group or C1-6 alkyl group), C2-6 alkenyl group (optionally mono- to tri-substituted by halogen atom, C3-5 cycloalkyl group, C1-6 alkoxy group, phenoxy group, 5 to 6-membered heterocyclic oxy group or C1-6 alkyl group), C2-6 alkynyl group (optionally mono- to tri-substituted by halogen atom, C3-5 cycloalkyl group, C1-6 alkoxy group, phenoxy group, 5 to 6-membered heterocyclic oxy group or C1-6 alkyl group), phenyl group (optionally mono- to tri-substituted by halogen atom, C3-5 cycloalkyl group, C1-6 alkoxy group or C1-6 alkyl group), C3-5 cycloalkyl C1-6 alkyl group (optionally mono- to tri-substituted by halogen atom or C1-6 alkyl group), C3-5 cycloalkylidene C1-6 alkyl group (optionally mono- to tri-substituted by halogen atom or C1-6 alkyl group), phenyl C1-6 alkyl group (optionally mono- to tri-substituted by halogen atom, C3-5 cycloalkyl group, C1-6 alkoxy group, phenoxy group, 5 to 6-membered heterocyclic oxy group or C1-6 alkyl group), phenyl C2-6 alkenyl group (optionally mono- to tri-substituted by halogen atom, C3-5 cycloalkyl group, C1-6 alkoxy group, phenoxy group, 5 to 6-membered heterocyclic oxy group or C1-6 alkyl group), 5 to 6-membered heterocyclic C1-6 alkyl group (optionally mono- to tri-substituted by halogen atom, C3-5 cycloalkyl group, C1-6 alkoxy group, phenoxy group, 5 to 6-membered heterocyclic oxy group or C1-6 alkyl group), C1-6 alkoxy group (optionally mono- to tri-substituted by halogen atom or C1-6 alkyl group), C3-6 alkenyloxy group (optionally mono- to tri-substituted by halogen atom or C1-6 alkyl group), C3-6 alkynyloxy group (optionally mono- to tri-substituted by halogen atom or C1-6 alkyl group), phenoxy group (optionally mono- to tri-substituted by halogen atom, C3-5 cycloalkyl group, C1-6 alkoxy group, phenoxy group, 5 to 6-membered heterocyclic oxy group or C1-6 alkyl group), C3-6 cycloalkyl C1-6 alkoxy group optionally mono- to tri-substituted by halogen atom, phenyl C1-6 alkoxy group (optionally mono- to tri-substituted by halogen atom, C3-5 cycloalkyl group, C1-6 alkoxy group, phenoxy group, 5 to 6-membered heterocyclic oxy group or C1-6 alkyl group), 5 to 6-membered heterocyclic C1-6 alkoxy group (optionally mono- to tri-substituted by halogen atom, C3-5 cycloalkyl group, C1-6 alkoxy group, phenoxy group, 5 to 6-membered heterocyclic oxy group or C1-6 alkyl group), phenoxy C1-6 alkyl group (optionally mono- to tri-substituted by halogen atom, C3-5 cycloalkyl group, C1-6 alkoxy group, phenoxy group or 5 to 6-membered heterocyclic oxy group) or 5 to 6-membered heterocyclic oxy C1-6 alkyl group (optionally mono- to tri-substituted by halogen atom, C3-5 cycloalkyl group or C1-6 alkoxy group)].

In the present specification, examples of the halogen atom include a fluorine atom, chlorine atom and bromine atom, examples of the C1-6 alkyl group include a methyl group, ethyl group, n-propyl group, iso-propyl group, n-butyl group, isobutyl group, n-pentyl group, iso-pentyl group, n-hexyl group, 4-methylpentyl group and 3-methylpentyl group, examples of the C2-6 alkenyl group include a vinyl group, allyl group, 2-butenyl group, 3-butenyl group, 2-pentenyl group, 3-pentenyl group, prenyl group, 3-methyl-3-butenyl group, 2-hexenyl group, 3-hexenyl group, 4-hexenyl group, 4-methyl-4-pentenyl group and 4-methyl-3-pentenyl group, examples of the C2-6 alkynyl group include an ethynyl group, propargyl group, 2-butynyl group, 3-butynyl group, 2-pentynyl group, 3-pentynyl group, 4-pentynyl group, 2-hexynyl group, 3-hexynyl group, 4-hexynyl group and 5-hexynyl group, the C3-8 cycloalkyl group includes a cyclopropyl group, cyclobutyl group, cyclopentyl group, cyclohexyl group, cycloheptyl group and cyclooctyl group, examples of the C6-10 aryl group include a phenyl group, indenyl group and naphthyl group, examples of the 5 to 10-membered heterocyclic group include a furyl group, thienyl group, thiazolyl group, pyrazolyl group, pyridyl group, pyrimidinyl group, pyrazinyl group, pyridazinyl group, benzofuranyl group, benzothienyl group, benzothiazolyl group, chromenyl group, isochromenyl group, thiochromenyl group and isothiochromenyl group, examples of the C3-8 cycloalkyl C1-6 alkyl group include a cyclopropylmethyl group, cyclopropylethyl group, cyclopropylpropyl group, cyclopropylbutyl group, cyclopropylpentyl group, cyclopropylhexyl group, cyclobutylmethyl group, cyclobutylethyl group, cyclobutylpropyl group, cyclobutylbutyl group, cyclobutylpentyl group, cyclopentylethyl group, cyclopentylpropyl group, cyclopentylbutyl group, cyclohexylethyl group and cyclohexylpropyl group, examples of the C3-8 cycloalkylidene C1-6 alkyl group include a cyclopropylidenemethyl group, cyclopropylideneethyl group, cyclopropylidenepropyl group, cyclopropylidenebutyl group, cyclopropylidenepentyl group, cyclopropylidenehexyl group, cyclobutylidenemethyl group, cyclobutylideneethyl group, cyclobutylidenepropyl group, cyclobutylidenebutyl group, cyclobutylidenepentyl group, cyclopentylideneethyl group, cyclopentylidenepropyl group, cyclopentylidenebutyl group, cyclohexylideneethyl group and cyclohexylidenepropyl group, examples of the C6-10 aryl C1-6 alkyl group includes a benzyl group, phenetyl group, phenylpropyl group, phenylbutyl group, phenylpentyl group, 2-methyl-4-phenylbutyl group, 2-methyl-5-phenylpentyl group, 3-methyl-5-phenylpentyl group and (2-naphthyl)ethyl group, examples of the 5 to 10-membered heterocyclic C1-6 alkyl group includes a furylmethyl group, furylethyl group, furylpropyl group, furylbutyl group, furylpentyl group, furylhexyl group, thienylmethyl group, thienylethyl group, thienylpropyl group, thienylbutyl group, thienylpentyl group and thienylhexyl group, examples of the C1-6 alkoxy group include a methoxy group, ethoxy group, n-propyloxy group, iso-propoxy group, n-butoxy group, iso-butoxy group, sec-butoxy group, n-pentyloxy group, iso-pentyloxy group, sec-pentyloxy group, n-hexyloxy group, iso-hexyloxy group, sec-hexyloxy group and 2,3-dimethylbutoxy group, examples of the C3-6 alkenyloxy group include an allyloxy group, 1-methyl-2-propenyloxy group, 2-butenyloxy group, 3-butenyloxy group, 2-pentenyloxy group, 3-pentenyloxy group, prenyloxy group, 3-methyl-3-butenyloxy group, 2-hexenyloxy group, 3-hexenyloxy group, 4-hexenyloxy group, 4-methyl-4-pentenyloxy group and 4-methyl-3-pentenyloxy group, examples of the C3-6 alkynyloxy group include a propargyloxy group, 2-butynyloxy group, 3-butynyloxy group, 2-pentynyloxy group, 3-pentynyloxy group, 4-pentynyloxy group, 2-hexynyloxy group, 3-hexynyloxy group, 4-hexynyloxy group and 5-hexynyloxy group, examples of the C3-8 cycloalkoxy group include a cyclopropyloxy group, cyclobutyloxy group, cyclopentyloxy group, cyclohexyloxy group, cycloheptyloxy group and cyclooctyloxy group, examples of the C6-10 aryloxy group include a phenoxy group and naphthoxy group, examples of the C3-8 cycloalkyl C1-6 alkoxy group include a cyclopropylmethoxy group, cyclopropylethoxy group, cyclopropylpropoxy group, cyclopropylbutoxy group, cyclopropylpentyloxy group, cyclopropylhexyloxy group, cyclobutylmethoxy group, cyclobutylethoxy group, cyclobutylpropoxy group, cyclobutylbutoxy group, cyclobutylpentyloxy group, cyclopentylethoxy group, cyclopentylpropoxy group, cyclopentylbutoxy group, cyclohexylethoxy group and cyclohexylpropoxy group, examples of the C6-10 aryl C1-6 alkoxy group include a benzyloxy group, phenetyloxy group, phenylpropyloxy group, phenylbutyloxy group, phenylpentyloxy group, 2-methyl-4-phenylbutyloxy group, 2-methyl-5-phenylpentyloxy group, 3-methyl-5-phenylpentyloxy group and (2-naphthyl)ethyloxy group, examples of the 5 to 10-membered heterocyclic C1-6 alkoxy group include a furylmethoxy group, furylethoxy group, furylpropyloxy group, furylbutyloxy group, furylpentyloxy group, furylhexyloxy group, thienylmethoxy group, thienylethoxy group, thienylpropyloxy group, thienylbutyloxy group, thienylpentyloxy group and thienylhexyloxy group, examples of the C1-6 alkylthio group includes a methylthio group, ethylthio group, n-propylthio group, iso-propylthio group, n-butylthio group, iso-butylthio group, n-pentylthio group, iso-pentylthio group, n-hexylthio group, 4-methylpentylthio group and 3-methylpentylthio group, examples of the C3-6 alkenylthio group include an allylthio group, 1-methyl-2-propenylthio group, 2-butenylthio group, 3-butenylthio group, 2-pentenylthio group, 3-pentenylthio group, prenylthio group, 3-methyl-3-butenylthio group, 2-hexenylthio group, 3-hexenylthio group, 4-hexenylthio group, 4-methyl-4-pentenylthio group and 4-methyl-3-pentenylthio group, examples of the C3-6 alkynylthio group include a propargylthio group, 2-butynylthio group, 3-butynylthio group, 2-pentynylthio group, 3-pentynylthio group, 4-pentynylthio group, 2-hexynylthio group, 3-hexynylthio group, 4-hexynylthio group and 5-hexynylthio group, examples of the C3-8 cycloalkylthio group include a cyclopropylthio group, cyclobutylthio group, cyclopentylthio group, cyclohexylthio group, cycloheptylthio group and cyclooctylthio group, examples of the C6-10 arylthio group include a phenylthio group and naphthylthio group, examples of the C3-8 cycloalkyl C1-6 alkylthio group include a cyclopropylmethylthio group, cyclopropylethylthio group, cyclopropylpropylthio group, cyclopropylbutylthio group, cyclopropylpentylthio group, cyclopropylhexylthio group, cyclobutylmethylthio group, cyclobutylethylthio group, cyclobutylpropylthio group, cyclobutylbutylthio group, cyclobutylpentylthio group, cyclopentylethylthio group, cyclopentylpropylthio group, cyclopentylbutylthio group, cyclohexylethylthio group and cyclohexylpropylthio group, examples of the C6-10 aryl C1-6 alkylthio group include a benzylthio group, phenetylthio group, phenylpropylthio group, phenylbutylthio group, phenylpentylthio group, 2-methyl-4-phenylbutylthio group, 2-methyl-5-phenylpentylthio group and 3-methyl-5-phenylpentylthio group, examples of the 5 to 10-membered heterocyclic C1-6 alkylthio group include a furylmethylthio group, furylethylthio group, furylpropylthio group, furylbutylthio group, furylpentylthio group, furylhexylthio group, thienylethylthio group, thienylmethylthio group, thienylpropylthio group, thienylbutylthio group, thienylpentylthio group and thienylhexylthio group, examples of the mono-C1-6 alkylamino group include a methylamino group, ethylamino group, n-propylamino group, iso-propylamino group, n-butylamino group, iso-butylamino group, n-pentylamino group, iso-pentylamino group, n-hexylamino group, 4-methylpentylamino group and 3-methylpentylamino group, examples of the mono-C3-6 alkenylamino group include an allylamino group, 2-butenylamino group, 3-butenylamino group, 2-pentenylamino group, 3-pentenylamino group, prenylamino group, 3-methyl-3-butenylamino group, 2-hexenylamino group, 3-hexenylamino group, 4-hexenylamino group, 4-methyl-4-pentenylamino group and 4-methyl-3-pentenylamino group, examples of the mono-C3-6 alkynylamino group include a propargylamino group, 2-butynylamino group, 3-butynylamino group, 2-pentynylamino group, 3-pentynylamino group, 4-pentynylamino group, 2-hexynylamino group, 3-hexynylamino group, 4-hexynylamino group and 5-hexynylamino group, examples of the mono-C3-8 cycloalkylamino group include a cyclopropylamino group, cyclobutylamino group, cyclopentylamino group, cyclohexylamino group, cycloheptylamino group and cyclooctylamino group, examples of the mono-C6-10 arylamino group include a phenylamino group and naphthylamino group, examples of the mono-C3-8 cycloalkyl C1-6 alkylamino group include a cyclopropylmethylamino group, cyclopropylethylamino group, cyclopropylpropylamino group, cyclopropylbutylamino group, cyclopropylpentylamino group, cyclopropylhexylamino group, cyclobutylmethylamino group, cyclobutylethylamino group, cyclobutylpropylamino group, cyclobutylbutylamino group, cyclobutylpentylamino group, cyclopentylethylamino group, cyclopentylpropylamino group, cyclopentylbutylamino group, cyclohexylethylamino group and cyclohexylpropylamino group, examples of the mono-C6-10 aryl C1-6 alkylamino group include a benzylamino group, phenetylamino group, phenylpropylamino group, phenylbutylamino group, phenylpentylamino group, 2-methyl-4-phenylbutylamino group, 2-methyl-5-phenylpentylamino group, 3-methyl-5-phenylpentylamino group and (2-naphthyl)ethylamino group, examples of the mono-5 to 10-membered heterocyclic C1-6 alkylamino group include a furylmethylamino group, furylethylamino group, furylpropylamino group, furylbutylamino group, furylpentylamino group, furylhexylamino group, thienylethylamino group, thienylmethylamino group, thienylpropylamino group, thienylbutylamino group, thienylpentylamino group and thienylhexylamino group, examples of the di-C1-6 alkylamino group includes a dimethylamino group, methylethylamino group, diethylamino group, methylpropylamino group and methylbutylamino group, examples of the N-C3-6 alkenyl-N-C1-6 alkylamino group include an N-allyl-N-methylamino group, N-(2-butenyl)-N-methylamino group and N-(3-butenyl)-N-methylamino group, examples of the N-C3-6 alkynyl-N-C1-6 alkylamino group include an N-methyl-N-propargylamino group, N-(2-butynyl)-N-methylamino group and N-(3-butynyl)-N-methylamino group, examples of the N-C3-8 _{C}y_{C}loalkyl-N-C1-6 alkylamino group include an N-cyclopropyl-N-methylamino group, N-cyclobutyl-N-methylamino group and N-cyclopentyl-N-methylamino group, examples of the N-C6-10 aryl-N-C1-6 alkylamino group include an N-methylanilino group, examples of the N-C3-8 cycloalkyl C1-6 alkyl-N-C1-6 alkylamino group include an N-cyclopropylmethyl-N-methylamino group, N-cyclopropylethyl-N-methylamino group, N-cyclopropylpropyl-N-methylamino group, N-cyclobutylmethyl-N-methylamino group, N-cyclobutylethyl-N-methylamino group and N-cyclobutylpropyl-N-methylamino group, examples of the N-C6-10 aryl C1-6 alkyl-N-C1-6 alkylamino group include an N-benzyl-N-methylamino group, N-phenetyl-N-methylamino group, N-phenylpropyl-N-methylamino group, N-phenylbutyl-N-methylamino group and N-phenylpentyl-N-methylamino group, examples of the N-5 to 10-membered heterocyclic C1-6 alkyl-N-C1-6 alkylamino group, C1-6 alkylcarbonyl group include an N-furylmethyl-N-methylamino group, N-furylethyl-N-methylamino group, N-furylpropyl-N-methylamino group, N-furylbutyl-N-methylamino group, N-furylpentyl-N-methylamino group, N-thienyl-N-methylamino group, N-thienylethyl-N-methylamino group, N-thienylpropyl-N-methylamino group, N-thienylbutyl-N-methylamino group and N-thienylpentyl-N-methylamino group, examples of the C1-6 alkylsulfonyl group includes a methanesulfonyl group, ethanesulfonyl group, propanesulfonyl group, butanesulfonyl group, pentanesulfonyl group and hexanesulfonyl group, examples of the C6-10 aryloxy C1-6 alkyl group include a benzyloxymethyl group, benzyloxyethyl group, benzyloxypropyl group, benzyloxybutyl group and benzyloxypentyl group, (2-naphthyl)oxymethyl group, examples of the 5 to 10-membered heterocyclic oxy C1-6 alkyl group include a furyloxymethyl group, furyloxyethyl group, furyloxypropyl group, furyloxybutyl group, furyloxypentyl group, thienyloxymethyl group, thienyloxyethyl group, thienyloxypropyl group, thienyloxybutyl group, thienyloxypentyl group, pyridyloxymethyl group, pyridyloxyethyl group, benzofuranyloxymethyl group and benzothienyloxymethyl group, and examples of the C6-10 aryl C2-6 alkel group include a styryl group and phenylpropenyl group.

More specifically, examples of E include a phenyl group, 2-furyl group, 3-furyl group, 2-thienyl group, 3-thienyl group, 5-phenylfuran-2-yl group, 5-phenoxyfuran-2-yl group, 5-(4-fluorophenoxy)furan-2-yl group, 5-(3-fluorophenoxy)furan-2-yl group, 5-(4-methylphenoxy)furan-2-yl group, 5-(4-chlorophenoxy)furan-2-yl group, 5-(3-chlorophenoxy)furan-2-yl group, 5-(3-methylphenoxy)furan-2-yl group, 5-(4-methoxyphenoxy)furan-2-yl group, 5-(3-methoxyphenoxy)furan-2-yl group, 5-benzylfuran-2-yl group, 5-(4-fluorophenyl)methylfuran-2-yl group, 5-(3-fluorophenyl)methylfuran-2-yl group, 5-(4-methylphenyl)methylfuran-2-yl group, 5-(4-chlorophenyl)methylfuran-2-yl group, 5-(3-chlorophenyl)methylfuran-2-yl group, 5-(3-methylphenyl)methylfuran-2-yl group, 5-(4-methoxyphenyl)methylfuran-2-yl group, 5-(3-methoxyphenyl)methylfuran-2-yl group, 5-phenoxythiophen-2-yl group, 5-(4-fluorophenoxy)thiophen-2-yl group, 5-(3-fluorophenoxy)thiophen-2-yl group, 5-(2-fluorophenoxy)thiophen-2-yl group, 5-(4-methylphenoxy)thiophen-2-yl group, 5-(4-chlorophenoxy)thiophen-2-yl group, 5-(3-chlorophenoxy)thiophen-2-yl group, 5-(3-methylphenoxy)thiophen-2-yl group, 5-(4-methoxyphenoxy)thiophen-2-yl group, 5-(3-methoxyphenoxy)thiophen-2-yl group, 5-(3-cyanophenoxy)thiophen-2-yl group, 5-benzyloxythiophen-2-yl group, 5-benzylthiophen-2-yl group, 5-(4-fluorophenyl)methylthiophen-2-yl group, 5-(3-fluorophenyl)methylthiophen-2-yl group, 5-(4-methylphenyl)methylthiophen-2-yl group, 5-(4-chlorophenyl)methylthiophen-2-yl group, 5-(3-chlorophenyl)methylthiophen-2-yl group, 5-(3-methylphenyl)methylthiophen-2-yl group, 5-(4-methoxyphenyl)methylthiophen-2-yl group, 5-(3-methoxyphenyl)methylthiophen-2-yl group, 5-(2-thienyl)methylthiophen-2-yl group, 5-(2-pyridyl)methylthiophen-2-yl group, 5-(2-benzofuranyl)methylthiophen-2-yl group, 5-phenoxythiophen-3-yl group, 5-(4-fluorophenoxy)thiophen-3-yl group, 5-(3-fluorophenoxy)thiophen-3-yl group, 5-(4-methylphenoxy) thiophen-3-yl group, 5-(4-chlorophenoxy) thiophen-3-yl group, 5-(3-chlorophenoxy) thiophen-3-yl group, 5-(3-methylphenoxy) thiophen-3-yl group, 5-(4-methoxyphenoxy) thiophen-3-yl group, 5-(3-methoxyphenoxy)thiophen-3-yl group, 3-methylphenyl group, 3-ethylphenyl group, 3-propylphenyl group, 3-butylphenyl group, 3-pentylphenyl group, 3-hexylphenyl group, 3-heptylphenyl group, 3-isobutylphenyl group, 3-(2-isobutenyl)phenyl group, 3-cyclopentylmethylidenephenyl group, 3-biphenyl group, 3-benzylphenyl group, 3-phenylaminophenyl group, 3-(N-phenyl-N-methylamino)phenyl group, 3-phenylthiophenyl group, 3-phenoxyphenyl group, 3-(2-fluorophenoxy)phenyl group, 3-(3-fluorophenoxy)phenyl group, 3-(4-fluorophenoxy)phenyl group, 3-(3,5-difluorophenoxy)phenyl group, 3-(2-chlorophenoxy)phenyl group, 3-(3-chlorophenoxy)phenyl group, 3-(4-chlorophenoxy)phenyl group, 3-(2-methylphenoxy)phenyl group, 3-(3-methylphenoxy)phenyl group, 3-(4-methylphenoxy)phenyl group, 3-(2-methoxyphenoxy)phenyl group, 3-(3-methoxyphenoxy)phenyl group, 3-(4-methoxyphenoxy)phenyl group, 3-(2-cyanophenoxy)phenyl group, 3-(3-cyanophenoxy)phenyl group, 3-(4-cyanophenoxy)phenyl group, 3-benzyloxyphenyl group, 3-(2-fluorophenylmethoxy)phenyl group, 3-(3-fluorophenylmethoxy)phenyl group, 3-(4-fluorophenylmethoxy)phenyl group, 3-(3,5-difluorophenylmethoxy)phenyl group, 3-(2-chlorophenylmethoxy)phenyl group, 3-(3-chlorophenylmethoxy)phenyl group, 3-(4-chlorophenylmethoxy)phenyl group, 3-(2-methylphenylmethoxy)phenyl group, 3-(3-methylphenylmethoxy)phenyl group, 3-(4-methylphenylmethoxy)phenyl group, 3-(2-methoxyphenylmethoxy)phenyl group, 3-(3-methoxyphenylmethoxy)phenyl group, 3-(4-methoxyphenylmethoxy)phenyl group, 3-(2-cyanophenylmethoxy)phenyl group, 3-(3-cyanophenylmethoxy)phenyl group, 3-(4-cyanophenylmethoxy)phenyl group, 3-benzylaminophenyl group, 3-(N-benzyl-N-methylamino)phenyl group, 3-(2-furylmethoxy)phenyl group, 3-(3-furylmethoxy)phenyl group, 3-(2-thienylmethoxy)phenyl group, 3-(3-thienylmethoxy)phenyl group, 3-(2-pyridylmethoxy) phenyl group, 3-(3-pyridylmethoxy)phenyl group, 3- (4-pyridylmethoxy) phenyl group, 3-(2-pyridyloxy)phenyl group, 3-(3-pyridyloxy)phenyl group, 3-(4-pyridyloxy)phenyl group, 3-(6-trifluoromethylpyridin-2-yloxy)phenyl group, 3-(5-trifluoromethylpyridin-2-yloxy)phenyl group, 3-(3-chloro-5-trifluoromethylpyridin-2-yloxy)phenyl group, 3-(2-pyrazinyloxy)phenyl group, 3-(6-chloropyridazin-3-yloxy)phenyl group, 3-methoxyphenyl group, 3-ethoxyphenyl group, 3-propoxyphenyl group, 3-(2-methylethoxy)phenyl group, 3-butoxyphenyl group, 3-(2-methylbutoxy)phenyl group, 3-(3-methylbutoxy)phenyl group, 3-pentyloxyphenyl group, 3-hexyloxyphenyl group, 3-heptyloxyphenyl group, 3-isoprenyloxyphenyl group, 3-(2-fluoroethoxy)phenyl group, 3-(3-fluoropropoxy)phenyl group, 3-(4-fluorobutoxy)phenyl group, 3-(5-fluoropentyloxy)phenyl group, 3-(6-fluorohexyloxy)phenyl group, 3-(7-fluoroheptyloxy)phenyl group, 3-(2-chloroethoxy)phenyl group, 3-(3-chloropropoxy)phenyl group, 3-(4-chlorobutoxy)phenyl group, 3-(5-chloropentyloxy)phenyl group, 3-(6-chlorohexyloxy)phenyl group, 3-(7-chloroheptyloxy)phenyl group, 3-(2-propenyloxy)phenyl group, 3-(2-butenyloxy)phenyl group, 3-(3-butenyloxy)phenyl group, 3-(2-pentenyloxy)phenyl group, 3-(3-pentenyloxy)phenyl group, 3-(4-pentenyloxy)phenyl group, 3-(2-hexenyloxy)phenyl group, 3-(3-hexenyloxy)phenyl group, 3-(4-hexenyloxy)phenyl group, 3-(5-hexenyloxy)phenyl group, 3-(2-heptenyloxy)phenyl group, 3-(3-heptenyloxy)phenyl group, 3-(4-heptenyloxy)phenyl group, 3-(5-heptenyloxy)phenyl group, 3-(6-heptenyloxy)phenyl group, 3-(3-chloro-2-propenyloxy)phenyl group, 3-(3,3-dichloro-2-propenyloxy)phenyl group, 3-(2-methyl-2-butenyloxy)phenyl group, 3-(3-methyl-2-butenyloxy)phenyl group, 3-(2-propynyloxy)phenyl group, 3-(2-butynyloxy)phenyl group, 3-(3-butynyloxy)phenyl group, 3-(2-pentynyloxy)phenyl group, 3-(3-pentynyloxy)phenyl group, 3-(4-pentynyloxy)phenyl group, 3-(2-hexynyloxy)phenyl group, 3-(3-hexynyloxy)phenyl group, 3-(4-hexynyloxy)phenyl group, 3-(5-hexynyloxy)phenyl group, 3-(2-heptynyloxy)phenyl group, 3-(3-heptynyloxy)phenyl group, 3-(4-heptynyloxy)phenyl group, 3-(5-heptynyloxy)phenyl group, 3-(6-heptynyloxy)phenyl group, 3-(2-methoxyethoxy)phenyl group, 3-(3-methoxypropoxy)phenyl group, 3-(4-methoxybutoxy)phenyl group, 3-(5-methoxypentyloxy)phenyl group, 3-(6-methoxyhexyloxy)phenyl group, 3-(7-methoxyheptyloxy)phenyl group, 3-(2-ethoxyethoxy)phenyl group, 3-(3-ethoxypropoxy)phenyl group, 3-(4-ethoxybutoxy)phenyl group, 3-(5-ethoxypentyloxy)phenyl group, 3-(6-ethoxyhexyloxy)phenyl group, 3-(7-ethoxyheptyloxy)phenyl group, 3-cyclopropylmethoxyphenyl group, 3-(2-cyclopropylethoxy)phenyl group, 3-(3-cyclopropylpropoxy)phenyl group, 3-(4-cyclopropylbutoxy)phenyl group, 3-(5-cyclopropylpentyloxy)phenyl group, 3-(6-cyclopropylhexyloxy)phenyl group, 3-(7-cyclopropylheptyloxy)phenyl group, 3-phenoxymethylphenyl group, 3-(2-phenylethyl)phenyl group, 3-(3-phenylpropyl)phenyl group, 3-(4-phenylbutyl)phenyl group, 3-(5-phenylpentyl)phenyl group, 3-(2-phenylethoxy)phenyl group, 3-(3-phenylpropoxy)phenyl group, 3-(4-phenylbutoxy)phenyl group, 3-(5-phenylpentyloxy)phenyl group, (Z)-3-styrylphenyl group, 2-fluoro-3-methylphenyl group, 2-fluoro-3-ethylphenyl group, 2-fluoro-3-propylphenyl group, 2-fluoro-3-butylphenyl group, 2-fluoro-3-pentylphenyl group, 2-fluoro-3-hexylphenyl group, 2-fluoro-3-heptylphenyl group, 2-fluoro-3-isobutylphenyl group, 2-fluoro-3-(2-isobutenyl)phenyl group, 2-fluoro-3-cyclopentylmethylidenephenyl group, 2-fluoro-3-biphenyl group, 2-fluoro-3-benzylphenyl group, 2-fluoro-3-phenylaminophenyl group, 2-fluoro-3-(N-phenyl-N-methylamino)phenyl group, 2-fluoro-3-phenylthiophenyl group, 2-fluoro-3-phenoxyphenyl group, 2-fluoro-3-(2-fluorophenoxy)phenyl group, 2-fluoro-3-(3-fluorophenoxy)phenyl group, 2-fluoro-3-(4-fluorophenoxy)phenyl group, 2-fluoro-3-(3,5-difluorophenoxy)phenyl group, 2-fluoro-3-(2-chlorophenoxy)phenyl group, 2-fluoro-3-(3-chlorophenoxy)phenyl group, 2-fluoro-3-(4-chlorophenoxy)phenyl group, 2-fluoro-3-(2-methylphenoxy)phenyl group, 2-fluoro-3-(3-methylphenoxy)phenyl group, 2-fluoro-3-(4-methylphenoxy)phenyl group, 2-fluoro-3-(2-methoxyphenoxy)phenyl group, 2-fluoro-3-(3-methoxyphenoxy)phenyl group, 2-fluoro-3-(4-methoxyphenoxy)phenyl group, 2-fluoro-3-(2-cyanophenoxy)phenyl group, 2-fluoro-3-(3-cyanophenoxy)phenyl group, 2-fluoro-3-(4-cyanophenoxy)phenyl group, 3-benzyloxyphenyl group, 2-fluoro-3-(2-fluorophenylmethoxy)phenyl group, 2-fluoro-3-(3-fluorophenylmethoxy)phenyl group, 2-fluoro-3-(4-fluorophenylmethoxy)phenyl group, 2-fluoro-3-(3,5-difluorophenylmethoxy)phenyl group, 2-fluoro-3-(2-chlorophenylmethoxy)phenyl group, 2-fluoro-3-(3-chlorophenylmethoxy)phenyl group, 2-fluoro-3-(4-chlorophenylmethoxy)phenyl group, 2-fluoro-3-(2-methylphenylmethoxy)phenyl group, 2-fluoro-3-(3-methylphenylmethoxy)phenyl group, 2-fluoro-3-(4-methylphenylmethoxy)phenyl group, 2-fluoro-3-(2-methoxyphenylmethoxy)phenyl group, 2-fluoro-3-(3-methoxyphenylmethoxy)phenyl group, 2-fluoro-3-(4-methoxyphenylmethoxy)phenyl group, 2-fluoro-3-(2-cyanophenylmethoxy)phenyl group, 2-fluoro-3-(3-cyanophenylmethoxy)phenyl group, 2-fluoro-3-(4-cyanophenylmethoxy)phenyl group, 2-fluoro-3-benzylaminophenyl group, 2-fluoro-3-(N-benzyl-N-methylamino)phenyl group, 2-fluoro-3-(2-furylmethoxy)phenyl group, 2-fluoro-3-(3-furylmethoxy)phenyl group, 2-fluoro-3-(2-thienylmethoxy)phenyl group, 2-fluoro-3-(3-thienylmethoxy)phenyl group, 2-fluoro-3-(2-pyridylmethoxy)phenyl group, 2-fluoro-3-(3-pyridylmethoxy)phenyl group, 2-fluoro-3-(4-pyridylmethoxy)phenyl group, 2-fluoro-3-(2-pyridyloxy)phenyl group, 2-fluoro-3-(3-pyridyloxy)phenyl group, 2-fluoro-3-(4-pyridyloxy)phenyl group, 2-fluoro-3-(6-trifluoromethylpyridin-2-yloxy)phenyl group, 2-fluoro-3-(5-trifluoromethylpyridin-2-yloxy)phenyl group, 2-fluoro-3-(3-chloro-5-trifluoromethylpyridin-2-yloxy)phenyl group, 2-fluoro-3-(2-pyrazinyloxy)phenyl group, 2-fluoro-3-(6-chloropyridazin-3-yloxy)phenyl group, 2-fluoro-3-methoxyphenyl group, 2-fluoro-3-ethoxyphenyl group, 2-fluoro-3-propoxyphenyl group, 2-fluoro-3-(2-methylethoxy)phenyl group, 2-fluoro-3-butoxyphenyl group, 2-fluoro-3-(2-methylbutoxy)phenyl group, 2-fluoro-3-(3-methylbutoxy)phenyl group, 2-fluoro-3-pentyloxyphenyl group, 2-fluoro-3-hexyloxyphenyl group, 2-fluoro-3-heptyloxyphenyl group, 2-fluoro-3-isoprenyloxyphenyl group, 2-fluoro-3-(2-fluoroethoxy)phenyl group, 2-fluoro-3-(3-fluoropropoxy)phenyl group, 2-fluoro-3-(4-fluorobutoxy)phenyl group, 2-fluoro-3-(5-fluoropentyloxy)phenyl group, 2-fluoro-3-(6-fluorohexyloxy)phenyl group, 2-fluoro-3-(7-fluoroheptyloxy)phenyl group, 2-fluoro-3-(2-chloroethoxy)phenyl group, 2-fluoro-3-(3-chloropropoxy)phenyl group, 2-fluoro-3-(4-chlorobutoxy)phenyl group, 2-fluoro-3-(5-chloropentyloxy)phenyl group, 2-fluoro-3-(6-chlorohexyloxy)phenyl group, 2-fluoro-3-(7-chloroheptyloxy)phenyl group, 2-fluoro-3-(2-propenyloxy)phenyl group, 2-fluoro-3-(2-butenyloxy)phenyl group, 2-fluoro-3-(3-butenyloxy)phenyl group, 2-fluoro-3-(2-pentenyloxy)phenyl group, 2-fluoro-3-(3-pentenyloxy)phenyl group, 2-fluoro-3-(4-pentenyloxy)phenyl group, 2-fluoro-3-(2-hexenyloxy)phenyl group, 2-fluoro-3-(3-hexenyloxy)phenyl group, 2-fluoro-3-(4-hexenyloxy)phenyl group, 2-fluoro-3-(5-hexenyloxy)phenyl group, 2-fluoro-3-(2-heptenyloxy)phenyl group, 2-fluoro-3-(3-heptenyloxy)phenyl group, 2-fluoro-3-(4-heptenyloxy)phenyl group, 2-fluoro-3-(5-heptenyloxy)phenyl group, 2-fluoro-3-(6-heptenyloxy)phenyl group, 2-fluoro-3-(3-chloro-2-propenyloxy)phenyl group, 2-fluoro-3-(3,3-dichloro-2-propenyloxy)phenyl group, 2-fluoro-3-(2-methyl-2-butenyloxy)phenyl group, 2-fluoro-3-(3-methyl-2-butenyloxy)phenyl group, 2-fluoro-3-(2-propynyloxy)phenyl group, 2-fluoro-3-(2-butynyloxy)phenyl group, 2-fluoro-3-(3-butynyloxy)phenyl group, 2-fluoro-3-(2-pentynyloxy)phenyl group, 2-fluoro-3-(3-pentynyloxy)phenyl group, 2-fluoro-3-(4-pentynyloxy)phenyl group, 2-fluoro-3-(2-hexynyloxy)phenyl group, 2-fluoro-3-(3-hexynyloxy)phenyl group, 2-fluoro-3-(4-hexynyloxy)phenyl group, 2-fluoro-3-(5-hexynyloxy)phenyl group, 2-fluoro-3-(2-heptynyloxy)phenyl group, 2-fluoro-3-(3-heptynyloxy)phenyl group, 2-fluoro-3-(4-heptynyloxy)phenyl group, 2-fluoro-3-(5-heptynyloxy)phenyl group, 2-fluoro-3-(6-heptynyloxy)phenyl group, 3-(2-methoxyethoxy)phenyl group, 2-fluoro-3-(3-methoxypropoxy)phenyl group, 2-fluoro-3-(4-methoxybutoxy)phenyl group, 2-fluoro-3-(5-methoxypentyloxy)phenyl group, 2-fluoro-3-(6-methoxyhexyloxy)phenyl group, 2-fluoro-3-(7-methoxyheptyloxy)phenyl group, 2-fluoro-3-(2-ethoxyethoxy)phenyl group, 2-fluoro-3-(3-ethoxypropoxy)phenyl group, 2-fluoro-3-(4-ethoxybutoxy)phenyl group, 2-fluoro-3-(5-ethoxypentyloxy)phenyl group, 2-fluoro-3-(6-ethoxyhexyloxy)phenyl group, 2-fluoro-3-(7-ethoxyheptyloxy)phenyl group, 2-fluoro-3-cyclopropylmethoxyphenyl group, 2-fluoro-3-(2-cyclopropylethoxy)phenyl group, 2-fluoro-3-(3-cyclopropylpropoxy)phenyl group, 2-fluoro-3-(4-cyclopropylbutoxy)phenyl group, 2-fluoro-3-(5-cyclopropylpentyloxy)phenyl group, 2-fluoro-3-(6-cyclopropylhexyloxy)phenyl group, 2-fluoro-3-(7-cyclopropylheptyloxy)phenyl group, 2-fluoro-3-phenoxymethylphenyl group, 2-fluoro-3-(2-phenylethyl)phenyl group, 2-fluoro-3-(3-phenylpropyl)phenyl group, 2-fluoro-3-(4-phenylbutyl)phenyl group, 2-fluoro-3-(5-phenylpentyl)phenyl group, 2-fluoro-3-(2-phenylethoxy)phenyl group, 2-fluoro-3-(3-phenylpropoxy)phenyl group, 2-fluoro-3-(4-phenylbutoxy)phenyl group, 2-fluoro-3-(5-phenylpentyloxy)phenyl group, (Z)-2-fluoro-3-styrylphenyl group, 4-methylphenyl group, 4-ethylphenyl group, 4-propylphenyl group, 4-butylphenyl group, 4-pentylphenyl group, 4-hexylphenyl group, 4-heptylphenyl group, 4-isobutylphenyl group, 4-(2-isobutenyl)phenyl group, 4-cyclopentylmethylidenephenyl group, 4-biphenyl group, 4-benzylphenyl group, 4-phenylaminophenyl group, 4-(N-phenyl-N-methylamino)phenyl group, 4-phenylthiophenyl group, 4-phenoxyphenyl group, 4-(2-fluorophenoxy)phenyl group, 4-(3-fluorophenoxy)phenyl group, 4-(4-fluorophenoxy)phenyl group, 4-(3,5-difluorophenoxy)phenyl group, 4-(2-chlorophenoxy)phenyl group, 4-(3-chlorophenoxy)phenyl group, 4-(4-chlorophenoxy)phenyl group, 4-(2-methylphenoxy)phenyl group, 4-(3-methylphenoxy)phenyl group, 4-(4-methylphenoxy)phenyl group, 4-(2-methoxyphenoxy)phenyl group, 4-(3-methoxyphenoxy)phenyl group, 4-(4-methoxyphenoxy)phenyl group, 4-(2-cyanophenoxy)phenyl group, 4-(3-cyanophenoxy)phenyl group, 4-(4-cyanophenoxy)phenyl group, 4-benzyloxyphenyl group, 4-(2-fluorophenylmethoxy)phenyl group, 4-(3-fluorophenylmethoxy)phenyl group, 4-(4-fluorophenylmethoxy)phenyl group, 4-(3,5-difluorophenylmethoxy)phenyl group, 4-(2-chlorophenylmethoxy)phenyl group, 4-(3-chlorophenylmethoxy)phenyl group, 4-(4-chlorophenylmethoxy)phenyl group, 4-(2-methylphenylmethoxy)phenyl group, 4-(3-methylphenylmethoxy)phenyl group, 4-(4-methylphenylmethoxy)phenyl group, 4-(2-methoxyphenylmethoxy)phenyl group, 4-(3-methoxyphenylmethoxy)phenyl group, 4-(4-methoxyphenylmethoxy)phenyl group, 4-(2-cyanophenylmethoxy)phenyl group, 4-(3-cyanophenylmethoxy)phenyl group, 4-(4-cyanophenylmethoxy)phenyl group, 4-benzylaminophenyl group, 4-(N-benzyl-N-methylamino)phenyl group, 4-(2-furylmethoxy)phenyl group, 4-(3-furylmethoxy)phenyl group, 4-(2-thienylmethoxy)phenyl group, 4-(3-thienylmethoxy)phenyl group, 4-(2-pyridylmethoxy)phenyl group, 4-(3-pyridylmethoxy)phenyl group, 4-(4-pyridylmethoxy)phenyl group, 4-(2-pyridyloxy)phenyl group, 4-(3-pyridyloxy)phenyl group, 4-(4-pyridyloxy)phenyl group, 4-(6-trifluoromethylpyridin-2-yloxy)phenyl group, 4-(5-trifluoromethylpyridin-2-yloxy)phenyl group, 4-(3-chloro-5-trifluoromethylpyridin-2-yloxy)phenyl group, 4-(2-pyrazinyloxy)phenyl group, 4-(6-chloropyridazin-3-yloxy)phenyl group, 4-methoxyphenyl group, 4-ethoxyphenyl group, 4-propoxyphenyl group, 4-(2-methylethoxy)phenyl group, 4-butoxyphenyl group, 4-(2-methylbutoxy)phenyl group, 4-(3-methylbutoxy)phenyl group, 4-pentyloxyphenyl group, 4-hexyloxyphenyl group, 4-heptyloxyphenyl group, 4-isoprenyloxyphenyl group, 4-(2-fluoroethoxy)phenyl group, 4-(3-fluoropropoxy)phenyl group, 4-(4-fluorobutoxy)phenyl group, 4-(5-fluoropentyloxy)phenyl group, 4-(6-fluorohexyloxy)phenyl group, 4-(7-fluoroheptyloxy)phenyl group, 4-(2-chloroethoxy)phenyl group, 4-(3-chloropropoxy)phenyl group, 4-(4-chlorobutoxy)phenyl group, 4-(5-chloropentyloxy)phenyl group, 4-(6-chlorohexyloxy)phenyl group, 4-(7-chloroheptyloxy)phenyl group, 4-(2-propenyloxy)phenyl group, 4-(2-butenyloxy)phenyl group, 4-(3-butenyloxy)phenyl group, 4-(2-pentenyloxy)phenyl group, 4-(3-pentenyloxy)phenyl group, 4-(4-pentenyloxy)phenyl group, 4-(2-hexenyloxy)phenyl group, 4-(3-hexenyloxy)phenyl group, 4-(4-hexenyloxy)phenyl group, 4-(5-hexenyloxy)phenyl group, 4-(2-heptenyloxy)phenyl group, 4-(3-heptenyloxy)phenyl group, 4-(4-heptenyloxy)phenyl group, 4-(5-heptenyloxy)phenyl group, 4-(6-heptenyloxy)phenyl group, 4-(3-chloro-2-propenyloxy)phenyl group, 4-(3,3-dichloro-2-propenyloxy)phenyl group, 4-(2-methyl-2-butenyloxy)phenyl group, 4-(3-methyl-2-butenyloxy)phenyl group, 4-(2-propynyloxy)phenyl group, 4-(2-butynyloxy)phenyl group, 4-(3-butynyloxy)phenyl group, 4-(2-pentynyloxy)phenyl group, 4-(3-pentynyloxy)phenyl group, 4-(4-pentynyloxy)phenyl group, 4-(2-hexynyloxy)phenyl group, 4-(3-hexynyloxy)phenyl group, 4-(4-hexynyloxy)phenyl group, 4-(5-hexynyloxy)phenyl group, 4-(2-heptynyloxy)phenyl group, 4-(3-heptynyloxy)phenyl group, 4-(4-heptynyloxy)phenyl group, 4-(5-heptynyloxy)phenyl group, 4-(6-heptynyloxy)phenyl group, 4-(2-methoxyethoxy)phenyl group, 4-(3-methoxypropoxy)phenyl group, 4-(4-methoxybutoxy)phenyl group, 4-(5-methoxypentyloxy)phenyl group, 4-(6-methoxyhexyloxy)phenyl group, 4-(7-methoxyheptyloxy)phenyl group, 4-(2-ethoxyethoxy)phenyl group, 4-(3-ethoxypropoxy)phenyl group, 4-(4-ethoxybutoxy)phenyl group, 4-(5-ethoxypentyloxy)phenyl group, 4-(6-ethoxyhexyloxy)phenyl group, 4-(7-ethoxyheptyloxy)phenyl group, 4-cyclopropylmethoxyphenyl group, 4-(2-cyclopropylethoxy)phenyl group, 4-(3-cyclopropylpropoxy)phenyl group, 4-(4-cyclopropylbutoxy)phenyl group, 4-(5-cyclopropylpentyloxy)phenyl group, 4-(6-cyclopropylhexyloxy)phenyl group, 4-(7-cyclopropylheptyloxy)phenyl group, 4-phenoxymethylphenyl group, 4-(2-phenylethyl)phenyl group, 4-(3-phenylpropyl)phenyl group, 4-(4-phenylbutyl)phenyl group, 4-(5-phenylpentyl)phenyl group, 4-(2-phenylethoxy)phenyl group, 4-(3-phenylpropoxy)phenyl group, 4-(4-phenylbutoxy)phenyl group, 4-(5-phenylpentyloxy)phenyl group, (Z)-4-styrylphenyl group, 2-fluoro-4-methylphenyl group, 2-fluoro-4-ethylphenyl group, 2-fluoro-4-propylphenyl group, 2-fluoro-4-butylphenyl group, 2-fluoro-4-pentylphenyl group, 2-fluoro-4-hexylphenyl group, 2-fluoro-4-heptylphenyl group, 2-fluoro-4-isobutylphenyl group, 2-fluoro-4-(2-isobutenyl)phenyl group, 2-fluoro-4-cyclopentylmethylidenephenyl group, 2-fluoro-4-phenylphenyl group, 2-fluoro-4-benzylphenyl group, 2-fluoro-4-phenylaminophenyl group, 2-fluoro-4-(N-phenyl-N-methylamino)phenyl group, 2-fluoro-4-phenylthiophenyl group, 2-fluoro-4-phenoxyphenyl group, 2-fluoro-4-(2-fluorophenoxy)phenyl group, 2-fluoro-4-(3-fluorophenoxy)phenyl group, 2-fluoro-4-(4-fluorophenoxy)phenyl group, 2-fluoro-4-(3,5-difluorophenoxy)phenyl group, 2-fluoro-4-(2-chlorophenoxy)phenyl group, 2-fluoro-4-(3-chlorophenoxy)phenyl group, 2-fluoro-4-(4-chlorophenoxy)phenyl group, 2-fluoro-4-(2-methylphenoxy)phenyl group, 2-fluoro-4-(3-methylphenoxy)phenyl group, 2-fluoro-4-(4-methylphenoxy)phenyl group, 2-fluoro-4-(2-methoxyphenoxy)phenyl group, 2-fluoro-4-(3-methoxyphenoxy)phenyl group, 2-fluoro-4-(4-methoxyphenoxy)phenyl group, 2-fluoro-4-(2-cyanophenoxy)phenyl group, 2-fluoro-4-(3-cyanophenoxy)phenyl group, 2-fluoro-4-(4-cyanophenoxy)phenyl group, 4-benzyloxyphenyl group, 2-fluoro-4-(2-fluorophenylmethoxy)phenyl group, 2-fluoro-4-(3-fluorophenylmethoxy)phenyl group, 2-fluoro-4-(4-fluorophenylmethoxy)phenyl group, 2-fluoro-4-(3,5-difluorophenylmethoxy)phenyl group, 2-fluoro-4-(2-chlorophenylmethoxy)phenyl group, 2-fluoro-4-(3-chlorophenylmethoxy)phenyl group, 2-fluoro-4-(4-chlorophenylmethoxy)phenyl group, 2-fluoro-4-(2-methylphenylmethoxy)phenyl group, 2-fluoro-4-(3-methylphenylmethoxy)phenyl group, 2-fluoro-4-(4-methylphenylmethoxy)phenyl group, 2-fluoro-4-(2-methoxyphenylmethoxy)phenyl group, 2-fluoro-4-(3-methoxyphenylmethoxy)phenyl group, 2-fluoro-4-(4-methoxyphenylmethoxy)phenyl group, 2-fluoro-4-(2-cyanophenylmethoxy)phenyl group, 2-fluoro-4-(3-cyanophenylmethoxy)phenyl group, 2-fluoro-4-(4-cyanophenylmethoxy)phenyl group, 2-fluoro-4-benzylaminophenyl group, 2-fluoro-4-(N-benzyl-N-methylamino)phenyl group, 2-fluoro-4-(2-furylmethoxy)phenyl group, 2-fluoro-4-(3-furylmethoxy)phenyl group, 2-fluoro-4-(2-thienylmethoxy)phenyl group, 2-fluoro-4-(3-thienylmethoxy)phenyl group, 2-fluoro-4-(2-pyridylmethoxy)phenyl group, 2-fluoro-4-(3-pyridylmethoxy)phenyl group, 2-fluoro-4-(4-pyridylmethoxy)phenyl group, 2-fluoro-4-(2-pyridyloxy)phenyl group, 2-fluoro-4-(3-pyridyloxy)phenyl group, 2-fluoro-4-(4-pyridyloxy)phenyl group, 2-fluoro-4-(6-trifluoromethylpyridin-2-yloxy)phenyl group, 2-fluoro-4-(5-trifluoromethylpyridin-2-yloxy)phenyl group, 2-fluoro-4-(3-chloro-5-trifluoromethylpyridin-2-yloxy)phenyl group, 2-fluoro-4-(2-pyrazinyloxy)phenyl group, 2-fluoro-4-(6-chloropyridazin-3-yloxy)phenyl group, 2-fluoro-4-methoxyphenyl group, 2-fluoro-4-ethoxyphenyl group, 2-fluoro-4-propoxyphenyl group, 2-fluoro-4-(2-methylethoxy)phenyl group, 2-fluoro-4-butoxyphenyl group, 2-fluoro-4-(2-methylbutoxy)phenyl group, 2-fluoro-4-(3-methylbutoxy)phenyl group, 2-fluoro-4-pentyloxyphenyl group, 2-fluoro-4-hexyloxyphenyl group, 2-fluoro-4-heptyloxyphenyl group, 2-fluoro-4-isoprenyloxyphenyl group, 2-fluoro-4-(2-fluoroethoxy)phenyl group, 2-fluoro-4-(3-fluoropropoxy)phenyl group, 2-fluoro-4-(4-fluorobutoxy)phenyl group, 2-fluoro-4-(5-fluoropentyloxy)phenyl group, 2-fluoro-4-(6-fluorohexyloxy)phenyl group, 2-fluoro-4-(7-fluoroheptyloxy)phenyl group, 2-fluoro-4-(2-chloroethoxy)phenyl group, 2-fluoro-4-(3-chloropropoxy)phenyl group, 2-fluoro-4-(4-chlorobutoxy)phenyl group, 2-fluoro-4-(5-chloropentyloxy)phenyl group, 2-fluoro-4-(6-chlorohexyloxy)phenyl group, 2-fluoro-4-(7-chloroheptyloxy)phenyl group, 2-fluoro-4-(2-propenyloxy)phenyl group, 2-fluoro-4-(2-butenyloxy)phenyl group, 2-fluoro-4-(3-butenyloxy)phenyl group, 2-fluoro-4-(2-pentenyloxy)phenyl group, 2-fluoro-4-(3-pentenyloxy)phenyl group, 2-fluoro-4-(4-pentenyloxy)phenyl group, 2-fluoro-4-(2-hexenyloxy)phenyl group, 2-fluoro-4-(3-hexenyloxy)phenyl group, 2-fluoro-4-(4-hexenyloxy)phenyl group, 2-fluoro-4-(5-hexenyloxy)phenyl group, 2-fluoro-4-(2-heptenyloxy)phenyl group, 2-fluoro-4-(3-heptenyloxy)phenyl group, 2-fluoro-4-(4-heptenyloxy)phenyl group, 2-fluoro-4-(5-heptenyloxy)phenyl group, 2-fluoro-4-(6-heptenyloxy)phenyl group, 2-fluoro-4-(3-chloro-2-propenyloxy)phenyl group, 2-fluoro-4-(3,3-dichloro-2-propenyloxy)phenyl group, 2-fluoro-4-(2-methyl-2-butenyloxy)phenyl group, 2-fluoro-4-(3-methyl-2-butenyloxy)phenyl group, 2-fluoro-4-(2-propynyloxy)phenyl group, 2-fluoro-4-(2-butynyloxy)phenyl group, 2-fluoro-4-(3-butynyloxy)phenyl group, 2-fluoro-4-(2-pentynyloxy)phenyl group, 2-fluoro-4-(3-pentynyloxy)phenyl group, 2-fluoro-4-(4-pentynyloxy)phenyl group, 2-fluoro-4-(2-hexynyloxy)phenyl group, 2-fluoro-4-(3-hexynyloxy)phenyl group, 2-fluoro-4-(4-hexynyloxy)phenyl group, 2-fluoro-4-(5-hexynyloxy)phenyl group, 2-fluoro-4-(2-heptynyloxy)phenyl group, 2-fluoro-4-(3-heptynyloxy)phenyl group, 2-fluoro-4-(4-heptynyloxy)phenyl group, 2-fluoro-4-(5-heptynyloxy)phenyl group, 2-fluoro-4-(6-heptynyloxy)phenyl group, 4-(2-methoxyethoxy)phenyl group, 2-fluoro-4-(3-methoxypropoxy)phenyl group, 2-fluoro-4-(4-methoxybutoxy)phenyl group, 2-fluoro-4-(5-methoxypentyloxy)phenyl group, 2-fluoro-4-(6-methoxyhexyloxy)phenyl group, 2-fluoro-4-(7-methoxyheptyloxy)phenyl group, 2-fluoro-4-(2-ethoxyethoxy)phenyl group, 2-fluoro-4-(3-ethoxypropoxy)phenyl group, 2-fluoro-4-(4-ethoxybutoxy)phenyl group, 2-fluoro-4-(5-ethoxypentyloxy)phenyl group, 2-fluoro-4-(6-ethoxyhexyloxy)phenyl group, 2-fluoro-4-(7-ethoxyheptyloxy)phenyl group, 2-fluoro-4-cyclopropylmethoxyphenyl group, 2-fluoro-4-(2-cyclopropylethoxy)phenyl group, 2-fluoro-4-(3-cyclopropylpropoxy)phenyl group, 2-fluoro-4-(4-cyclopropylbutoxy)phenyl group, 2-fluoro-4-(5-cyclopropylpentyloxy)phenyl group, 2-fluoro-4-(6-cyclopropylhexyloxy)phenyl group, 2-fluoro-4-(7-cyclopropylheptyloxy)phenyl group, 2-fluoro-4-phenoxymethylphenyl group, 2-fluoro-4-(2-phenylethyl)phenyl group, 2-fluoro-4-(3-phenylpropyl)phenyl group, 2-fluoro-4-(4-phenylbutyl)phenyl group, 2-fluoro-4-(5-phenylpentyl)phenyl group, 2-fluoro-4-(2-phenylethoxy)phenyl group, 2-fluoro-4-(3-phenylpropoxy)phenyl group, 2-fluoro-4-(4-phenylbutoxy)phenyl group, 2-fluoro-4-(5-phenylpentyloxy)phenyl group, (Z)-2-fluoro-4-styrylphenyl group, 1-benzylpyrrol-3-yl group, 1-(2-fluorophenylmethyl)pyrrol-3-yl group, 1-(3-fluorophenylmethyl)pyrrol-3-yl group, 1-(4-fluorophenylmethyl)pyrrol-3-yl group, 1-(2-phenylethyl)pyrrol-3-yl group, 1-benzylpyrazol-4-yl group, 1-phenylpyrazol-4-yl group, 2-methoxythiazol-5-yl group, 2-ethoxythiazol-5-yl group, 2-propoxythiazol-5-yl group, 2-butoxythiazol-5-yl group, 2-pentyloxythiazol-5-yl group, 2-benzyloxythiazol-5-yl group, 2-phenoxythiazol-5-yl group, 2-methylthiazol-5-yl group, 2-butylthiazol-5-yl group, 2-phenylthiazol-5-yl group and 2-benzylthiazol-5-yl group.

Examples of the present compound include preferably a compound of the formula (I) in which G is any group selected from among G¹ to G¹², E is a 2-furyl group optionally having one member selected from the substituent group e-7 or substituent group e-8, a 2-thienyl group optionally having one member selected from the substituent group e-7 or substituent group e-8, a 3-pyrrolyl group optionally having one member selected from the substituent group e-7 or substituent group e-8 or a phenyl group optionally having one member selected from the substituent group e-7 or substituent group e-8, R¹ is a hydrogen atom or amino group, R² is a hydrogen atom or a member selected from the substituent group c-5 and substituent group c-6, R³ is a hydrogen atom or halogen atom, and R⁴ is a hydrogen atom.

Examples of the present compound include compounds represented by the following formulae (2) to (133) or salts thereof or hydrates thereof. 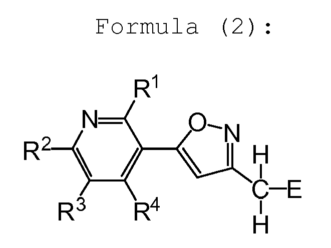 [wherein, R¹, R², R³, R⁴ and E represent the same meaning as described above.] 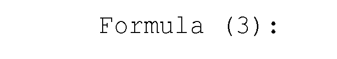 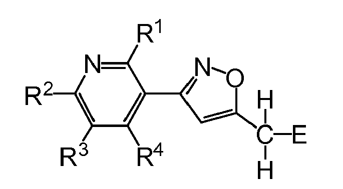 [wherein, R¹, R², R³, R⁴ and E represent the same meaning as described above.] 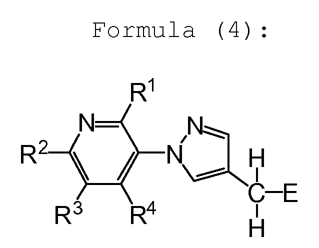 [wherein, R¹, R², R³, R⁴ and E represent the same meaning as described above.] 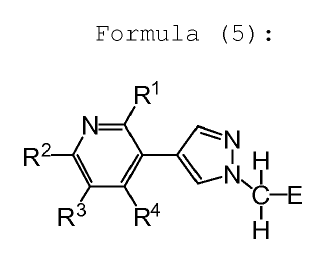 [wherein, R¹, R², R³, R⁴ and E represent the same meaning as described above.] 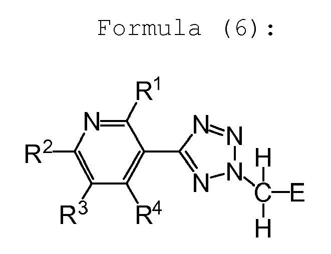 [wherein, R¹, R², R³, R⁴ and E represent the same meaning as described above.] 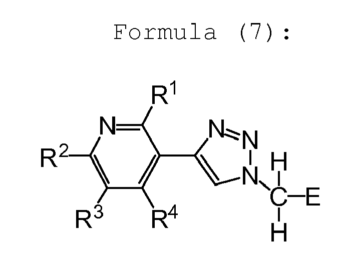 [wherein, R¹, R², R³, R⁴ and E represent the same meaning as described above.] 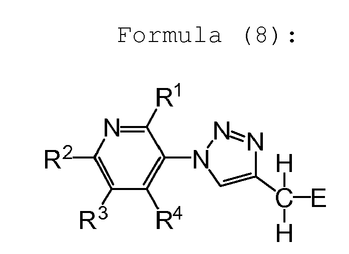 [wherein, R¹, R², R³, R⁴ and E represent the same meaning as described above.] 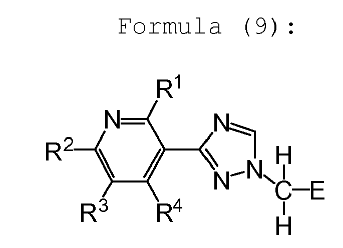 [wherein, R¹, R², R³, R⁴ and E represent the same meaning as described above.] 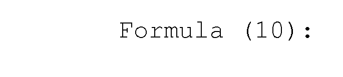 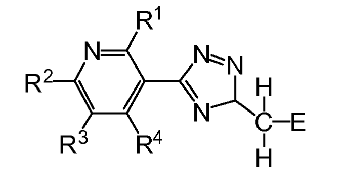 [wherein, R¹, R², R³, R⁴ and E represent the same meaning as described above.] 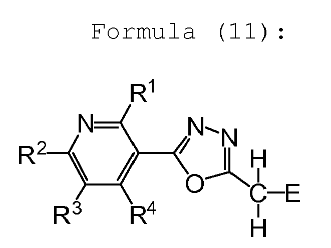 [wherein, R¹, R², R³, R⁴ and E represent the same meaning as described above.] 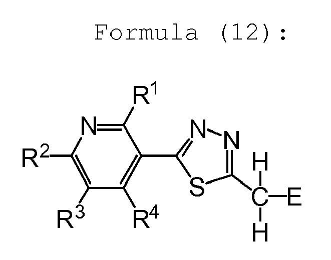 [wherein, R¹, R², R³, R⁴ and E represent the same meaning as described above.] 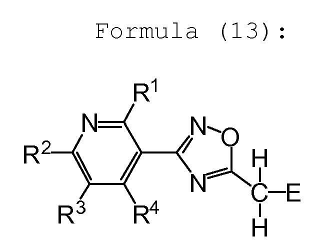 [wherein, R¹, R², R³, R⁴ and E represent the same meaning as described above.] 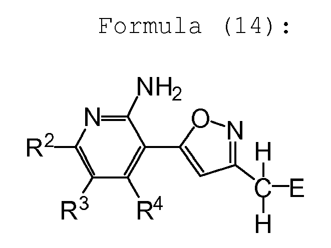 [wherein, R², R³, R⁴ and E represent the same meaning as described above.] 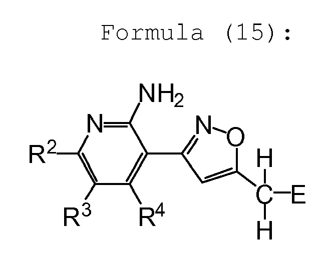 [wherein, R², R³, R⁴ and E represent the same meaning as described above.] 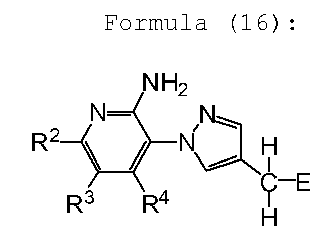 [wherein, R², R³, R⁴ and E represent the same meaning as described above.] 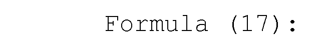 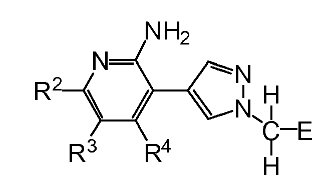 [wherein, R², R³, R⁴ and E represent the same meaning as described above.] 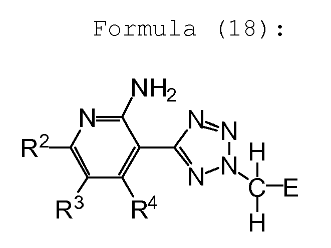 [wherein, R², R³, R⁴ and E represent the same meaning as described above.] 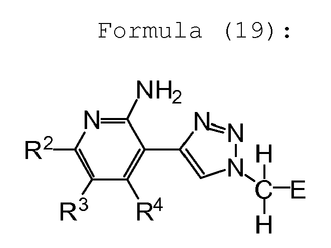 [wherein, R², R³, R⁴ and E represent the same meaning as described above.] 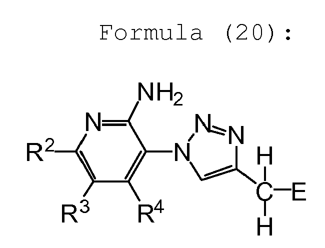 [wherein, R², R³, R⁴ and E represent the same meaning as described above.] 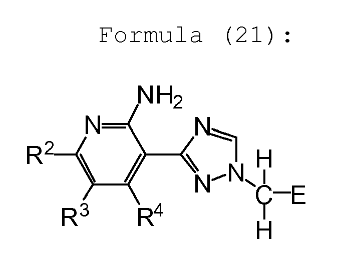 [wherein, R², R³, R⁴ and E represent the same meaning as described above.] 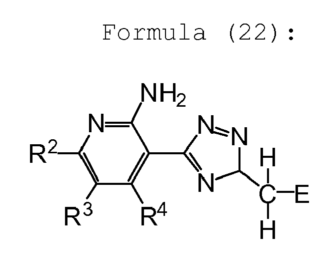 [wherein, R², R³, R⁴ and E represent the same meaning as described above.] 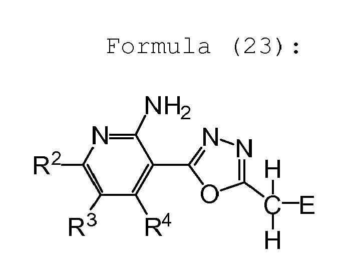 [wherein, R², R³, R⁴ and E represent the same meaning as described above.] 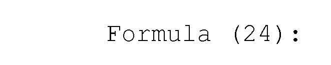 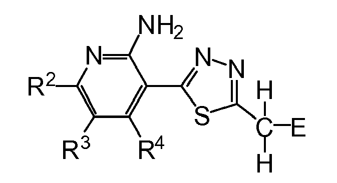 [wherein, R², R³, R⁴ and E represent the same meaning as described above.] 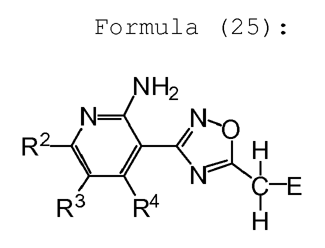 [wherein, R², R³, R⁴ and E represent the same meaning as described above.] 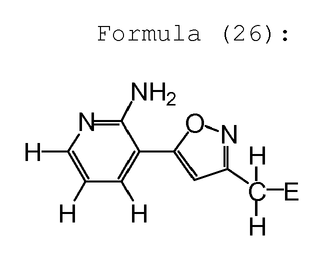 [wherein, E represents the same meaning as described above.] 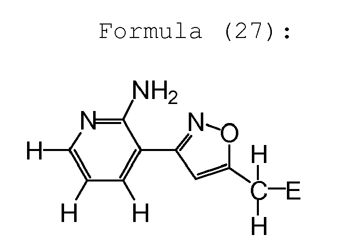 [wherein, E represents the same meaning as described above.] 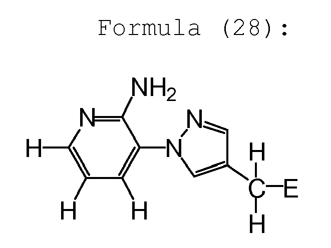 [wherein, E represents the same meaning as described above.] 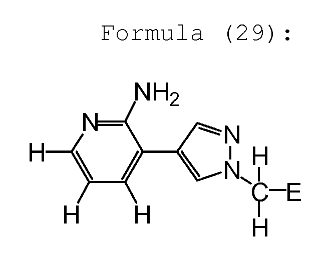 [wherein, E represents the same meaning as described above.] 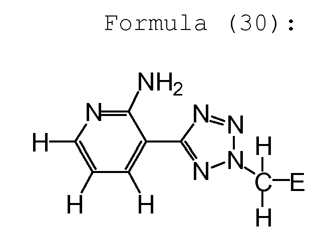 [wherein, E represents the same meaning as described above.] 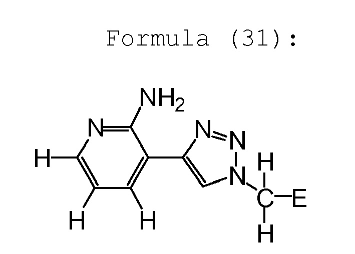 [wherein, E represents the same meaning as described above.] 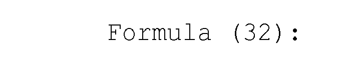 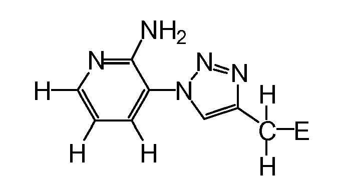 [wherein, E represents the same meaning as described above.] 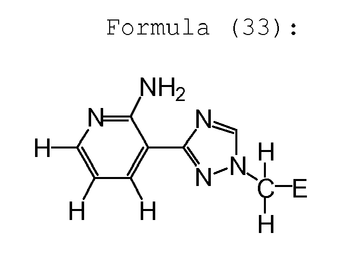 [wherein, E represents the same meaning as described above.] 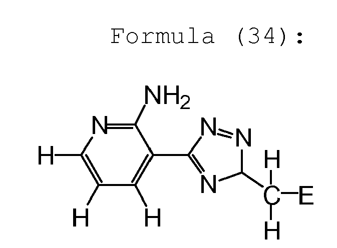 [wherein, E represents the same meaning as described above.] 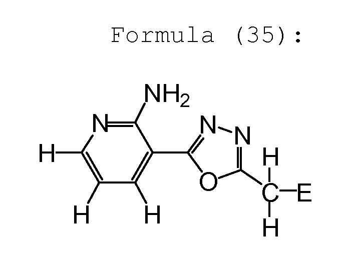 [wherein, E represents the same meaning as described above.] 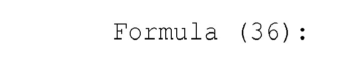 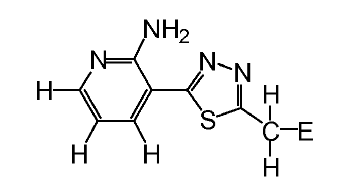 [wherein, E represents the same meaning as described above.] 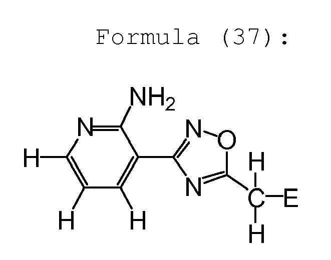 [wherein, E represents the same meaning as described above.] 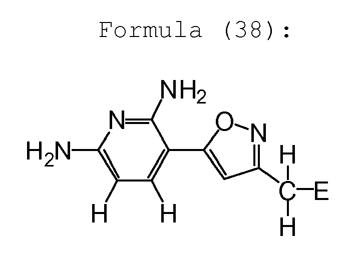 [wherein, E represents the same meaning as described above.] 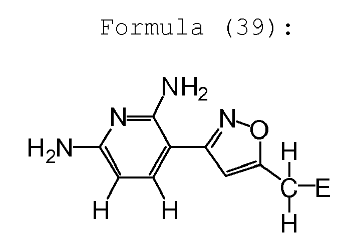 [wherein, E represents the same meaning as described above.] 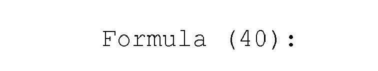 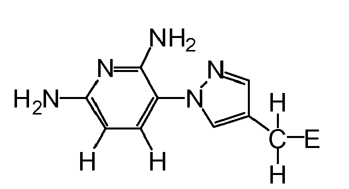 [wherein, E represents the same meaning as described above.] 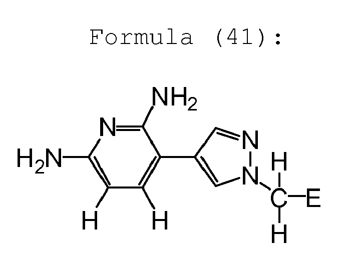 [wherein, E represents the same meaning as described above.] 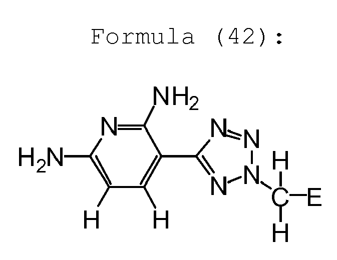 [wherein, E represents the same meaning as described above.] 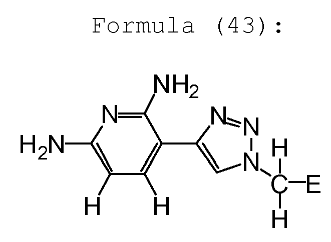 [wherein, E represents the same meaning as described above.] 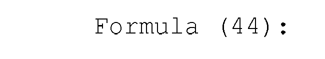 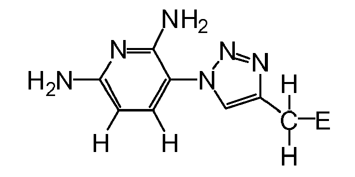 [wherein, E represents the same meaning as described above.] 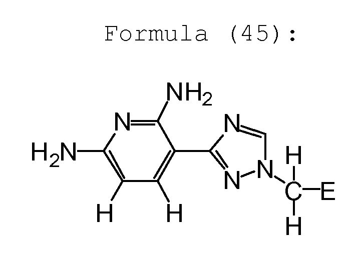 [wherein, E represents the same meaning as described above.] 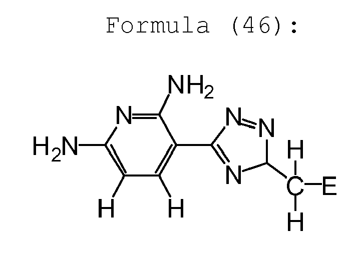 [wherein, E represents the same meaning as described above.] 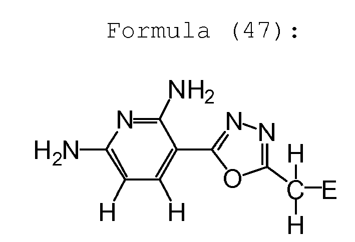 [wherein, E represents the same meaning as described above.] 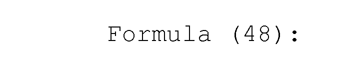 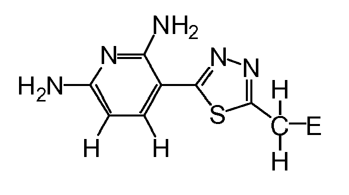 [wherein, E represents the same meaning as described above.] 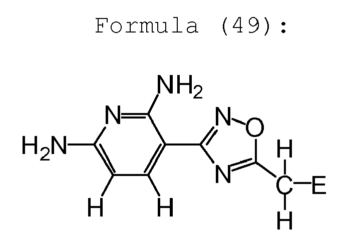 [wherein, E represents the same meaning as described above.] 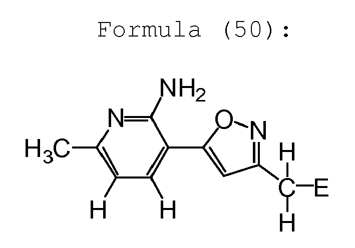 [wherein, E represents the same meaning as described above.] 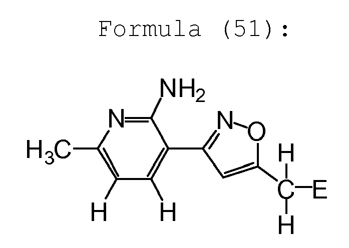 [wherein, E represents the same meaning as described above.] 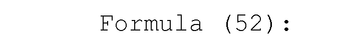 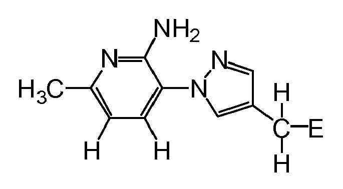 [wherein, E represents the same meaning as described above.] 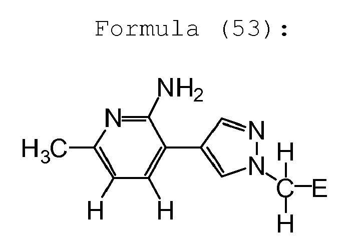 [wherein, E represents the same meaning as described above.] 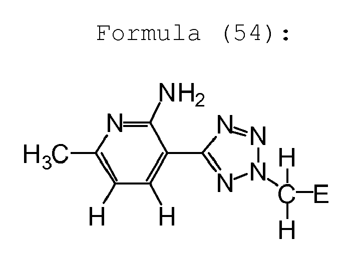 [wherein, E represents the same meaning as described above.] 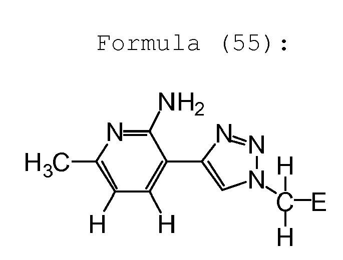 [wherein, E represents the same meaning as described above.] 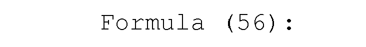 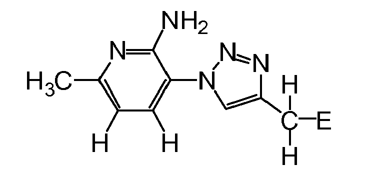 [wherein, E represents the same meaning as described above.] 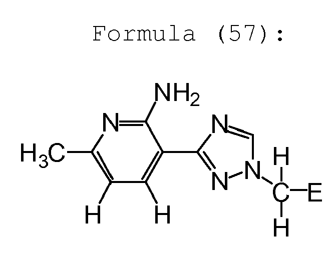 [wherein, E represents the same meaning as described above.] 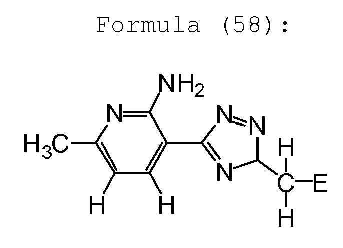 [wherein, E represents the same meaning as described above.] 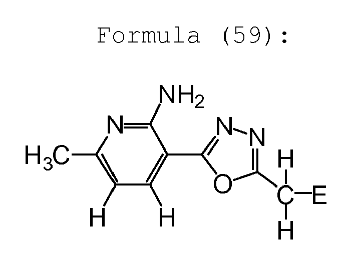 [wherein, E represents the same meaning as described above.] 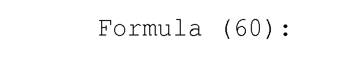 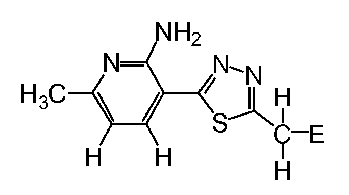 [wherein, E represents the same meaning as described above.] 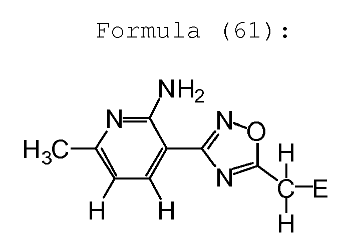 [wherein, E represents the same meaning as described above.] 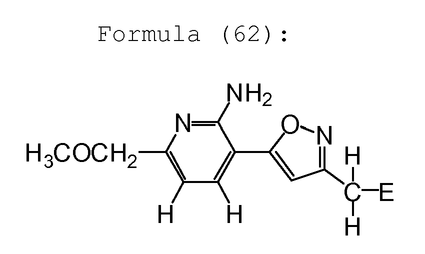 [wherein, E represents the same meaning as described above.] 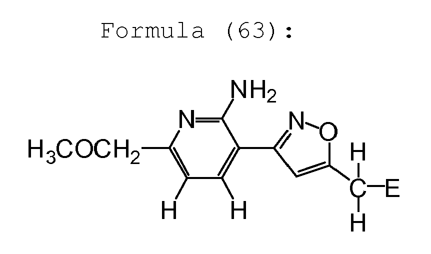 [wherein, E represents the same meaning as described above.] 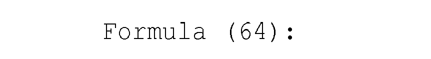 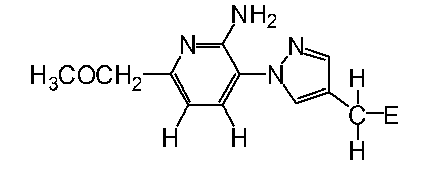 [wherein, E represents the same meaning as described above.] 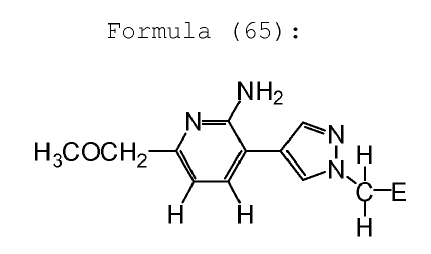 [wherein, E represents the same meaning as described above.] 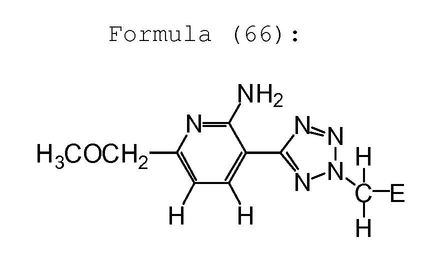 [wherein, E represents the same meaning as described above.] 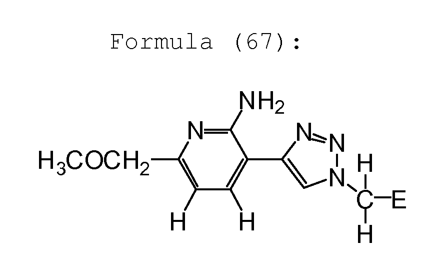 [wherein, E represents the same meaning as described above.] 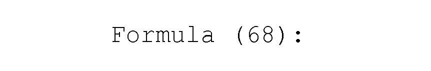 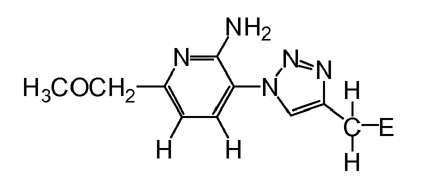 [wherein, E represents the same meaning as described above.] 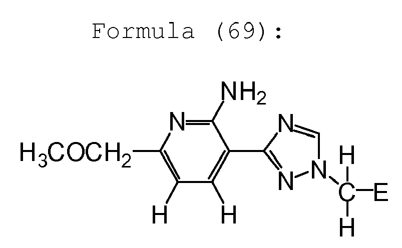 [wherein, E represents the same meaning as described above.] 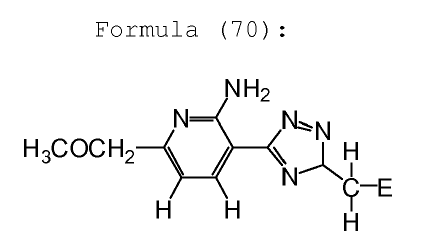 [wherein, E represents the same meaning as described above.] 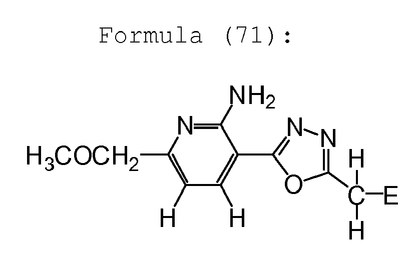 [wherein, E represents the same meaning as described above.] 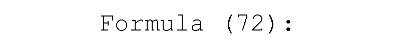 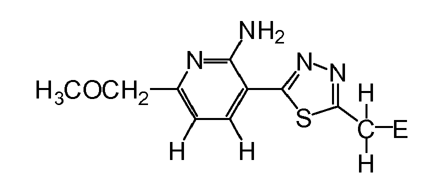 [wherein, E represents the same meaning as described above.] 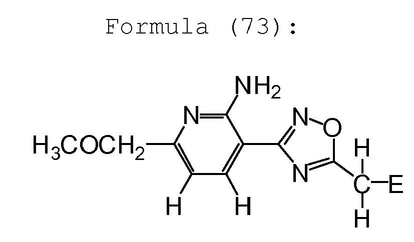 [wherein, E represents the same meaning as described above.] 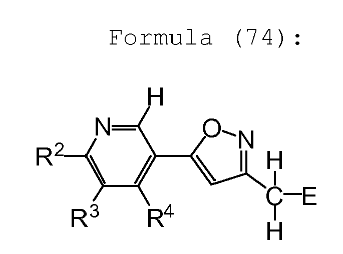 [wherein, R², R³, R⁴ and E represent the same meaning as described above.] 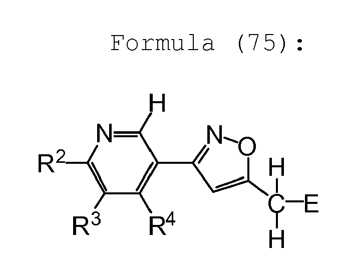 [wherein, R², R³, R⁴ and E represent the same meaning as described above.] 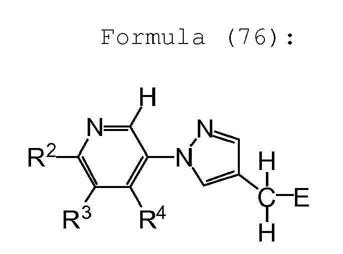 [wherein, R², R³, R⁴ and E represent the same meaning as described above.] 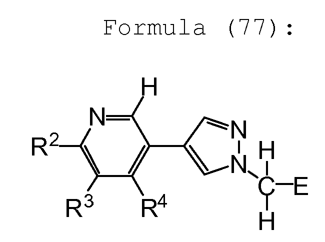 [wherein, R², R³, R⁴ and E represent the same meaning as described above.] 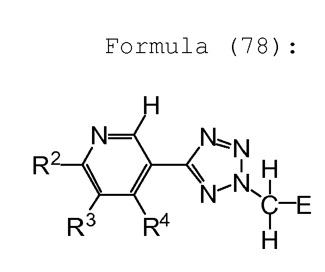 [wherein, R², R³, R⁴ and E represent the same meaning as described above.] 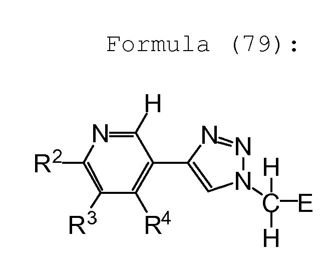 [wherein, R², R³, R⁴ and E represent the same meaning as described above.] 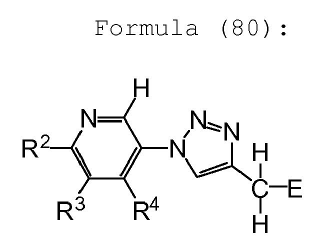 [wherein, R², R³, R⁴ and E represent the same meaning as described above.] 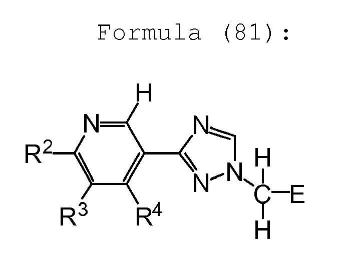 [wherein, R², R³, R⁴ and E represent the same meaning as described above.] 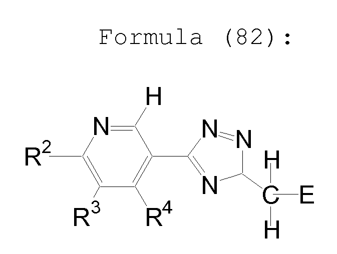 [wherein, R², R³, R⁴ and E represent the same meaning as described above.] 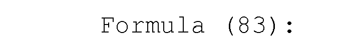 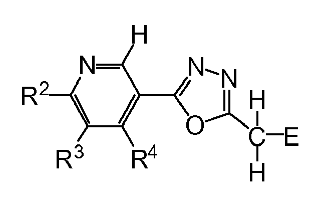 [wherein, R², R³, R⁴ and E represent the same meaning as described above.] 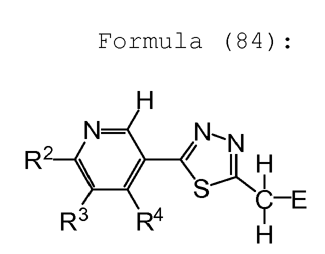 [wherein, R², R³, R⁴ and E represent the same meaning as described above.] 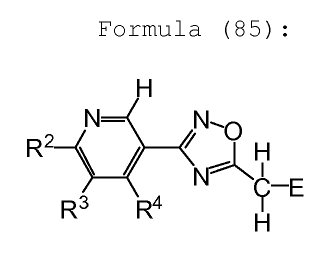 [wherein, R², R³, R⁴ and E represent the same meaning as described above.] 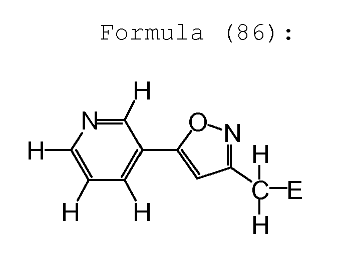 [wherein, E represents the same meaning as described above.] 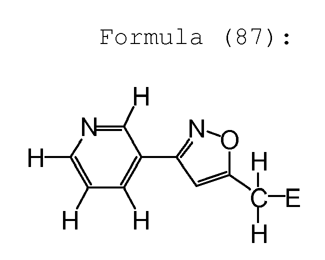 [wherein, E represents the same meaning as described above.] 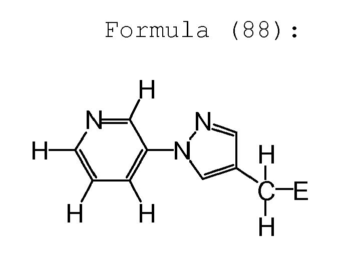 [wherein, E represents the same meaning as described above.] 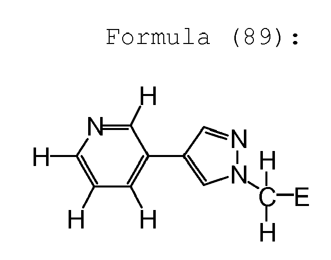 [wherein, E represents the same meaning as described above.] 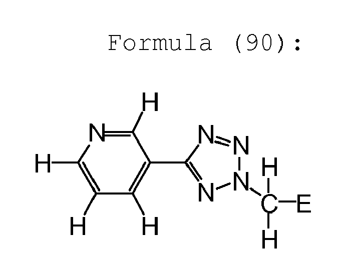 [wherein, E represents the same meaning as described above.] 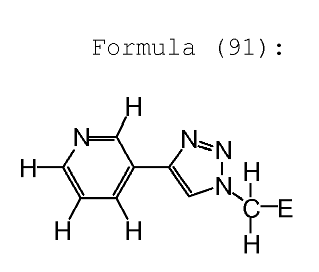 [wherein, E represents the same meaning as described above.] 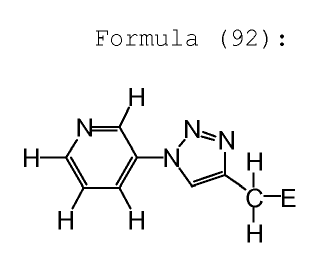 [wherein, E represents the same meaning as described above.] 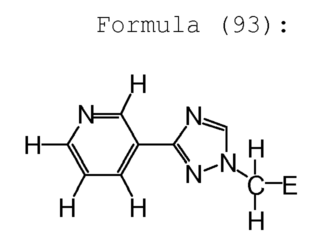 [wherein, E represents the same meaning as described above.] 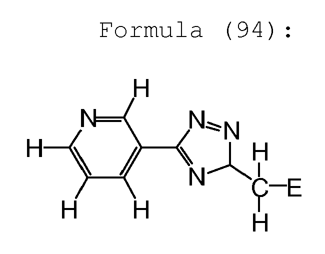 [wherein, E represents the same meaning as described above.] 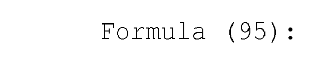 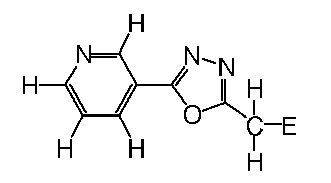 [wherein, E represents the same meaning as described above.] 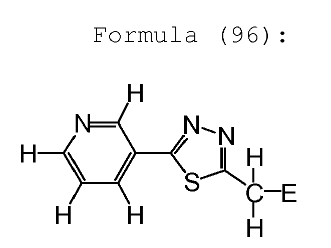 [wherein, E represents the same meaning as described above.] 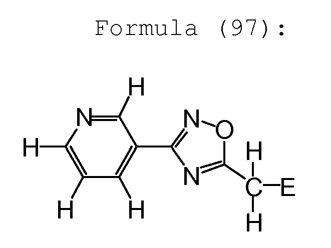 [wherein, E represents the same meaning as described above.] 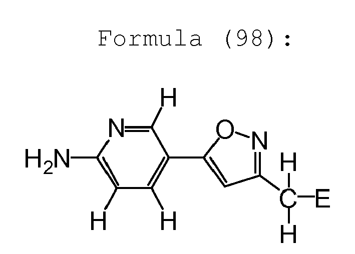 [wherein, E represents the same meaning as described above.] 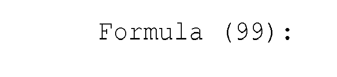 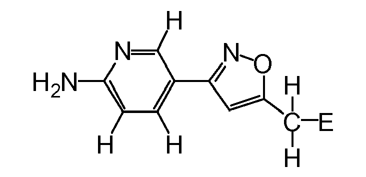 [wherein, E represents the same meaning as described above.] 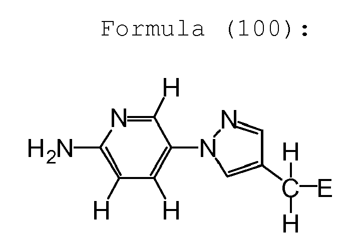 [wherein, E represents the same meaning as described above.] 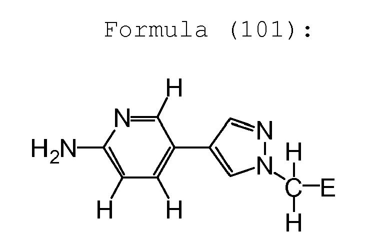 [wherein, E represents the same meaning as described above.] 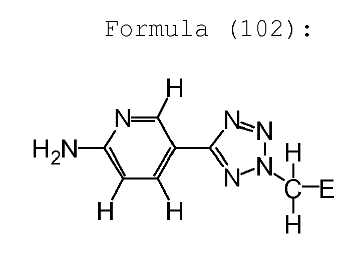 [wherein, E represents the same meaning as described above.] 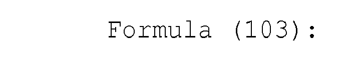 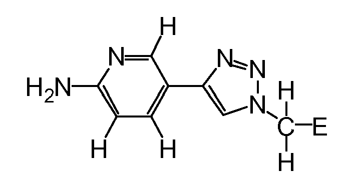 [wherein, E represents the same meaning as described above.] 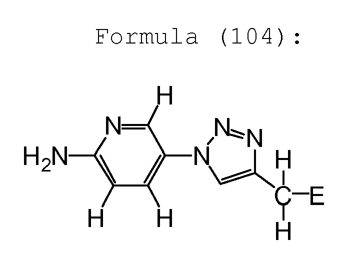 [wherein, E represents the same meaning as described above.] 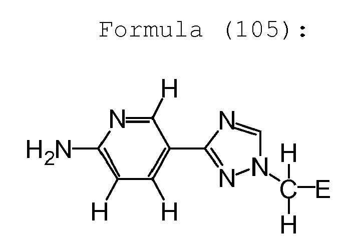 [wherein, E represents the same meaning as described above.] 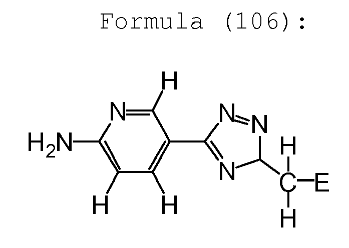 [wherein, E represents the same meaning as described above.] 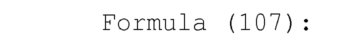 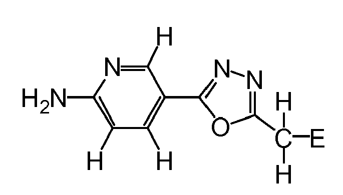 [wherein, E represents the same meaning as described above.] 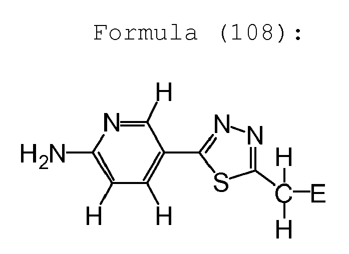 [wherein, E represents the same meaning as described above.] 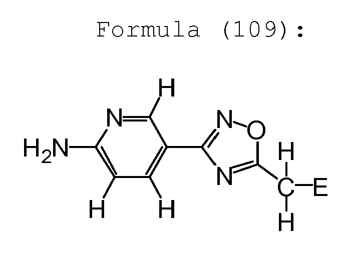 [wherein, E represents the same meaning as described above.] 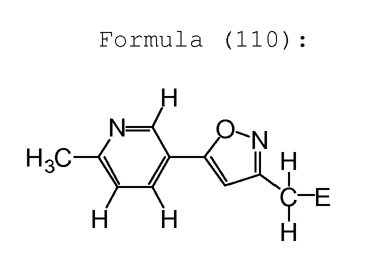 [wherein, E represents the same meaning as described above.] 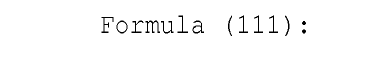 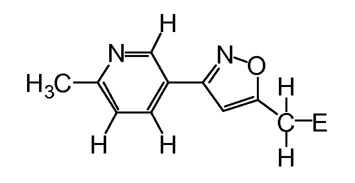 [wherein, E represents the same meaning as described above.] 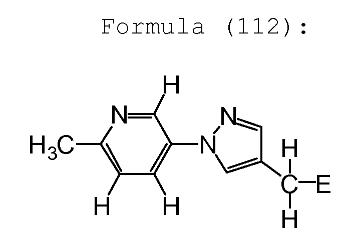 [wherein, E represents the same meaning as described above.] 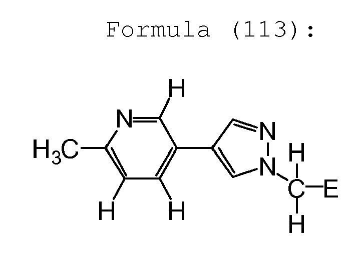 [wherein, E represents the same meaning as described above.] 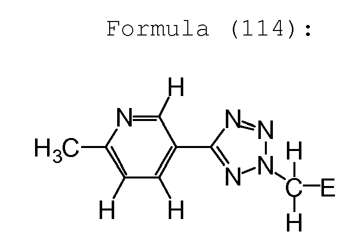 [wherein, E represents the same meaning as described above.] 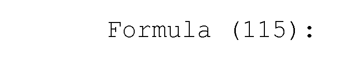 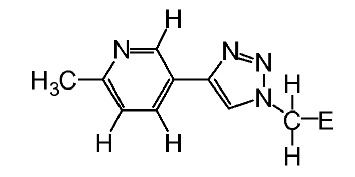 [wherein, E represents the same meaning as described above.] 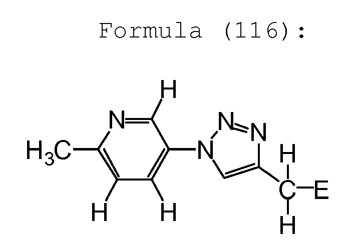 [wherein, E represents the same meaning as described above.] 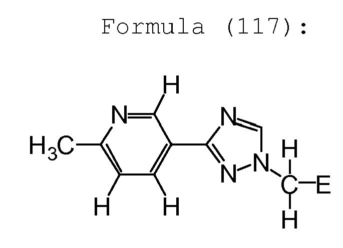 [wherein, E represents the same meaning as described above.] 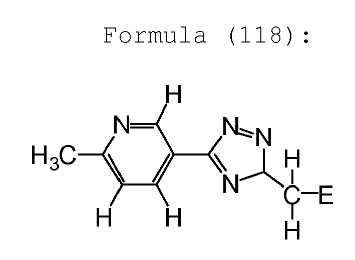 [wherein, E represents the same meaning as described above.] 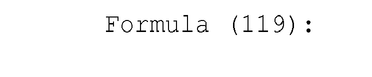 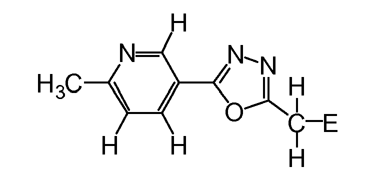 [wherein, E represents the same meaning as described above.] 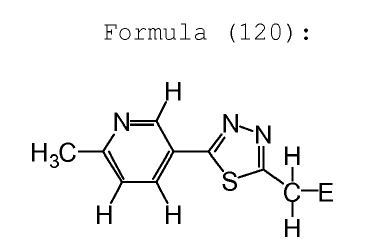 [wherein, E represents the same meaning as described above.] 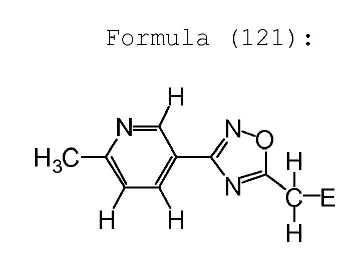 [wherein, E represents the same meaning as described above.] 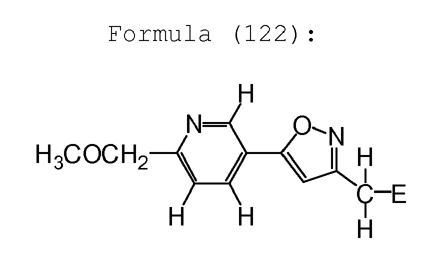 [wherein, E represents the same meaning as described above.] 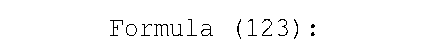 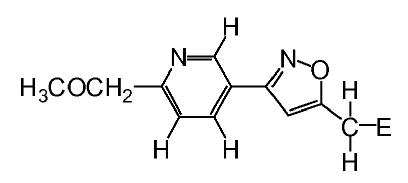 [wherein, E represents the same meaning as described above.] 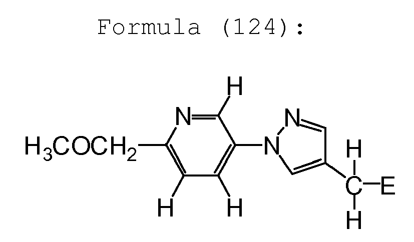 [wherein, E represents the same meaning as described above.] 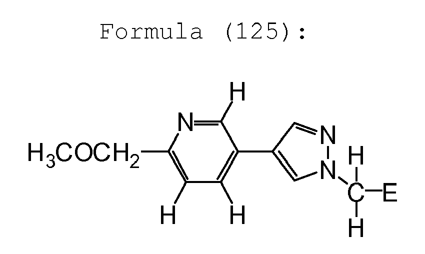 [wherein, E represents the same meaning as described above.] 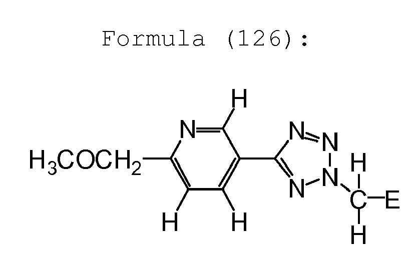 [wherein, E represents the same meaning as described above.] 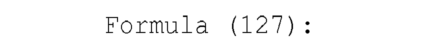 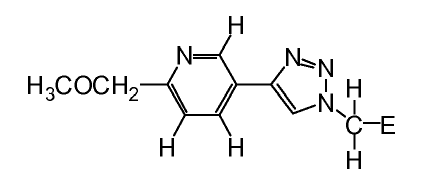 [wherein, E represents the same meaning as described above.] 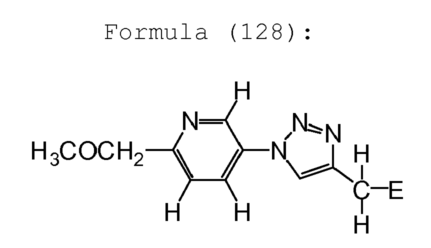 [wherein, E represents the same meaning as described above.] 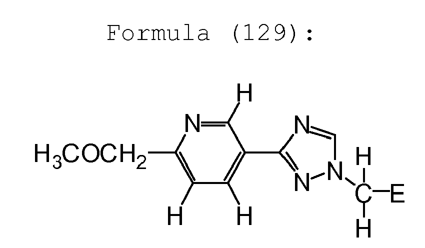 [wherein, E represents the same meaning as described above.] 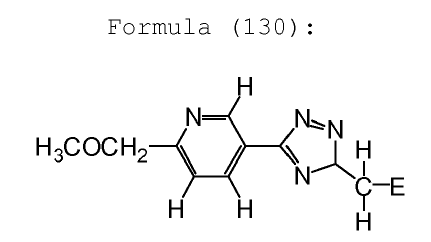 [wherein, E represents the same meaning as described above.] 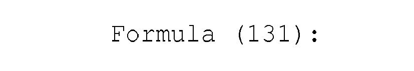 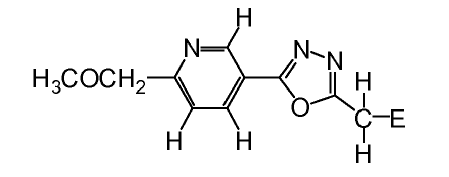 [wherein, E represents the same meaning as described above.] 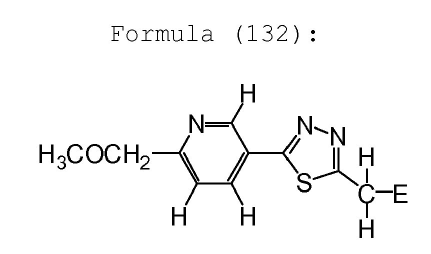 [wherein, E represents the same meaning as described above.] 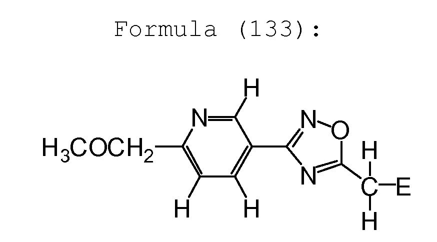 [wherein, E represents the same meaning as described above.]

Specific examples of the present compound will be shown below.

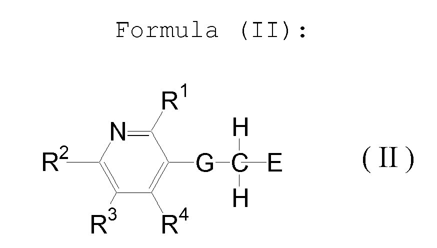

Compounds of the formula (II) in which G is G¹ and R¹, R², R³, R⁴ and E are moieties shown in line numbers 1 to 454 of Table 1 are expressed as the present compounds 1 to 454.

Compounds of the formula (II) in which G is G² and R¹, R², R³, R⁴ and E are moieties shown in line numbers 1 to 454 of Table 1 are expressed as the present compounds 501 to 954.

Compounds of the formula (II) in which G is G³ and R¹, R², R³, R⁴ and E are moieties shown in line numbers 1 to 454 of Table 1 are expressed as the present compounds 1001 to 1454.

Compounds of the formula (II) in which G is G⁴ and R¹, R², R³, R⁴ and E are moieties shown in line numbers 1 to 454 of Table 1 are expressed as the present compounds 1501 to 1954.

Compounds of the formula (II) in which G is G⁵ and R¹, R², R³, R⁴ and E are moieties shown in line numbers 1 to 454 of Table 1 are expressed as the present compounds 2001 to 2454.

Compounds of the formula (II) in which G is G⁶ and R¹, R², R³, R⁴ and E are moieties shown in line numbers 1 to 454 of Table 1 are expressed as the present compounds 2501 to 2954.

Compounds of the formula (II) in which G is G⁷ and R¹, R², R³, R⁴ and E are moieties shown in line numbers 1 to 454 of Table 1 are expressed as the present compounds 3001 to 3454.

Compounds of the formula (II) in which G is G⁸ and R¹, R², R³, R⁴ and E are moieties shown in line numbers 1 to 454 of Table 1 are expressed as the present compounds 3501 to 3954.

Compounds of the formula (II) in which G is G⁹ and R¹, R², R³, R⁴ and E are moieties shown in line numbers 1 to 454 of Table 1 are expressed as the present compounds 4001 to 4454.

Compounds of the formula (II) in which G is G¹⁰ and R¹, R², R³, R⁴ and E are moieties shown in line numbers 1 to 454 of Table 1 are expressed as the present compounds 4501 to 4954.

Compounds of the formula (II) in which G is G¹¹ and R¹, R², R³, R⁴ and E are moieties shown in line numbers 1 to 454 of Table 1 are expressed as the present compounds 5001 to 5454.

Compounds of the formula (II) in which G is G¹² and R¹, R², R³, R⁴ and E are moieties shown in line numbers 1 to 454 of Table 1 are expressed as the present compounds 5501 to 5954.

**Table 1**

| line number | R¹ | R² | R³ | R⁴ | E |
|---|---|---|---|---|---|
| 1 | NH2 | H | H | H | phenyl |
| 2 | NH2 | H | H | H | 4-fluorophenyl |
| 3 | NH2 | H | H | H | 4-phenoxyphenyl |
| 4 | NH2 | H | H | H | 4-biphenyl |
| 5 | NH2 | H | H | H | 3-biphenyl |
| 6 | NH2 | H | H | H | 3-phenoxyphenyl |
| 7 | NH2 | H | H | H | 3-benzyloxyphenyl |
| 8 | NH2 | H | H | H | 3-methoxyphenyl |
| 9 | NH2 | H | H | H | 4-methoxyphenyl |
| 10 | NH2 | H | H | H | 4-pentylphenyl |
| 11 | NH2 | H | H | H | 3-pentylphenyl |
| 12 | NH2 | NH2 | H | H | 2-fluorophenyl |
| 13 | NH2 | NH2 | H | | 2-fluoro-3-methoxyphenyl |
| 14 | NH2 | NH2 | H | H | 4-benzyloxyphenyl |
| 15 | NH2 | NH2 | H | H | 3-phenoxyphenyl |
| 16 | NH2 | NH2 | H | H | 4-(4-fluorophenoxy)phenyl |
| 17 | NH2 | NH2 | H | H | 4-phenoxyphenyl |
| 18 | NH2 | H | H | H | 2-fluoro-3-methoxyphenyl |
| 19 | NH2 | F | H | H | 4-(4-fluorophenoxy)phenyl |
| 20 | NH2 | H | H | H | 3-(3-methylphenoxy)phenyl |
| 21 | NH2 | H | H | H | 4-(4-methylphenoxy)phenyl |
| 22 | NH2 | H | H | H | 3-phenoxymethylphenyl |
| 23 | NH2 | H | H | H | 4-phenoxymethylphenyl |
| 24 | NH2 | H | H | H | 3-(2-phenylethyl)phenyl |
| 25 | NH2 | H | H | H | 4-(2-phenylethyl)phenyl |
| 26 | NH2 | H | H | H | 3-(2-phenylethoxy)phenyl |
| 27 | NH2 | H | H | H | 4-(2-phenylethoxy)phenyl |
| 28 | NH2 | H | H | H | 4-(3-fluorophenyl)methoxyphenyl |
| 29 | NH2 | CH3 | H | H | 4-phenoxyphenyl |
| 30 | NH2 | NH2 | H | H | 4-(3-chlorophenyl)methoxyphenyl |
| 31 | NH2 | H | H | H | 4-benzylphenyl |
| 32 | NH2 | H | H | H | 4-(4-fluorophenyl)methoxyphenyl |
| 33 | NH2 | H | H | H | 4-fluoro-3-phenoxyphenyl |
| 34 | NH2 | H | H | H | 4-(4-fluorophenoxy)phenyl |
| 35 | NH2 | H | H | H | 5-phenoxyfuran-2-yl |
| 36 | NH2 | H | H | H | 5-(3-fluorophenyl)methylfuran-2-yl |
| 37 | NH2 | H | H | H | 5-phenoxythiophen-2-yl |
| 38 | NH2 | NH2 | H | H | 5-phenoxythiophen-2-yl |
| 39 | NH2 | H | H | H | 5-(3-fluorophenoxy)thiophen-2-yl |
| 40 | NH2 | H | H | H | 5-benzylthiophen-2-yl |
| 41 | NH2 | H | H | H | 5-(3-chlorophenyl)methylthiophen-2-yl |
| 42 | NH2 | H | H | H | 2-phenoxythiazol-5-yl |
| 43 | NH2 | H | H | H | 2-benzyloxythiazol-5-yl |
| 44 | NH2 | H | H | H | 2-butoxythiazol-5-yl |
| 45 | NH2 | Cl | H | H | 4-benzyloxyphenyl |
| 46 | NH2 | F | H | H | 4-phenoxyphenyl |
| 47 | NH2 | H | H | H | 4-(2-methoxyphenoxy)phenyl |
| 48 | NH2 | H | H | H | 4-(3-methoxyphenoxy)phenyl |
| 49 | NH2 | H | H | H | 4-(4-methoxyphenoxy)phenyl |
| 50 | NH2 | H | H | H | 3-(2-methoxyphenoxy)phenyl |
| 51 | NH2 | H | H | H | 3-(3-methoxyphenoxy)phenyl |
| 52 | NH2 | H | H | H | 3-(4-methoxyphenoxy)phenyl |
| 53 | NH2 | Cl | F | H | 4-phenoxyphenyl |
| 54 | NH2 | NH2 | F | H | 4-phenoxyphenyl |
| 55 | NH2 | H | H | H | 2-fluoro-4-benzyloxyphenyl |
| 56 | NH2 | H | H | H | 4-(2-nitrophenyl)methoxyphenyl |
| 57 | NH2 | H | H | H | 4-(2-fluorophenyl)methoxyphenyl |
| 58 | NH2 | H | H | H | 4-(2-methylphenyl)methoxyphenyl |
| 59 | NH2 | H | H | H | 4-(3-methylphenyl)methoxyphenyl |
| 60 | NH2 | H | H | H | 4-(4-methylphenyl)methoxyphenyl |
| 61 | NH2 | Cl | H | H | 4-phenoxyphenyl |
| 62 | NH2 | CH2OCH3 | H | H | 4-phenoxyphenyl |
| 63 | NH2 | H | Br | H | 4-phenoxyphenyl |
| 64 | NH2 | H | H | H | 4-ethoxyphenyl |
| 65 | NH2 | H | H | H | 4-butoxyphenyl |
| 66 | NH2 | H | H | H | 4-pentyloxyphenyl |
| 67 | NH2 | H | H | H | 4-heptyloxyphenyl |
| 68 | NH2 | H | H | H | 4-hydroxyphenyl |
| 69 | NH2 | H | H | H | 1-(3-fluorophenyl)methylpyrrol-3-yl |
| 70 | NH2 | H | H | H | furan-2-yl |
| 71 | NH2 | H | H | H | 4-(3-methoxypropoxy)phenyl |
| 72 | NH2 | H | H | H | 4-(5-methoxypentyloxy)phenyl |
| 73 | NH2 | H | H | H | 4-(6-methoxyhexyloxy)phenyl |
| 74 | NH2 | H | H | H | 4-(7-methoxyheptyloxy)phenyl |
| 75 | NH2 | H | H | H | 4-(2-ethoxyethoxy)phenyl |
| 76 | NH2 | H | H | H | 4-(4-ethoxybutoxy)phenyl |
| 77 | NH2 | CH3 | H | H | 4-benzyloxyphenyl |
| 78 | NH2 | H | Cl | H | 4-phenoxyphenyl |
| 79 | NH2 | CH3 | H | H | 4-(3-methoxyphenoxy)phenyl |
| 80 | NH2 | CH3 | H | H | 3-(4-methoxyphenoxy)phenyl |
| 81 | NH2 | CH3 | H | H | 4-(2-phenylethyl)phenyl |
| 82 | NH2 | CH3 | H | H | 4-(4-fluorophenoxy)phenyl |
| 83 | NH2 | CH3 | H | H | 5-phenoxythiophen-2-yl |
| 84 | NH2 | CH3 | H | H | 4-benzylphenyl |
| 85 | NH2 | H | H | H | 4-(4-pentynyloxy)phenyl |
| 86 | NH2 | H | H | H | 4-(5-hexynyloxy)phenyl |
| 87 | NH2 | H | H | H | 4-(2-chlorophenylmethoxy)phenyl |
| 88 | NH2 | H | H | H | 4-(3-chlorophenylmethoxy)phenyl |
| 89 | NH2 | H | H | H | 4-(2-methoxyphenylmethoxy)phenyl |
| 90 | NH2 | H | H | H | 4-(3-methoxyphenylmethoxy)phenyl |
| 91 | NH2 | H | H | H | 4-(4-methoxyphenylmethoxy)phenyl |
| 92 | NH2 | CH3 | H | H | 3-phenoxyphenyl |
| 93 | NH2 | CH2OCH3 | H | H | 2-fluorophenyl |
| 94 | NH2 | CH3 | H | H | 2-fluoro-3-methoxyphenyl |
| 95 | NH2 | CH3 | H | H | 3-methoxyphenyl |
| 96 | NH2 | CH3 | H | H | 4-(4-methylphenoxy)phenyl |
| 97 | NH2 | CH3 | H | H | 3-(3-methylphenoxy)phenyl |
| 98 | NH2 | CH3 | H | H | 3-pentylphenyl |
| 99 | NH2 | CH3 | H | H | 4-pentylphenyl |
| 100 | NH2 | CH3 | H | H | 4-phenoxymethylphenyl |
| 101 | NH2 | Cl | H | H | 2-fluorophenyl |
| 102 | NH2 | H | H | H | 4-(2-propynyloxy)phenyl |
| 103 | NH2 | H | H | H | 4-(2-propenyloxy)phenyl |
| 104 | NH2 | H | H | H | 3-fluorophenyl |
| 105 | NH2 | H | H | H | 3-methylphenyl |
| 106 | NH2 | H | H | H | 4-methylphenyl |
| 107 | NH2 | H | H | H | 4-ethylphenyl |
| 108 | NH2 | H | H | H | 2-methylphenyl |
| 109 | NH2 | H | H | H | 4-butylphenyl |
| 110 | NH2 | H | H | H | 4-hexylphenyl |
| 111 | NH2 | H | H | H | 4-heptylphenyl |
| 112 | NH2 | H | H | H | 3-fluoro-4-phenoxyphenyl |
| 113 | NH2 | H | H | H | 4-((3,5-difluorophenyl)methoxy)phenyl |
| 114 | NH2 | H | H | H | 4-((2-fluoro-3-methoxyphenyl)methoxy)phenyl |
| 115 | NH2 | H | H | H | 4-(3-chloro-4-fluorophenoxy)phenyl |
| 116 | NH2 | H | H | H | 4-(4-chloro-3-ethylphenoxy)phenyl |
| 117 | NH2 | H | H | H | 4-(2,4-dimethylphenoxy)phenyl |
| 118 | NH2 | H | H | H | 4-(3,4-dimethylphenoxy)phenyl |
| 119 | NH2 | H | H | H | 4-(2,4,6-trimethylphenoxy)phenyl |
| 120 | NH2 | H | H | H | 4-(2,3,6-trimethylphenoxy)phenyl |
| 121 | NH2 | H | H | H | 4-(3-ethylphenoxy)phenyl |
| 122 | NH2 | H | H | H | 4-(3-(1-methylethyl)phenoxy)phenyl |
| 123 | NH2 | H | H | H | 4-(4-propylphenoxy)phenyl |
| 124 | NH2 | H | H | H | 4-(4-butylphenoxy)phenyl |
| 125 | NH2 | H | H | H | 4-(2-chlorophenoxy)phenyl |
| 126 | NH2 | H | H | H | 4-(3,5-dichlorophenoxy)phenyl |
| 127 | NH2 | H | H | H | 4-(3,4-dichlorophenoxy)phenyl |
| 128 | NH2 | H | H | H | 4-(2-chloro-4,5-dimethylphenoxy)phenyl |
| 129 | NH2 | H | H | H | thiophen-2-yl |
| 130 | NH2 | H | H | H | 4- (4-trifluoromethoxyphenoxy)phenyl |
| 131 | NH2 | H | H | H | 4-(2,5-dimethylphenoxy)phenyl |
| 132 | NH2 | H | H | H | 4-(4-trifluoromethylphenoxy)phenyl |
| 133 | NH2 | H | H | H | 3-(4-methylphenoxy)phenyl |
| 134 | NH2 | H | H | H | 4-(3-methylphenoxy)phenyl |
| 135 | NH2 | H | H | H | 3-ethylphenyl |
| 136 | NH2 | H | H | H | 3-propylphenyl |
| 137 | NH2 | H | H | H | 3-butylphenyl |
| 138 | NH2 | H | H | H | 3-hexylphenyl |
| 139 | NH2 | H | H | H | 3-(2-methylpropyl)phenyl |
| 140 | NH2 | H | H | H | 3-[(2-fluorophenyl)methoxy]phenyl |
| 141 | NH2 | H | H | H | 3-[(3-fluorophenyl)methoxy]phenyl |
| 142 | NH2 | H | H | H | 3-[(4-fluorophenyl)methoxy]phenyl |
| 143 | NH2 | H | H | H | 3-[(2-methylphenyl)methoxy]phenyl |
| 144 | NH2 | H | H | H | 3-[(3-methylphenyl)methoxy]phenyl |
| 145 | NH2 | H | H | H | 3-[(2-methoxyphenyl)methoxy]phenyl |
| 146 | NH2 | H | H | H | 3-[(3-methoxyphenyl)methoxy]phenyl |
| 147 | NH2 | H | H | H | 3-[(4-methoxyphenyl)methoxy]phenyl |
| 148 | NH2 | H | H | H | 4-(4-ethylphenoxy)phenyl |
| 149 | NH2 | H | H | H | 4-(5,6,7,8-tetrahydronaphthalen-2-yloxy)phenyl |
| 150 | NH2 | H | H | H | 4-(3-methylphenylthio)phenyl |
| 151 | NH2 | H | H | H | 4-(3-methoxyphenylthio)phenyl |
| 152 | NH2 | H | H | H | 4-(4-methoxyphenylthio)phenyl |
| 153 | NH2 | H | H | H | 4-(4-methylthiophenoxy)phenyl |
| 154 | NH2 | H | H | H | 4-(2-methyl-4-methylthiophenoxy)phenyl |
| 155 | NH2 | H | H | H | 4-(2,3-dimethyl-4-methylthiophenoxy)phenyl |
| 156 | NH2 | H | H | H | 4-(4-chloro-3,5-dimethylphenoxy)phenyl |
| 157 | NH2 | H | H | H | 4-(3-ethyl-4-methylthiophenoxy)phenyl |
| 158 | NH2 | H | H | H | 3-(4-methylthiophenoxy)phenyl |
| 159 | NH2 | H | H | H | 4-(3-chloro-4-methylthiophenoxy)phenyl |
| 160 | NH2 | H | H | H | 4-(3-chloro-4-ethylthiophenoxy)phenyl |
| 161 | NH2 | H | H | H | 4-(3-methoxy-4-methylthiophenoxy)phenyl |
| 162 | NH2 | H | H | H | 4-(2,6-dimethylphenoxy)phenyl |
| 163 | NH2 | H | H | H | 4-(4-bromophenoxy)phenyl |
| 164 | NH2 | H | H | H | 3-ethoxyphenyl |
| 165 | NH2 | H | H | H | 3-propoxyphenyl |
| 166 | NH2 | H | H | H | 3-butoxyphenyl |
| 167 | NH2 | H | H | H | 3-pentyloxyphenyl |
| 168 | NH2 | H | H | H | 3-hexyloxyphenyl |
| 169 | NH2 | H | H | H | 3-heptyloxyphenyl |
| 170 | NH2 | H | H | H | 3-(4-pentynyloxy)phenyl |
| 171 | NH2 | H | H | H | 4-(5-hexynyloxy)phenyl |
| 172 | NH2 | H | H | H | 3-(2-butynyloxy)phenyl |
| 173 | NH2 | H | H | H | 3-(2-pentynyloxy)phenyl |
| 174 | NH2 | H | H | H | 4-(3-chlorophenoxy)phenyl |
| 175 | NH2 | H | H | H | 4-(3-fluorophenoxy)phenyl |
| 176 | NH2 | CH3 | H | H | 2-fluoro-3-(4-pentynyloxy)phenyl |
| 177 | NH2 | H | H | H | 4-(3-trifluoromethylphenoxy)phenyl |
| 178 | NH2 | H | H | H | 4-(3-trifluoromethoxyphenoxy)phenyl |
| 179 | NH2 | H | H | H | 4-(2-methylphenoxy)phenyl |
| 180 | NH2 | H | H | H | 4-(2-fluorophenoxy)phenyl |
| 181 | NH2 | H | H | H | 5-(2-methylphenoxy)thiophen-2-yl |
| 182 | NH2 | H | H | H | 4-(2-trifluoromethylphenoxy)phenyl |
| 183 | NH2 | H | H | H | 4-(4-chlorophenoxy)phenyl |
| 184 | NH2 | CH3 | H | CH3 | 4-phenoxyphenyl |
| 185 | NH2 | H | H | H | 4-(4-ethoxyphenoxy)phenyl |
| 186 | NH2 | H | H | H | 4-(4-propoxyphenoxy)phenyl |
| 187 | NH2 | H | H | H | 4-(3-ethoxyphenoxy)phenyl |
| 188 | NH2 | H | H | H | 4-(3-butoxyphenoxy)phenyl |
| 189 | NH2 | H | H | H | 4-(indan-5-yloxy)phenyl |
| 190 | NH2 | H | H | H | 4-(benzo[1,3]dioxol-5-yloxy)phenyl |
| 191 | NH2 | H | H | H | 4-(naphthalen-2-yloxy)phenyl |
| 192 | NH2 | H | H | H | 4-(pyridin-3-yloxy)phenyl |
| 193 | NH2 | H | H | H | 3-(4-methoxybutoxy)phenyl |
| 194 | NH2 | H | H | H | 3-(4-ethoxybutoxy)phenyl |
| 195 | NH2 | H | H | H | 3-(3-ethoxypropoxy)phenyl |
| 196 | NH2 | H | H | H | 3-benzylphenyl |
| 197 | NH2 | CH3 | H | H | 4-(3-methylphenoxy)phenyl |
| 198 | NH2 | H | H | H | 3-(4-pentenyloxy)phenyl |
| 199 | NH2 | H | H | H | 3-(5-hexenyloxy)phenyl |
| 200 | NH2 | H | H | H | 3-(2-methylphenoxy)phenyl |
| 201 | NH2 | H | H | H | 3-(2-fluorophenoxy)phenyl |
| 202 | NH2 | H | H | H | 3-(2-chlorophenoxy)phenyl |
| 203 | NH2 | H | H | H | 3-(3-chlorophenoxy)phenyl |
| 204 | NH2 | H | H | H | 3-(3-trifluoromethylphenoxy)phenyl |
| 205 | NH2 | H | H | H | 3-(3-trifluoromethoxyphenoxy)phenyl |
| 206 | NH2 | H | H | H | 3-(3-ethoxyphenoxy)phenyl |
| 207 | NH2 | H | H | H | 3-(3-ethylphenoxy)phenyl |
| 208 | NH2 | H | H | H | 3-(3-fluorophenoxy)phenyl |
| 209 | NH2 | H | H | H | 2-fluoro-3-(4-pentynyloxy)phenyl |
| 210 | NH2 | H | H | H | 3-(4-fluorophenoxy)phenyl |
| 211 | NH2 | H | H | H | 3-(4-chlorophenoxy)phenyl |
| 212 | NH2 | H | H | H | 3-(4-trifluoromethoxyphenoxy)phenyl |
| 213 | NH2 | H | H | H | 3-(4-ethoxyphenoxy)phenyl |
| 214 | NH2 | H | H | H | 3-(4-ethylphenoxy)phenyl |
| 215 | NH2 | H | H | H | 3-(4-propylphenoxy)phenyl |
| 216 | NH2 | H | H | H | 3-phenylthiophenyl |
| 217 | NH2 | H | H | H | 4-phenylthiophenyl |
| 218 | NH2 | H | H | H | 3-(indan-5-yloxy)phenyl |
| 219 | NH2 | H | H | H | 3-(5,6,7,8-tetrahydronaphthalen-2-yloxy)phenyl |
| 220 | NH2 | H | H | H | 3-(benzo[1,3]dioxol-5-yloxy)phenyl |
| 221 | NH2 | H | H | H | 3-(naphthalen-2-yloxy)phenyl |
| 222 | NH2 | H | H | H | 3-(pyridin-3-yloxy)phenyl |
| 223 | NH2 | H | H | H | 2-fluoro-3-butoxyphenyl |
| 224 | NH2 | H | H | H | 2-fluoro-3-pentyloxyphenyl |
| 225 | NH2 | H | H | H | 2-fluoro-3-hexyloxyphenyl |
| 226 | NH2 | H | H | H | 2-fluoro-3-heptyloxyphenyl |
| 227 | NH2 | H | H | H | 2-fluoro-3-phenoxyphenyl |
| 228 | NH2 | H | H | H | 2-fluorophenyl |
| 229 | NH2 | H | H | H | 2-chlorophenyl |
| 230 | NH2 | H | H | H | 3-trifluoromethylphenyl |
| 231 | NH2 | H | H | H | 2-fluoro-3-propoxyphenyl |
| 232 | NH2 | H | H | H | 2-fluoro-3-(2-propenyloxy)phenyl |
| 233 | NH2 | H | H | H | 3-(2-propynyloxy)phenyl |
| 234 | NH2 | H | H | H | 2-fluoro-3-(2-propynyloxy)phenyl |
| 235 | NH2 | H | H | H | 5-(4-methylphenoxy)thiophen-2-yl |
| 236 | NH2 | H | H | H | 2,6-difluorophenyl |
| 237 | NH2 | H | H | H | 3,5-difluorophenyl |
| 238 | NH2 | H | H | H | 3,4-difluorophenyl |
| 239 | NH2 | H | H | H | 5-(3-methylphenoxy)thiophen-2-yl |
| 240 | NH2 | CH3 | H | H | 5-(3-methylphenoxy)thiophen-2-yl |
| 241 | NH2 | H | H | H | 5-(4-chlorophenoxy)thiophen-2-yl |
| 242 | NH2 | H | H | H | 2-fluoro-5-phenoxyphenyl |
| 243 | NH2 | CH3 | H | H | 5-(4-methylphenoxy)thiophen-2-yl |
| 244 | NH2 | H | H | H | 5-(3-chlorophenoxy)thiophen-2-yl |
| 245 | NH2 | H | H | H | 5-(4-fluorophenoxy)thiophen-2-yl |
| 246 | NH2 | H | H | H | 3-(4-trifluoromethylphenoxy)phenyl |
| 247 | NH2 | H | H | H | 4-(3,5-dimethylphenoxy)phenyl |
| 248 | H | H | H | H | 2-fluorophenyl |
| 249 | H | H | H | H | 2-fluoro-3-methoxyphenyl |
| 250 | H | H | H | H | 4-benzyloxyphenyl |
| 251 | NH2 | H | H | H | 4-benzyloxyphenyl |
| 252 | H | H | H | H | 3-phenoxyphenyl |
| 253 | H | H | H | H | 4-(4-fluorophenoxy)phenyl |
| 254 | H | H | H | H | 4-phenoxyphenyl |
| 255 | H | H | H | H | 4-(3-chlorophenyl)methoxyphenyl |
| 256 | H | H | H | H | 5-phenoxythiophen-2-yl |
| 257 | H | H | F | H | 4-phenoxyphenyl |
| 258 | H | CH3 | H | H | 2-fluorophenyl |
| 259 | H | CH3 | H | H | 2-fluoro-3-methoxyphenyl |
| 260 | H | CH3 | H | H | 4-benzyloxyphenyl |
| 261 | H | CH3 | H | H | 3-phenoxyphenyl |
| 262 | H | CH3 | H | H | 4-(4-fluorophenoxy)phenyl |
| 263 | H | CH3 | H | H | 4-phenoxyphenyl |
| 264 | H | CH3 | H | H | 4-(3-chlorophenyl)methoxyphenyl |
| 265 | H | CH3 | H | H | 5-phenoxythiophen-2-yl |
| 266 | H | CH3 | F | H | 4-phenoxyphenyl |
| 267 | NH2 | H | H | F | 4-phenoxyphenyl |
| 268 | NH2 | H | F | F | 4-phenoxyphenyl |
| 269 | H | NH2 | H | H | 2-fluorophenyl |
| 270 | H | NH2 | H | H | 2-fluoro-3-methoxyphenyl |
| 271 | H | NH2 | H | H | 4-benzyloxyphenyl |
| 272 | H | NH2 | H | H | 3-phenoxyphenyl |
| 273 | H | NH2 | H | H | 4-(4-fluorophenoxy)phenyl |
| 274 | H | NH2 | H | H | 4-phenoxyphenyl |
| 275 | H | NH2 | H | H | 4-(3-chlorophenyl)methoxyphenyl |
| 276 | H | NH2 | H | H | 5-phenoxythiophen-2-yl |
| 277 | H | NH2 | F | H | 4-phenoxyphenyl |
| 278 | NH2 | H | H | H | 4-[(pyridin-2-yloxy)methyl]phenyl |
| 279 | NH2 | H | H | H | 4-[(6-methylpyridin-2-yloxy)methyl]phenyl |
| 280 | NH2 | H | H | H | 4-(butoxymethyl)phenyl |
| 281 | NH2 | H | H | H | 4-(cyclopropylmethoxy)phenyl |
| 282 | NH2 | H | H | H | 4-[(pyridin-2-yl)methoxy]phenyl |
| 283 | NH2 | H | H | H | 4-[(6-methylpyridin-2-yl)methoxy]phenyl |
| 284 | NH2 | H | H | H | 4-[(4-methylpyridin-2-yl)methoxy]phenyl |
| 285 | NH2 | H | H | H | 2-(phenylmethoxy)pyridin-5-yl |
| 286 | NH2 | NH2 | H | H | 4-[(pyridin-2-yloxy)methyl]phenyl |
| 287 | NH2 | NH2 | H | H | 4-[(6-methylpyridin-2-yloxy)methyl]phenyl |
| 288 | NH2 | NH2 | H | H | 4-(butoxymethyl)phenyl |
| 289 | NH2 | NH2 | H | H | 4-(2-fluorophenyl)methoxyphenyl |
| 290 | NH2 | NH2 | H | H | 4-(3-fluorophenyl)methoxyphenyl |
| 291 | NH2 | NH2 | H | H | 4-(4-fluorophenyl)methoxyphenyl |
| 292 | NH2 | NH2 | H | H | 4-(cyclopropylmethoxy)phenyl |
| 293 | NH2 | NH2 | H | H | 4-[(pyridin-2-yl)methoxy]phenyl |
| 294 | NH2 | NH2 | H | H | 4-[(6-methylpyridin-2-yl)methoxy]phenyl |
| 295 | NH2 | NH2 | H | H | 4-[(4-methylpyridin-2-yl)methoxy]phenyl |
| 296 | NH2 | NH2 | H | H | 2-benzyloxypyridin-5-yl |
| 297 | NH2 | CH2OCH3 | H | H | 4-benzyloxyphenyl |
| 298 | NH2 | CH2OCH3 | H | H | 4-[(pyridin-2-yl)methoxy]phenyl |
| 299 | NH2 | H | H | H | 4-[(5-fluoropyridin-2-yl)methoxy]phenyl |
| 300 | NH2 | H | H | H | 4-[(5-methylpyridin-2-yl)methoxy]phenyl |
| 301 | NH2 | H | H | H | 4-[(4-methylpyridin-2-yloxy)methyl]phenyl |
| 302 | NH2 | H | H | H | 4-[(5-methylpyridin-2-yloxy)methyl]phenyl |
| 303 | NH2 | H | H | H | 4-[(6-fluoropyridin-2-yl)methoxy]phenyl |
| 304 | NH2 | H | H | H | 4-[(5-methylfuran-2-yl)methyl]phenyl |
| 305 | NH2 | H | H | H | 4-[(2-methylpyridin-4-yl)methoxy]phenyl |
| 306 | NH2 | H | H | H | 4-[(pyridin-4-yl)methoxy]phenyl |
| 307 | NH2 | H | H | H | 4-[(pyridin-3-yl)methoxy]phenyl |
| 308 | NH2 | H | H | H | 4-[(4-chloropyridin-2-yl)methoxy]phenyl |
| 309 | NH2 | H | H | H | 4-[(6-chloropyridin-2-yl)methoxy]phenyl |
| 310 | NH2 | H | H | H | 2-phenoxypyridin-5-yl |
| 311 | NH2 | H | H | H | 2-(phenoxymethyl)pyridin-5-yl |
| 312 | NH2 | H | H | H | 4-[(6-fluoropyridin-2-yloxy)methyl]phenyl |
| 313 | NH2 | H | H | H | 4-[(5-fluoropyridin-2-yloxy)methyl]phenyl |
| 314 | NH2 | H | H | H | 1-benzylpyrrol-3-yl |
| 315 | NH2 | H | H | H | 2-[(4-fluorophenyl)methoxy]pyridin-5-yl |
| 316 | NH2 | H | H | H | 4-[(4-fluoropyridin-2-yloxy)methyl]phenyl |
| 317 | NH2 | H | H | H | 3-[(pyridin-2-yl)methoxy]phenyl |
| 318 | NH2 | H | H | H | 4-[(5-chlorofuran-2-yl)methyl]phenyl |
| 319 | NH2 | H | H | H | 4-[(5-chloropyridin-2-yl)methoxy]phenyl |
| 320 | NH2 | H | H | H | 3-(cyclopropylmethoxy)phenyl |
| 321 | NH2 | H | H | H | 4-(benzylamino)phenyl |
| 322 | NH2 | H | H | H | 4-(phenylamino)phenyl |
| 323 | NH2 | H | H | H | 2-(3-fluorophenoxy)pyridin-5-yl |
| 324 | NH2 | H | H | H | 2-[(4-fluorophenoxy)methyl]pyridin-5-yl |
| 325 | NH2 | H | H | H | 4-[(phenylamino)methyl]phenyl |
| 326 | NH2 | H | H | H | 2-(2-fluorophenoxy)pyridin-5-yl |
| 327 | NH2 | H | H | H | 2-(4-fluorophenoxy)pyridin-5-yl |
| 328 | NH2 | H | H | H | 4-[(thiophen-3-yl)methyl]phenyl |
| 329 | NH2 | H | H | H | 4-cyclopentyloxyphenyl |
| 330 | NH2 | H | H | H | 4-cyclohexyloxyphenyl |
| 331 | NH2 | H | H | H | 4-[2-(furan-2-yl)ethyl]phenyl |
| 332 | NH2 | H | H | H | 4-[2-(tetrahydrofuran-2-yl)ethyl]phenyl |
| 333 | NH2 | H | H | H | 3-(pyridin-2-yl)phenyl |
| 334 | NH2 | H | H | H | 3-fluoro-4-[(pyridin-2-yl)methoxy]phenyl |
| 335 | NH2 | H | H | H | 4-[(benzothiazol-2-yl)methoxy]phenyl |
| 336 | NH2 | H | H | H | 2-(3,4-difluorophenylmethoxy)pyridin-5-yl |
| 337 | NH2 | H | H | H | 2-(2,4-difluorophenylmethoxy)pyridin-5-yl |
| 338 | NH2 | H | H | H | 5-(4-fluorophenoxy)thiophen-2-yl |
| 339 | NH2 | H | H | H | 5-(4-methylphenylmethyl)thiophen-2-yl |
| 340 | NH2 | H | H | H | 4-[2-(pyridin-2-yl)ethyl]phenyl |
| 341 | NH2 | H | H | H | 3-fluoro-4-[(5-fluoropyridin-2-yl)methoxy]phenyl |
| 342 | NH2 | H | H | H | 2-fluoro-4-[(pyridin-2-yl)methoxy]phenyl |
| 343 | NH2 | H | H | H | 2-fluoro-4-[(5-fluoropyridin-2-yl)methoxy]phenyl |
| 344 | NH2 | H | H | H | 2-phenylthiopyridin-5-yl |
| 345 | NH2 | H | H | H | 4-[(6-methoxypyridin-2-yl)methoxy]phenyl |
| 346 | NH2 | H | H | H | 2-(pyridin-3-yloxy)pyridin-5-yl |
| 347 | NH2 | NH2 | H | H | 4-[(5-methylpyridin-2-yl)methoxy]phenyl |
| 348 | NH2 | NH2 | H | H | 4-[(4-methylpyridin-2-yloxy)methyl]phenyl |
| 349 | NH2 | NH2 | H | H | 4-[(5-methylpyridin-2-yloxy)methyl]phenyl |
| 350 | NH2 | NH2 | H | H | 4-[(6-fluoropyridin-2-yl)methoxy]phenyl |
| 351 | NH2 | NH2 | H | H | 4-[(5-methylfuran-2-yl)methyl]phenyl |
| 352 | NH2 | NH2 | H | H | 5-(4-methylphenylmethyl)thiophen-2-yl |
| 353 | NH2 | NH2 | H | H | 4-[(pyridin-4-yl)methoxy]phenyl |
| 354 | NH2 | NH2 | H | H | 4-[(pyridin-3-yl)methoxy]phenyl |
| 355 | NH2 | NH2 | H | H | 4-[(4-chloropyridin-2-yl)methoxy]phenyl |
| 356 | NH2 | NH2 | H | H | 4-[(6-chloropyridin-2-yl)methoxy]phenyl |
| 357 | NH2 | NH2 | H | H | 2-phenoxypyridin-5-yl |
| 358 | NH2 | NH2 | H | H | 4-[(6-fluoropyridin-2-yloxy)methyl]phenyl |
| 359 | NH2 | NH2 | H | H | 4-[(5-fluoropyridin-2-yloxy)methyl]phenyl |
| 360 | NH2 | NH2 | H | H | 2-[(4-fluorophenyl)methoxy]pyridin-5-yl |
| 361 | NH2 | NH2 | H | H | 1-benzylpyrrol-3-yl |
| 362 | NH2 | NH2 | H | H | 2-[(4-fluorophenyl)methoxy]pyridin-5-yl |
| 363 | NH2 | NH2 | H | H | 4-[(4-fluoropyridin-2-yloxy)methyl]phenyl |
| 364 | NH2 | NH2 | H | H | 3-[(pyridin-2-yl)methoxy]phenyl |
| 365 | NH2 | NH2 | H | H | 4-[(5-chlorofuran-2-yl)methyl]phenyl |
| 366 | NH2 | NH2 | H | H | 2-phenoxypyridin-5-yl |
| 367 | NH2 | NH2 | H | H | 4-[(5-chloropyridin-2-yl)methoxy]phenyl |
| 368 | NH2 | NH2 | H | H | 3-butoxyphenyl |
| 369 | NH2 | NH2 | H | H | 3-(cyclopropylmethoxy)phenyl |
| 370 | NH2 | NH2 | H | H | 3-(phenoxymethyl)pyridin-6-yl |
| 371 | NH2 | NH2 | H | H | 4-(benzylamino)phenyl |
| 372 | NH2 | NH2 | H | H | 4-(phenylamino)phenyl |
| 373 | NH2 | NH2 | H | H | 4-butylphenyl |
| 374 | NH2 | NH2 | H | H | 2-(3-fluorophenoxy)pyridin-5-yl |
| 375 | NH2 | NH2 | H | H | 2-[(4-fluorophenoxy)methyl]pyridin-5-yl |
| 376 | NH2 | NH2 | H | H | 4-[(phenylamino)methyl]phenyl |
| 377 | NH2 | NH2 | H | H | 2-(2-fluorophenoxy)pyridin-5-yl |
| 378 | NH2 | NH2 | H | H | 2-(4-fluorophenoxy)pyridin-5-yl |
| 379 | NH2 | NH2 | H | H | 4-[(thiophen-3-yl)methyl]phenyl |
| 380 | NH2 | NH2 | H | H | 4-cyclopentyloxyphenyl |
| 381 | NH2 | NH2 | H | H | 3-(pyridin-2-yl)phenyl |
| 382 | NH2 | NH2 | H | H | 4-cyclohexyloxyphenyl |
| 383 | NH2 | NH2 | H | H | 4-[2-(furan-2-yl)ethyl]phenyl |
| 384 | NH2 | NH2 | H | H | 4-(3-fluorophenoxy)phenyl |
| 385 | NH2 | NH2 | H | H | 4-[2-(tetrahydrofuran-2-yl)ethyl]phenyl |
| 386 | NH2 | NH2 | H | H | 4-(2-fluorophenoxy)phenyl |
| 387 | NH2 | NH2 | H | H | 5-phenoxypyridin-3-yl |
| 388 | NH2 | NH2 | H | H | 3-(pyridin-2-yl)phenyl |
| 389 | NH2 | NH2 | H | H | 3-biphenyl |
| 390 | NH2 | NH2 | H | H | 4-phenoxymethylphenyl |
| 391 | NH2 | NH2 | H | H | 4-[(3-fluoropyridin-2-yl)methoxy]phenyl |
| 392 | NH2 | NH2 | H | H | 3-fluoro-4-[(pyridin-2-yl)methoxy]phenyl |
| 393 | NH2 | NH2 | H | H | 4-[(benzothiazol-2-yl)methoxy]phenyl |
| 394 | NH2 | NH2 | H | H | 2-(3,4-difluorophenylmethoxy)pyridin-5-yl |
| 395 | NH2 | NH2 | H | H | 2-(2,4-difluorophenylmethoxy)pyridin-5-yl |
| 396 | NH2 | NH2 | H | H | 2-(pyridin-2-yl)methoxypyridin-5-yl |
| 397 | NH2 | NH2 | H | H | 5-(4-fluorophenoxy)thiophen-2-yl |
| 398 | NH2 | NH2 | H | H | 5-(4-methylphenylmethyl)thiophen-2-yl |
| 399 | NH2 | NH2 | H | H | 3-fluoro-4-[(5-fluoropyridin-2-yl)methoxy]phenyl |
| 400 | NH2 | NH2 | H | H | 2-fluoro-4-[(pyridin-2-yl)methoxy]phenyl |
| 401 | NH2 | NH2 | H | H | 4-[2-(pyridin-2-yl)ethyl]phenyl |
| 402 | NH2 | NH2 | H | H | 1-(3-fluorophenylmethyl)pyrrol-3-yl |
| 403 | NH2 | NH2 | H | H | 2-phenylthiopyridin-5-yl |
| 404 | NH2 | NH2 | H | H | 4-(3-methoxyphenylmethoxy)phenyl |
| 405 | NH2 | NH2 | H | H | 4-[(6-methoxypyridin-2-yl)methoxy]phenyl |
| 406 | NH2 | NH2 | H | H | 2-(pyridin-3-yloxy)pyridin-5-yl |
| 407 | NH2 | CH2OCH3 | H | H | 4-[(5-methylfuran-2-yl)methyl]phenyl |
| 408 | NH2 | CH2OCH3 | H | H | 4-[(pyridin-2-yloxy)methyl]phenyl |
| 409 | NH2 | CH2OCH3 | H | H | 2-phenoxypyridin-5-yl |
| 410 | NH2 | CH2OCH3 | H | H | 4-[(5-chlorofuran-2-yl)methyl]phenyl |
| 411 | NH2 | CH2OH | H | H | 4-benzyloxyphenyl |
| 412 | NH2 | CH3 | H | H | 4-[(pyridin-2-yloxy)methyl]phenyl |
| 413 | NH2 | H | Cl | H | 4-[(pyridin-2-yloxy)methyl]phenyl |
| 414 | NH2 | H | F | H | 4-benzyloxyphenyl |
| 415 | NH2 | H | F | H | 4-[(5-fluoropyridin-2-yloxy)methyl]phenyl |
| 416 | NH2 | H | F | H | 4-[(4-chloropyridin-2-yloxy)methyl]phenyl |
| 417 | H | NH2 | H | H | 4-[(pyridin-2-yloxy)methyl]phenyl |
| 418 | H | NH2 | H | H | 4-[(pyridin-2-yl)methoxy]phenyl |
| 419 | H | NH2 | H | H | 4-[(5-methylfuran-2-yl)methyl]phenyl |
| 420 | H | NH2 | H | H | 2-(phenylmethoxy)pyridin-5-yl |
| 421 | H | NH2 | H | H | 4-[(pyridin-4-yl)methoxy]phenyl |
| 422 | H | NH2 | H | H | 2-phenoxypyridin-5-yl |
| 423 | H | NH2 | H | H | 4-[(5-chlorofuran-2-yl)methyl]phenyl |
| 424 | H | NH2 | H | H | 4-[(4-chloropyridin-2-yloxy)methyl]phenyl |
| 425 | H | NH2 | H | H | 4-[(5-fluoropyridin-2-yloxy)methyl]phenyl |
| 426 | H | NH2 | H | H | 2-(2-fluorophenoxy)pyridin-5-yl |
| 427 | H | NH2 | H | H | 2-(4-fluorophenoxy)pyridin-5-yl |
| 428 | H | CH3 | H | H | 4-[(pyridin-2-yloxy)methyl]phenyl |
| 429 | NH2 | H | H | H | 4-(2-propynyloxymethyl)phenyl |
| 430 | NH2 | H | H | H | 4-(cyclopropoxymethyl)phenyl |
| 431 | NH2 | H | H | H | 4-ethoxymethylphenyl |
| 432 | NH2 | H | H | H | 4-(cyclobutoxymethyl)phenyl |
| 433 | H | H | H | H | 4-benzyloxyphenyl |
| 434 | H | H | H | H | 4-[(pyridin-2-yloxy)methyl]phenyl |
| 435 | H | H | H | H | 4-[(pyridin-2-yl)methoxy]phenyl |
| 436 | NH2 | H | H | H | 2-fluoro-4-[(pyridin-2-yl)methoxy]phenyl |
| 437 | NH2 | H | H | H | 4-benzylthiophenyl |
| 438 | NH2 | H | H | H | 4-phenylthiomethylphenyl |
| 439 | NH2 | H | H | H | 4-bromophenyl |
| 440 | NH2 | H | H | H | 5-(4-fluorophenyl)methylfuran-2-yl |
| 441 | NH2 | H | H | H | 4-(pyridin-2-yloxy)phenyl |
| 442 | NH2 | H | H | H | 2-phenylmethylpyridin-5-yl |
| 443 | NH2 | NH2 | H | H | 2-fluoro-4-[(pyridin-2-yl)methoxy]phenyl |
| 444 | NH2 | NH2 | H | H | 4-[(3-cyanophenyl)methoxy]phenyl |
| 445 | NH2 | NH2 | H | H | 4-[(3-ethynylphenyl)methoxy]phenyl |
| 446 | NH2 | NH2 | H | H | 4-(2-chloropyrazin-6-yloxy)phenyl |
| 447 | NH2 | NH2 | H | H | 4-benzylthiophenyl |
| 448 | NH2 | NH2 | H | H | 4-phenylthiomethylphenyl |
| 449 | NH2 | NH2 | H | H | 4-(3-methyl-2-butenyloxy)phenyl |
| 450 | NH2 | NH2 | H | H | 4-(2-propynyloxy)phenyl |
| 451 | NH2 | NH2 | H | H | 4-bromophenyl |
| 452 | NH2 | NH2 | H | H | 5-(4-fluorophenyl)methylfuran-2-yl |
| 453 | NH2 | NH2 | H | H | 4-(pyridin-2-yloxy)phenyl |
| 454 | NH2 | NH2 | H | H | 2-phenylmethylpyridin-5-yl |

Further specific examples of the present compound will be shown in Table 2.

**Table 2**

| Compound number | Structure | Compound number | Structure |
|---|---|---|---|
| 286 | 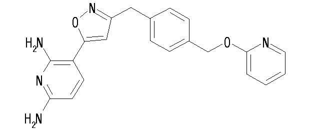 | 396 | 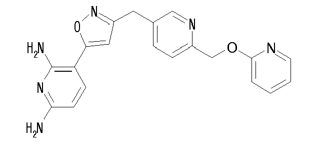 |
| 287 | 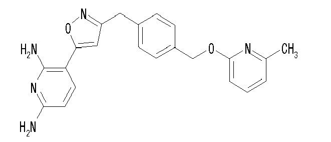 | 397 | 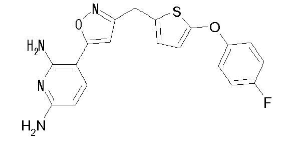 |
| 288 | 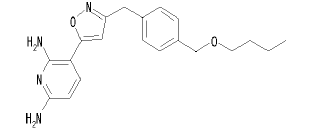 | 398 | 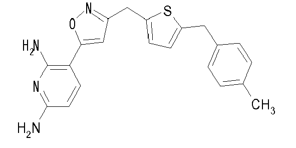 |
| 17 | 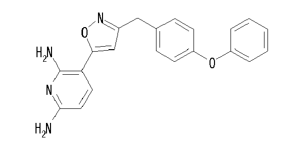 | 399 | 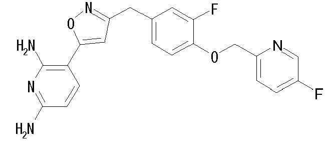 |
| 289 | 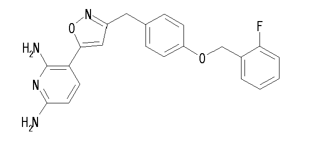 | 400 | 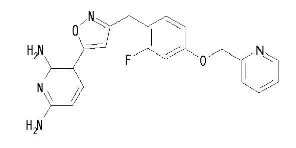 |
| 290 | 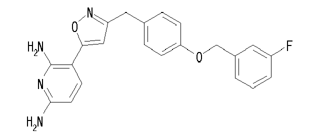 | 401 | 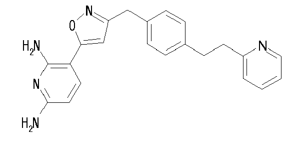 |
| 291 | 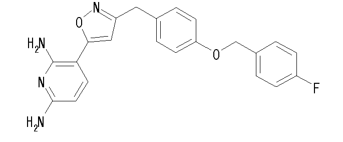 | 402 | 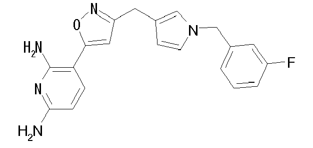 |
| 292 | 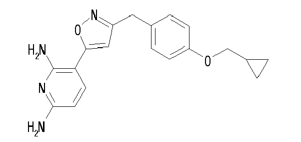 | 403 | 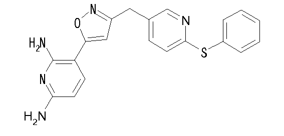 |
| 293 | 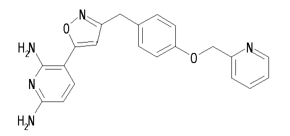 | 404 | 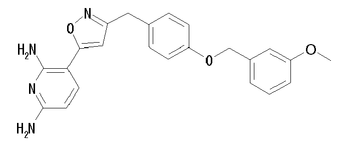 |
| 294 | 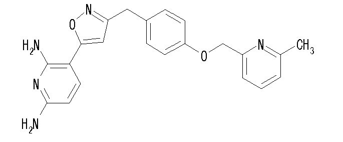 | 405 | 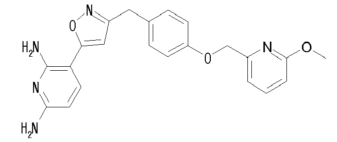 |
| 295 | 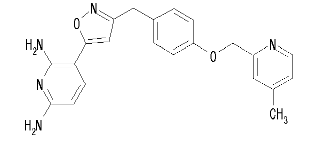 | 406 | 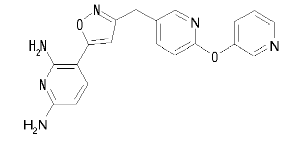 |
| 296 | 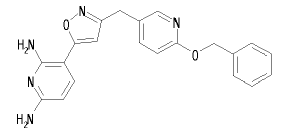 | 407 | 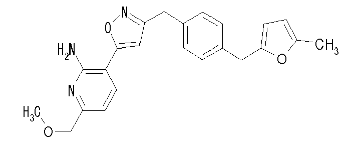 |
| 297 | 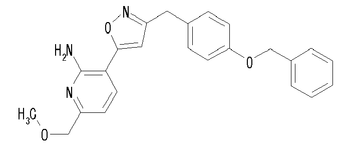 | 408 | 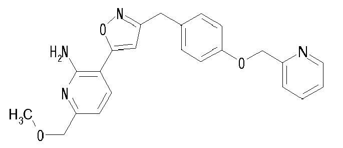 |
| 298 | 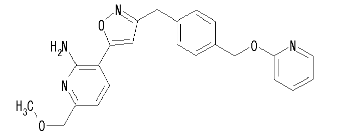 | 409 | 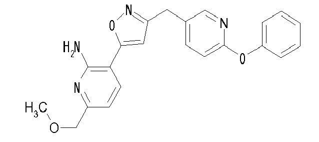 |
| 271 | 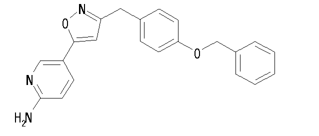 | 410 | 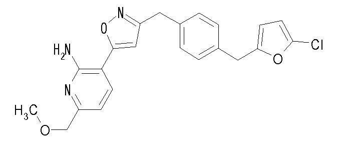 |
| 751 | 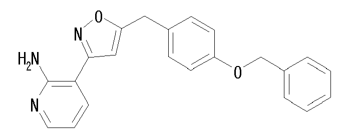 | 411 | 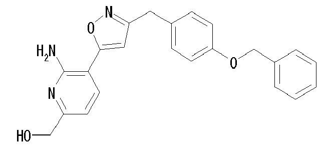 |
| 778 | 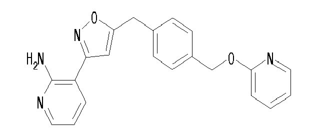 | 77 | 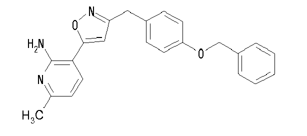 |
| 1751 | 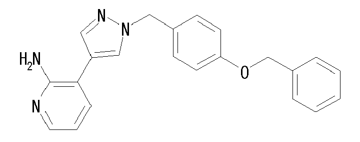 | 412 | 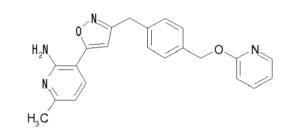 |
| 1778 | 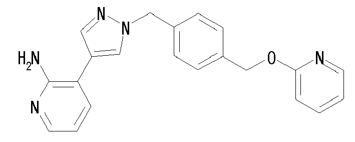 | 413 | 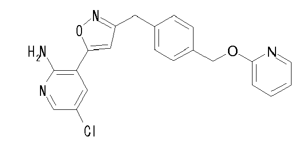 |
| 1780 | 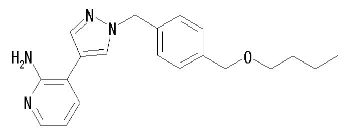 | 414 | 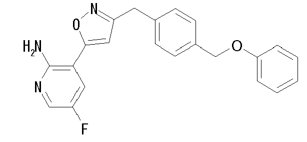 |
| 1506 | 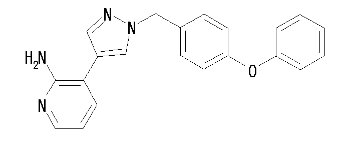 | 415 | 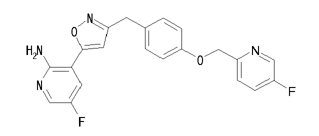 |
| 1503 | 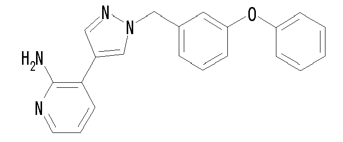 | 416 | 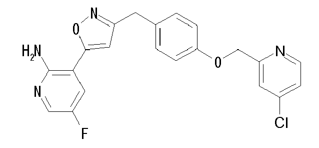 |
| 1514 | 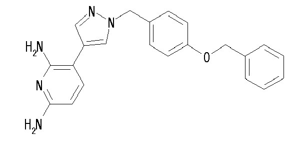 | 417 | 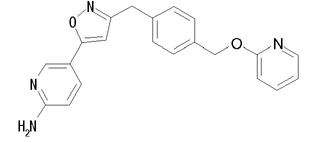 |
| 1786 | 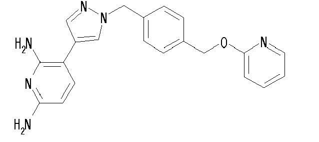 | 418 | 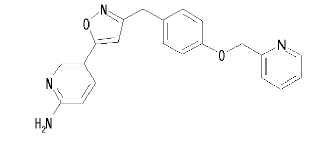 |
| 1788 | 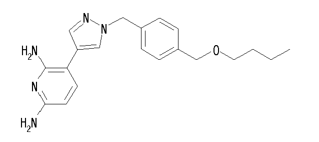 | 419 | 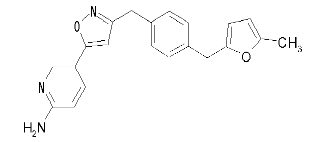 |
| 1014 | 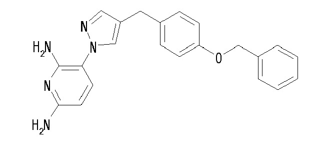 | 420 | 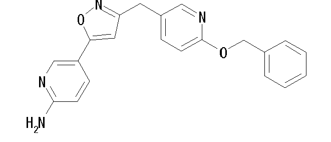 |
| 296 | 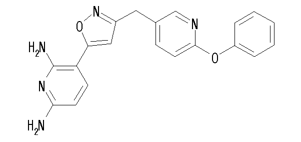 | 421 | 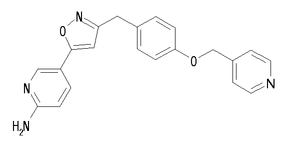 |
| 299 | 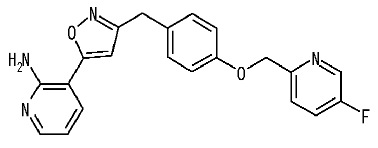 | 422 | 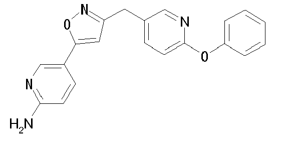 |
| 300 | 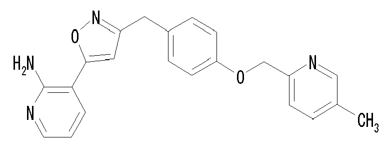 | 423 | 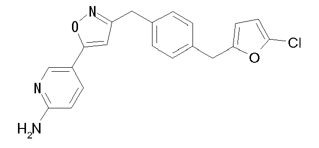 |
| 301 | 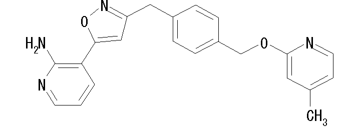 | 424 | 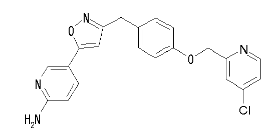 |
| 302 | 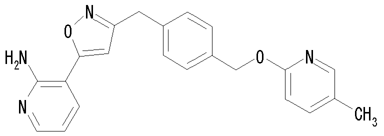 | 425 | 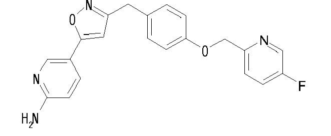 |
| 303 | 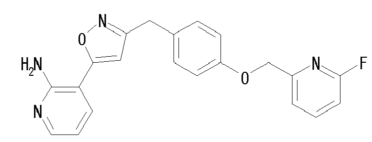 | 426 | 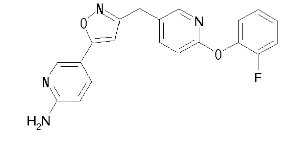 |
| 304 | 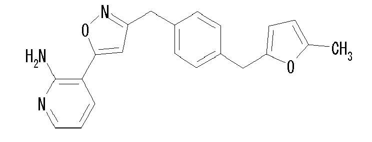 | 427 | 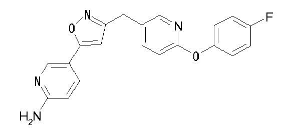 |
| 305 | 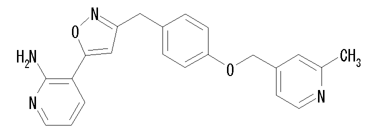 | 778 | 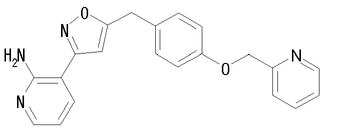 |
| 153 | 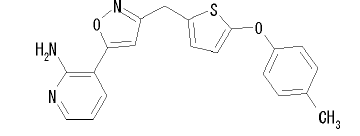 | 428 | 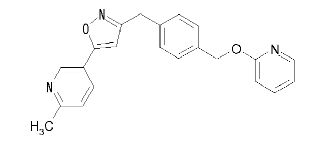 |
| 306 | 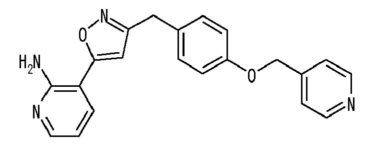 | 1785 | 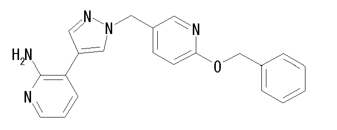 |
| 307 | 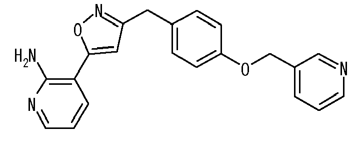 | 1782 | 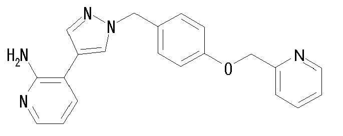 |
| 308 | 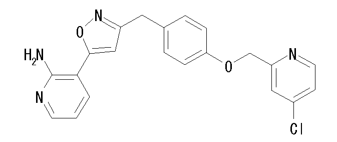 | 1810 | 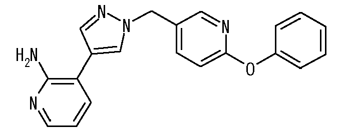 |
| 309 | 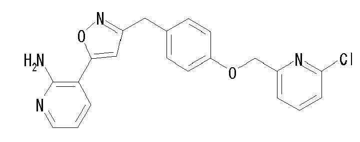 | 1929 | 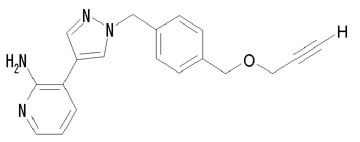 |
| 310 | 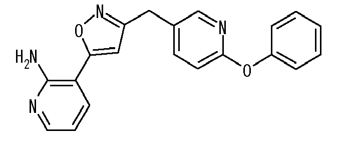 | 1930 | 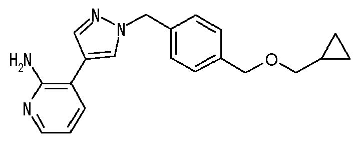 |
| 311 | 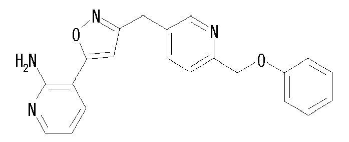 | 1931 | 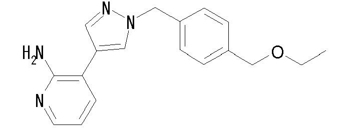 |
| 312 | 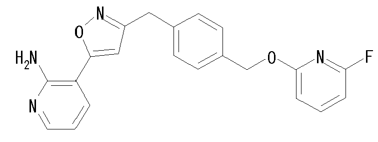 | 1932 | 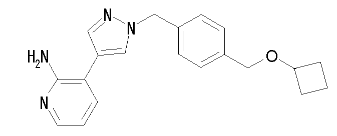 |
| 313 | 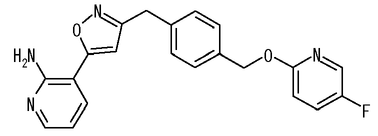 | 1785 | 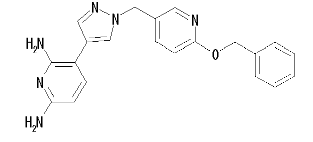 |
| 314 | 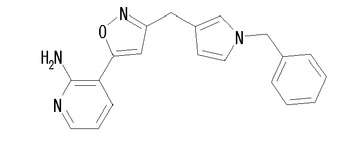 | 1293 | 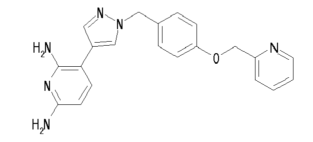 |
| 315 | 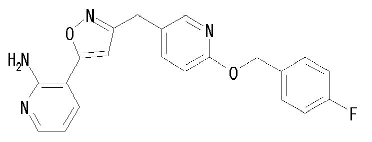 | 1796 | 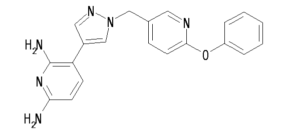 |
| 316 | 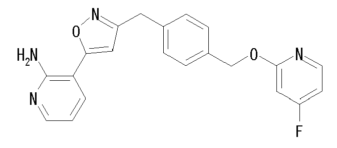 | 433 | 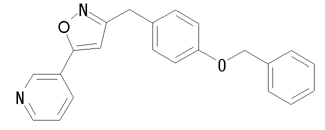 |
| 317 | 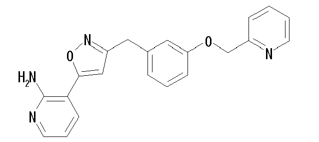 | 434 | 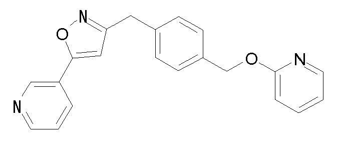 |
| 7 | 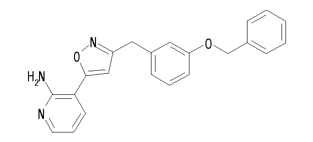 | 435 | 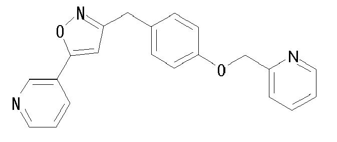 |
| 318 | 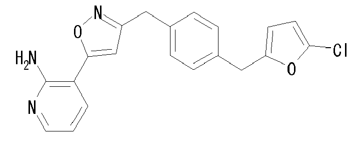 | 436 | 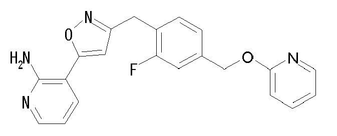 |
| 319 | 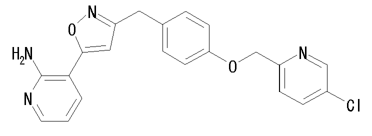 | 437 | 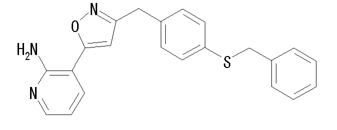 |
| 6 | 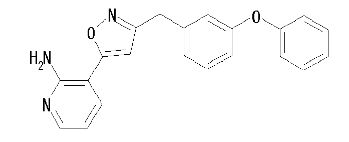 | 438 | 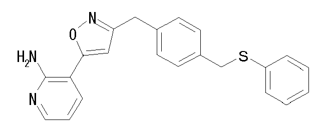 |
| 166 | 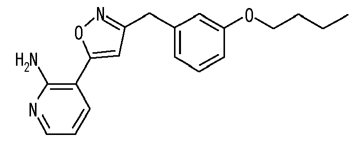 | 439 | 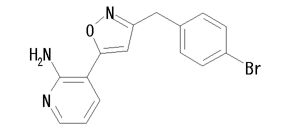 |
| 320 | 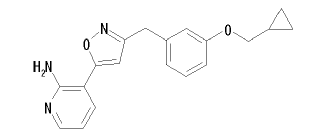 | 440 | 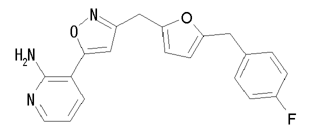 |
| 65 | 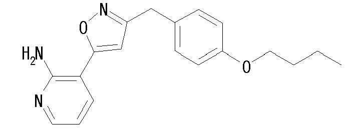 | 441 | 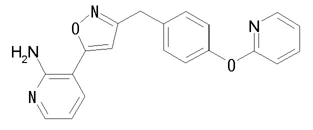 |
| 321 | 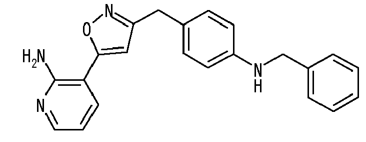 | 442 | 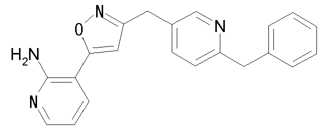 |
| 322 | 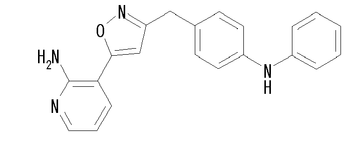 | 443 | 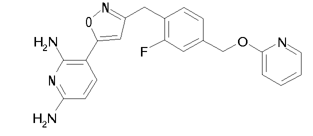 |
| 109 | 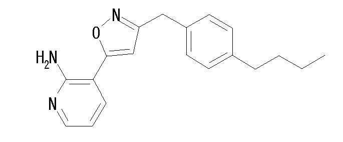 | 444 | 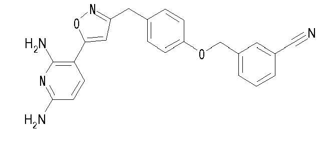 |
| 323 | 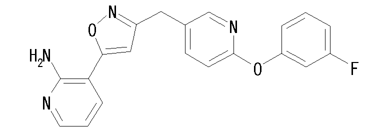 | 445 | 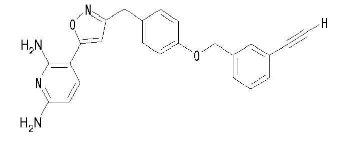 |
| 324 | 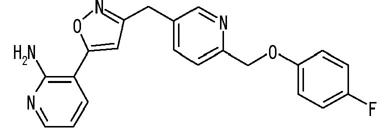 | 446 | 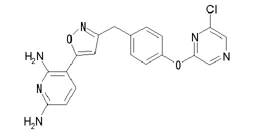 |
| 325 | 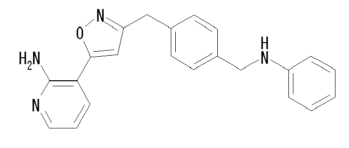 | 447 | 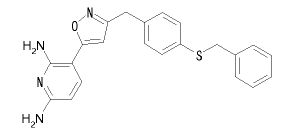 |
| 326 | 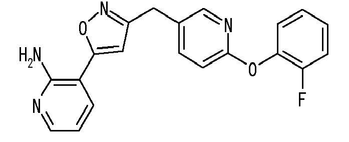 | 448 | 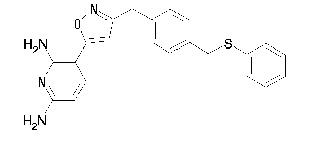 |
| 327 | 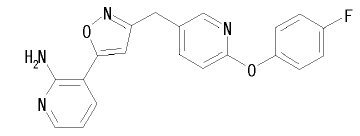 | 449 | 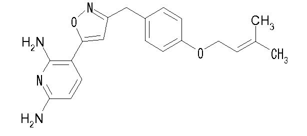 |
| 192 | 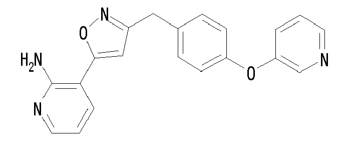 | 450 | 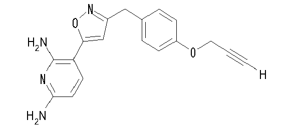 |
| 328 | 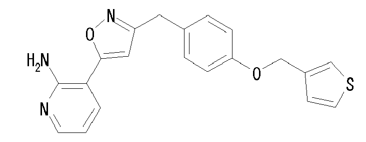 | 451 | 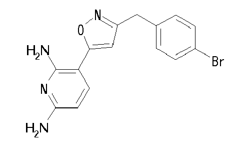 |
| 329 | 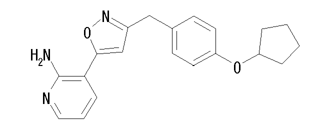 | 452 | 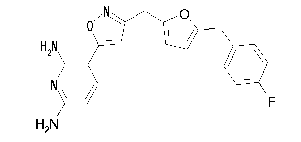 |
| 330 | 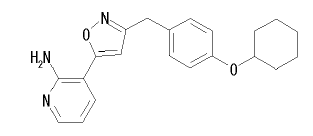 | 453 | 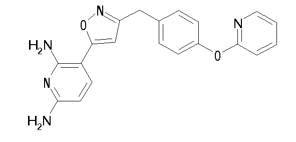 |
| 331 | 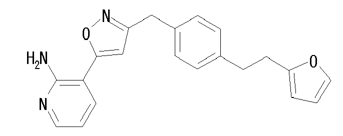 | 454 | 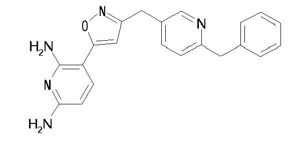 |
| 175 | 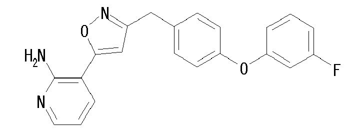 | 455 | 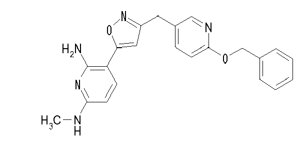 |
| 332 | 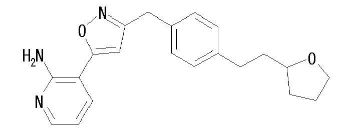 | 456 | 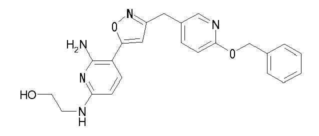 |
| 180 | 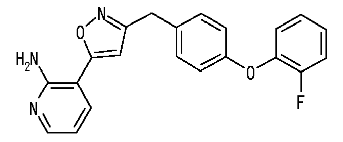 | 457 | 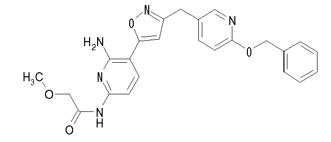 |
| 333 | 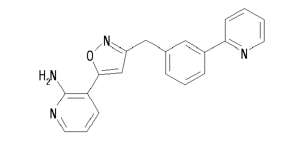 | 458 | 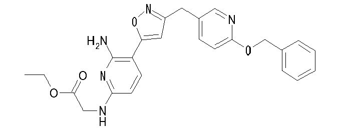 |
| 5 | 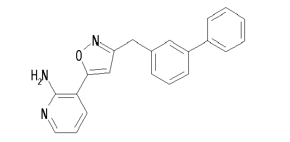 | 3 | 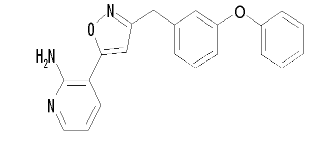 |
| 23 | 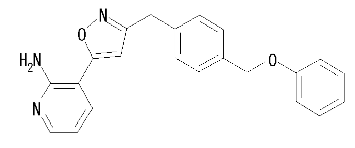 | 2751 | 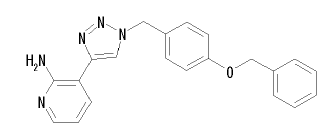 |
| 334 | 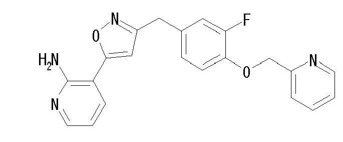 | 2760 | 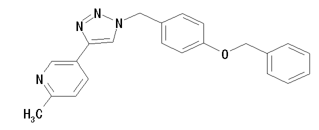 |
| 335 | 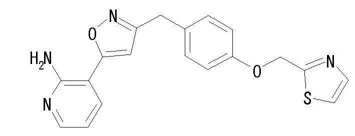 | 2771 | 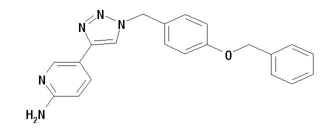 |
| 336 | 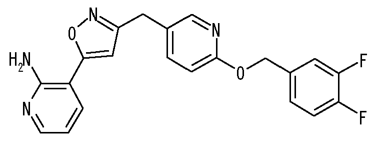 | 2514 | 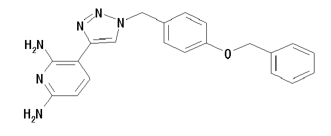 |
| 337 | 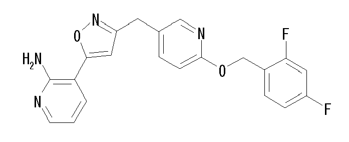 | 2251 | 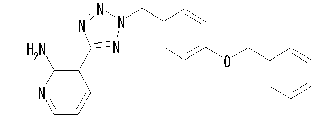 |
| 338 | 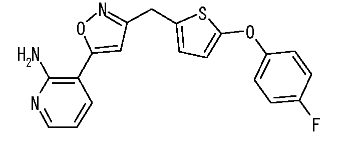 | 2297 | 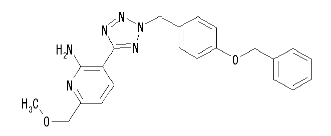 |
| 339 | 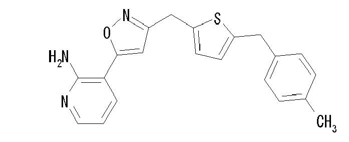 | 2014 | 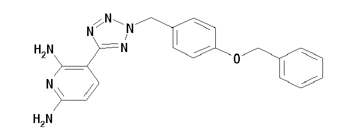 |
| 340 | 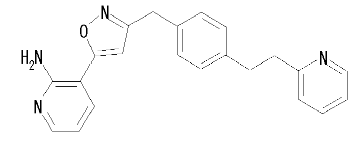 | 5251 | 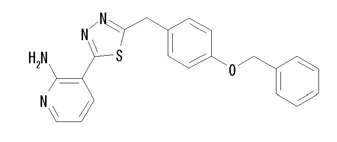 |
| 341 | 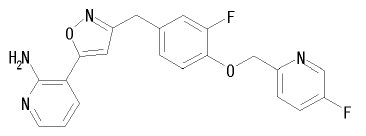 | 4751 | 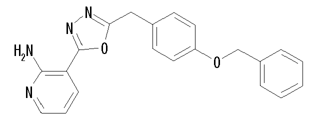 |
| 342 | 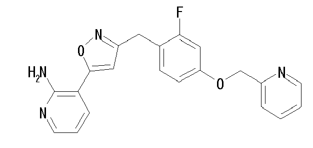 | 5014 | 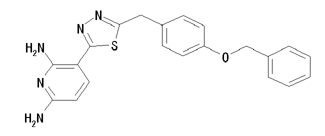 |
| 343 | 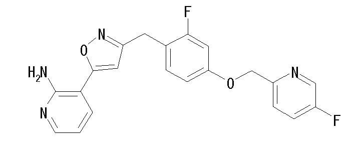 | 5297 | 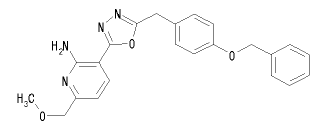 |
| 344 | 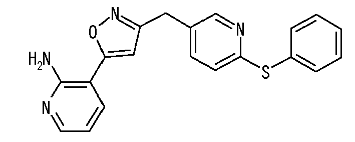 | 3251 | 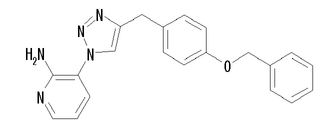 |
| 90 | 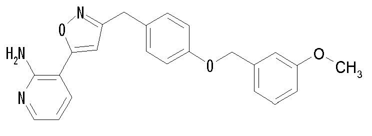 | 5751 | 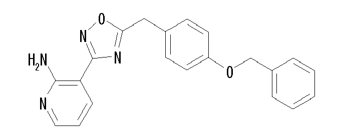 |
| 345 | 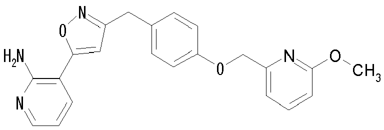 | 3751 | 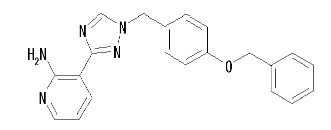 |
| 346 | 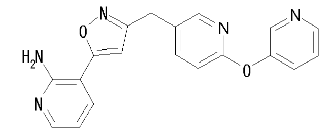 | 4251 | 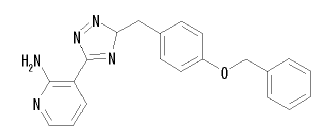 |
| 347 | 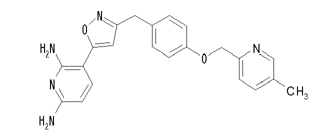 | 2818 | 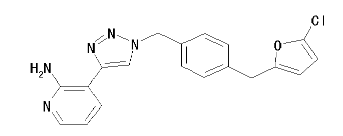 |
| 348 | 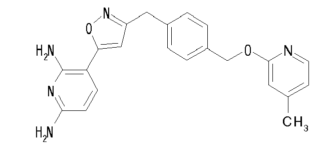 | 2960 | 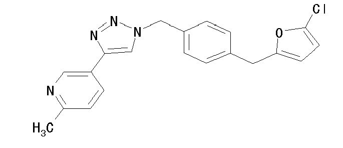 |
| 349 | 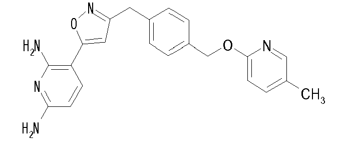 | 2923 | 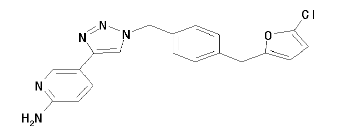 |
| 350 | 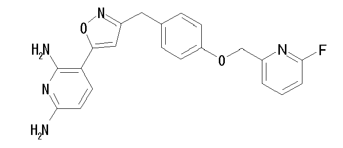 | 2910 | 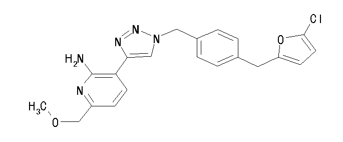 |
| 351 | 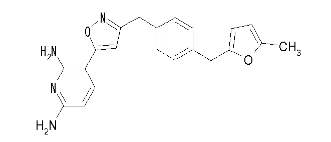 | 2865 | 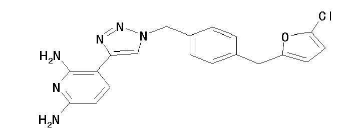 |
| 352 | 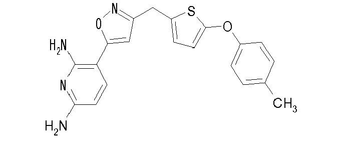 | 2318 | 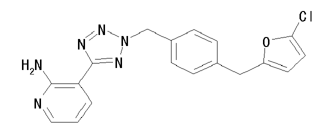 |
| 353 | 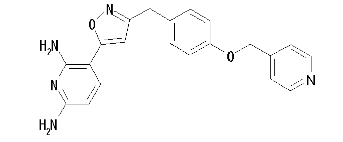 | 2865 | 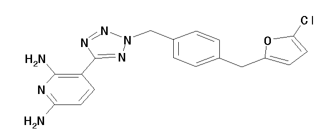 |
| 354 | 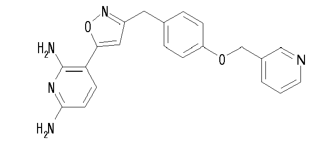 | 5782 | 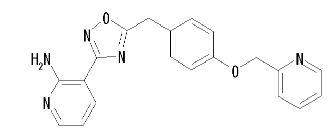 |
| 355 | 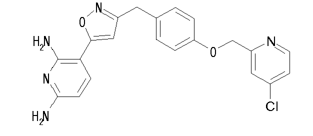 | 4782 | 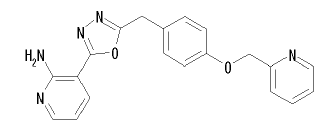 |
| 356 | 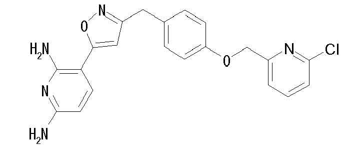 | 2782 | 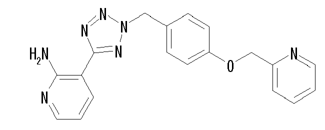 |
| 357 | 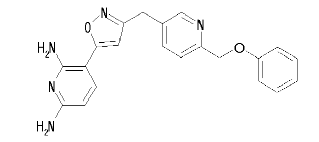 | 2778 | 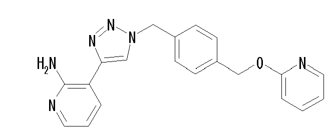 |
| 358 | 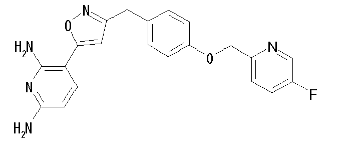 | 2278 | 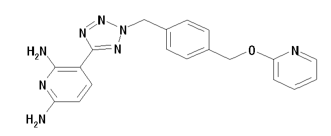 |
| 359 | 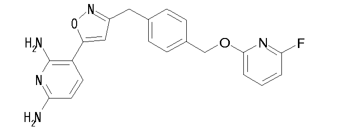 | 2503 | 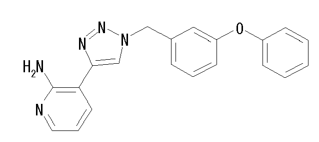 |
| 360 | 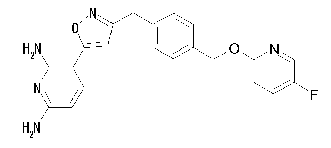 | 2772 | 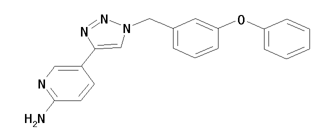 |
| 361 | 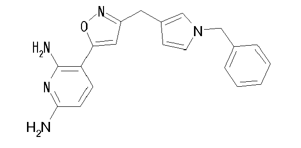 | 2515 | 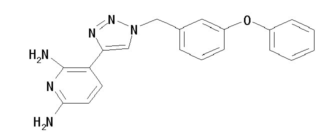 |
| 362 | 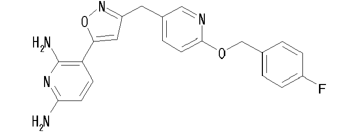 | 2006 | 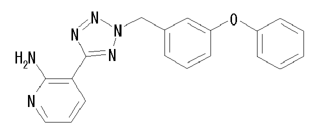 |
| 363 | 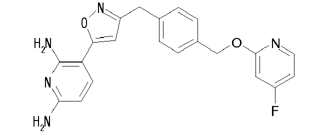 | 2506 | 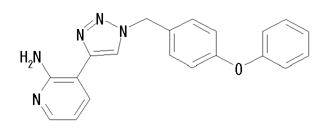 |
| 364 | 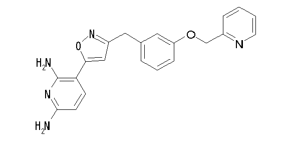 | 2774 | 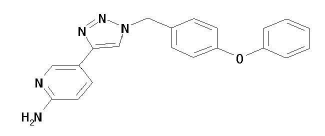 |
| 14 | 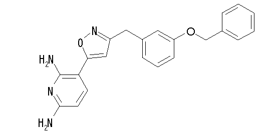 | 2517 | 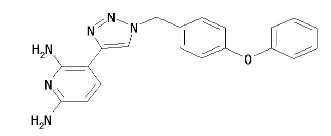 |
| 365 | 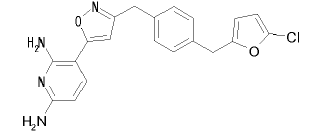 | 2003 | 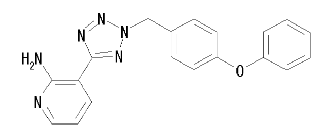 |
| 366 | 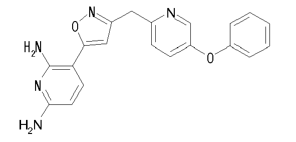 | 2793 | 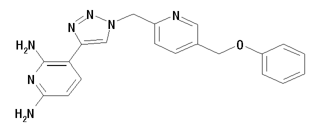 |
| 367 | 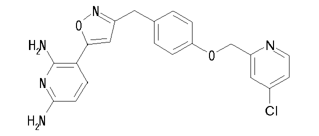 | 2782 | 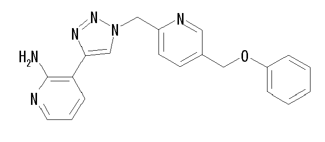 |
| 15 | 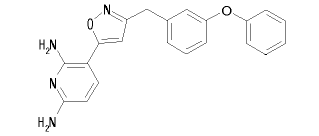 | 2782 | 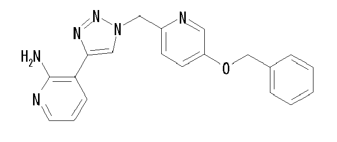 |
| 368 | 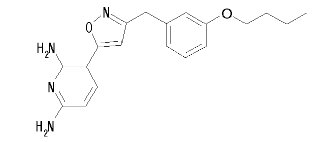 | 2282 | 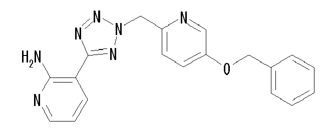 |
| 369 | 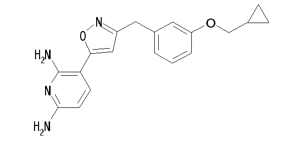 | 2293 | 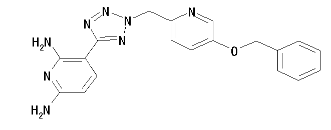 |
| 288 | 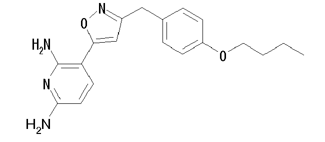 | 2961 | 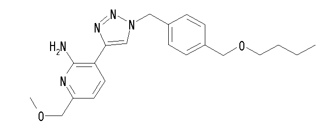 |
| 370 | 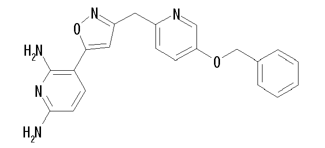 | 2788 | 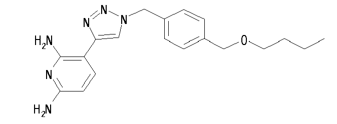 |
| 371 | 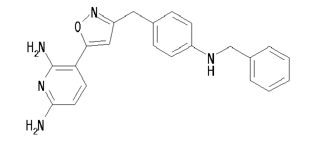 | 2780 | 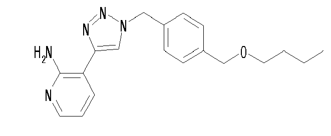 |
| 372 | 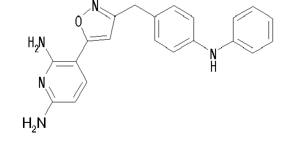 | 2962 | 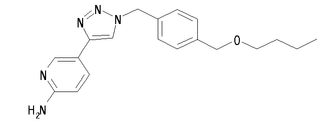 |
| 373 | 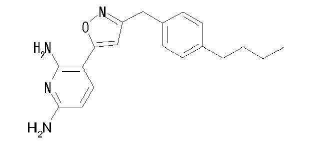 | 4780 | 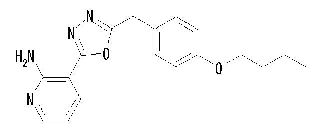 |
| 374 | 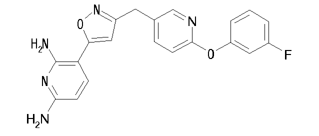 | 2037 | 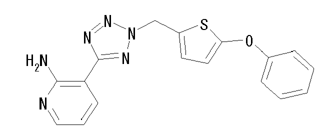 |
| 375 | 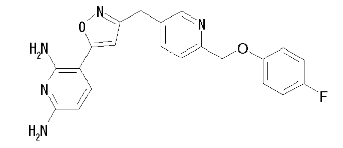 | 2276 | 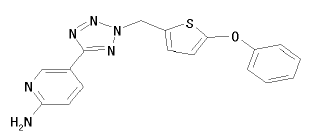 |
| 376 | 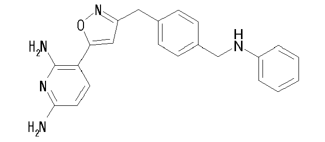 | 2463 | 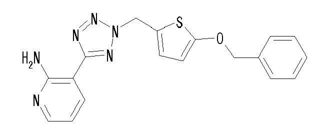 |
| 377 | 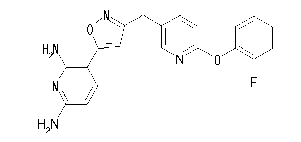 | 2001 | 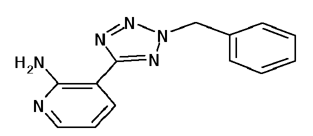 |
| 378 | 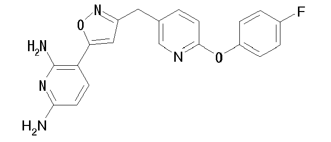 | 2064 | 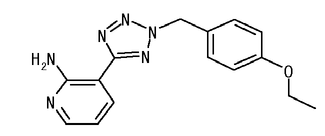 |
| 379 | 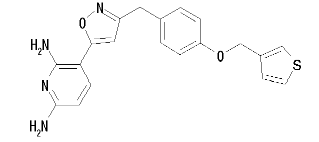 | 2463 | 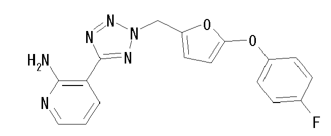 |
| 380 | 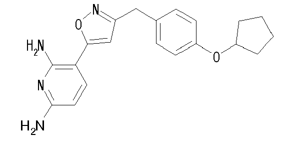 | 2465 | 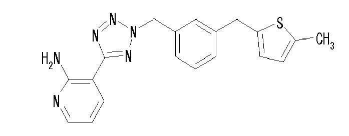 |
| 381 | 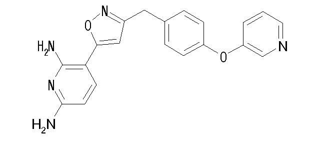 | 2304 | 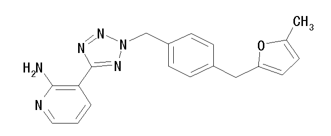 |
| 382 | 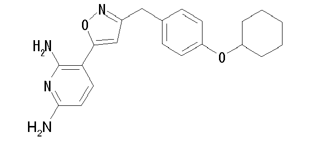 | 2466 | 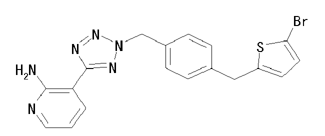 |
| 383 | 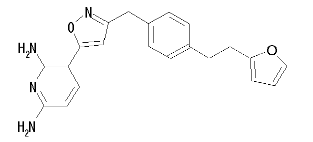 | 2314 | 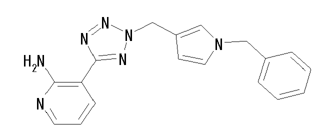 |
| 1518 | 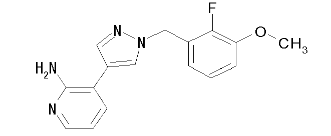 | 5506 | 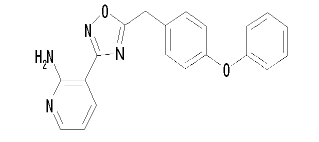 |

In the present specification, when the compound represented by the structural formula has stereoisomers, this compound encompasses all stereoisomers thereof and mixtures thereof. When the compound represented by the structural formula has tautomers, this compound encompasses all tautomers thereof and mixtures thereof. That is, in the present invention, all stereoisomers of the present compound and mixtures thereof can be used.

The present compound can be produced, for example, by production methods described in WO2007/052615 pamphlet and WO2008/035726 pamphlet.

In the case of the agricultural composition of the present invention, in general, the present compound is mixed with a liquid carrier, a solid carrier, a gas carrier, a surfactant, etc. Thereafter, formulation auxiliary reagents such as a binder or a thickener are added thereto, as necessary, so that the mixture can be formulated into a wettable powder, a granulated wettable powder, a flowable reagent, a granule, a dry flowable reagent, an emulsion, an aqueous liquid reagent, an oil reagent, a smoking reagent, an aerosol, a microcapsule, etc., and the thus produced formulations can be then used. The present compound is comprised in such a formulation at a weight ratio generally between 0.1% and 99%, and preferably between 0.2% and 90%.

Examples of a liquid carrier used in formulation include water, alcohols (e.g. methanol, ethanol, 1-propanol, 2-propanol, ethylene glycol, etc.), ketones (e.g. acetone, methyl ethyl ketone, etc.), ethers (e.g. dioxane, tetrahydrofuran, ethylene glycol monomethyl ether, diethylene glycol monomethyl ether, propylene glycol monomethyl ether, etc.), aliphatic hydrocarbons (e.g. hexane, octane, cyclohexane, coal oil, fuel oil, machine oil, etc.), aromatic hydrocarbons (e.g. benzene, toluene, xylene, solvent naphtha, methylnaphthalene, etc.), halogenated hydrocarbons (e.g. dichloromethane, chloroform, carbon tetrachloride, etc.), acid amides (e.g. dimethylformamide, dimethylacetamide, N-methylpyrrolidone, etc.), esters (e.g. ethyl acetate, butyl acetate, fatty acid glycerin ester, etc.), and nitriles (e.g. acetonitrile, propionitrile, etc.). Two or more types of such liquid carriers can be mixed at an appropriate ratio and can be then used.

Examples of a solid carrier used in formulation include vegetable powders (e.g. soybean powder, tobacco powder, wheat flour, wood flour, etc.), mineral powders (e.g. clays such as kaoline, bentonite, acid clay, or clay; talcs such as talcum powder or pyrophyllite powder; silicas such as diatomaceous earth or mica powder; etc.), alumina, sulfur powder, activated carbon, sugars (e.g. lactose, glucose, etc.), inorganic salts (e.g. calcium carbonate, sodium bicarbonate, etc.), and a glass hollow body (produced by subjecting a natural holohyaline to a burning process and then encapsulating air bubbles therein). Two or more types of such solid carriers can be mixed at an appropriate ratio and can be then used. Such liquid carrier or solid carrier is used at a ratio generally between 1% to 99% by weight, and preferably between approximately 10% and 99% by weight, based on the total weight of a pharmaceutical.

As an emulsifier, a dispersant, a spreader, a penetrant, a moisturizer, or the like used in the formulation, a surfactant is generally used. Examples of such a surfactant include: anionic surfactants such as alkyl sulfate ester salts, alkylaryl sulfonates, dialkyl sulfosuccinates, polyoxyethylene alkyl aryl ether phosphates, lignosulfonates, or naphthalenesulfonate formamide polycondensates; and nonionic surfactants such as a polyoxyethylene alkyl ether, a polyoxyethylene alkyl aryl ether, a polyoxyethylene alkyl polyoxy propylene block copolymer, or a sorbitan fatty acid ester. These surfactants can also be used in combination of two or more types. Such a surfactant is used at a ratio generally between 0.1% and 50% by weight, and preferably between approximately 0.1% and 25% by weight, based on the total weight of a pharmaceutical.

Examples of a binder or a thickener include dextrin, sodium salts of carboxymethyl cellulose, a polycarboxylic acid polymer, polyvinylpyrrolidone, polyvinyl alcohol, sodium lignosulfonate, calcium lignosulfonate, sodium polyacrylate, gum Arabic, sodium alginate, mannitol, sorbitol, bentonite mineral matter, polyacrylic acid and a derivative thereof, sodium salts of carboxymethyl cellulose, white carbon, and natural sugar derivatives (e.g. xanthan gum, Cyamoposis Gum, etc.).

Moreover, the agricultural composition of the present invention can be mixed with other types of disinfectants, insecticides, acaricides, nematicides, herbicides, plant growth regulators, fertilizers, or soil improvers, and they can be used. Otherwise, the present agricultural composition can be used together with the aforementioned reagents, without mixing with them.

Examples of such other disinfectants include: azole disinfectant compounds such as propiconazole, prothioconazole, triadimenol, prochloraz, penconazole, tebuconazole, flusilazole, diniconazole, bromuconazole, epoxiconazole, difenoconazole, cyproconazole, metconazole, triflumizole, tetraconazole, microbutanil, fenbuconazole, hexaconazole, fluquinconazole, triticonazole, bitertanol, imazalil, flutriafol, simeconazole, or ipconazole; cyclic amine disinfectant compounds such as fenpropimorph, tridemorph, or fenpropidin; benzimidazole disinfectant compounds such as carbendazim, benomyl, thiabendazole, or thiophanate-methyl; procymidone; cyprodinil; pyrimethanil; diethofencarb; thiuram; fluazinam, mancozeb; iprodione; vinclozolin, chlorothalonil; captan; mepanipyrim, fenpiclonil; fludioxonil; dichlofluanid; folpet; kresoxim-methyl; azoxystrobin; trifloxystrobin; fluoxastrobin; picoxystrobin; pyraclostrobin; dimoxystrobin; pyribencarb; metominostrobin; enestroburin; spiroxamine; quinoxyfen; fenhexamid; famoxadone; fenamidone; zoxamide; etaboxam; amisulbrom; iprovalicarb; benthiavalicarb; cyazofamid; mandipropamid; boscalid; penthiopyrad; metrafenone; fluopyran; bixafen; cyflufenamid; proquinazid; orysastrobin; furametpyr; thifluzamide; mepronil; flutolanil; flusulfamide; fluopicolide; metalaxyl M; kiralaxyl; fosetyl; cymoxanil; pencycuron; tolclofos methyl; carpropamid; diclocymet; fenoxanil; tricyclazole; pyroquilon; probenazole; isotianil; tiadinil; tebufroquin; diclomezine; kasugamycin; ferimzone; fthalide; validamycin; hydroxyisoxazole; iminoctadin-acetate; isoprothiolane; oxolinic acid; oxytetracycline; streptomycin; basic copper chloride; cupric hydroxide; basic copper sulfate; organic copper; and sulfur.

Examples of other insecticides include the following compounds:
(1) Organophosphorus compounds
   acephate, Aluminium phosphide, butathiofos, cadusafos, chlorethoxyfos, chlorfenvinphos, chlorpyrifos, chlorpyrifosmethyl, cyanophos: CYAP, diazinon, DCIP(dichlorodiisopropyl ether), dichlofenthion: ECP, dichlorvos: DDVP, dimethoate, dimethylvinphos, disulfoton, EPN, ethion, ethoprophos, etrimfos, fenthion: MPP, fenitrothion: MEP, fosthiazate, formothion, Hydrogen phosphide, isofenphos, isoxathion, malathion, mesulfenfos, methidathion: DMTP, monocrotophos, naled: BRP, oxydeprofos: ESP, parathion, phosalone, phosmet: PMP, pirimiphos-methyl, pyridafenthion, quinalphos, phenthoate: PAP, profenofos, propaphos, prothiofos, pyraclorfos, salithion, sulprofos, tebupirimfos, temephos, tetrachlorvinphos, terbufos, thiometon, trichlorphon: DEP, vamidothion, phorate, cadusafos, etc.;
(2) Carbamate compounds
   alanycarb, bendiocarb, benfuracarb, BPMC, carbaryl, carbofuran, carbosulfan, cloethocarb, ethiofencarb, fenobucarb, fenothiocarb, fenoxycarb, furathiocarb, isoprocarb: MIPC, metolcarb, methomyl, methiocarb, NAC, oxamyl, pirimicarb, propoxur: PHC, XMC, thiodicarb, xylylcarb, aldicarb, etc.;
(3) Synthetic pyrethroid compounds
   acrinathrin, allethrin, benfluthrin, beta-cyfluthrin, bifenthrin, cycloprothrin, cyfluthrin, cyhalothrin, cypermethrin, deltamethrin, esfenvalerate, ethofenprox, fenpropathrin, fenvalerate, flucythrinate, flufenoprox, flumethrin, fluvalinate, halfenprox, imiprothrin, permethrin, prallethrin, pyrethrins, resmethrin, sigma-cypermethrin, silafluofen, tefluthrin, tralomethrin, transfluthrin, tetramethrin, phenothrin, cyphenothrin, alpha-cypermethrin, zeta-cypermethrin, lambda-cyhalothrin, furamethrin, tau-fluvalinate, 2,3,5,6-tetrafluoro-4-(methoxymethyl)benzyl(E2)-(1RS, 3RS; 1RS, 3SR)-2,2-dimethyl-3-prop-1-enyl cyclopropane carboxylate, 2,3,5,6-tetrafluoro-4-methylbenzyl(EZ)-(1RS, 3RS; 1RS, 3SR)-2,2-dimethyl-3-prop-1-enyl cyclopropane carboxylate, 2,3,5,6-tetrafluoro-4-(methoxymethyl)benzyl(1RS, 3RS; 1RS, 3SR)-2,2-dimethyl-3-(2-methyl-1-propenyl) cyclopropane carboxylate, etc.;
(4) Nereistoxin compounds
   cartap, bensultap, thiocyclam, monosultap, bisultap, etc.;
(5) Neonicotinoid compounds
   imidacloprid, nitenpyram, acetamiprid, thiamethoxam, thiacloprid, dinotefuran, clothianidin, etc.;
(6) Benzoylurea compounds
   chlorfluazuron, bistrifluron, diafenthiuron, diflubenzuron, fluazuron, flucycloxuron, flufenoxuron, hexaflumuron, lufenuron, novaluron, noviflumuron, teflubenzuron, triflumuron, triazlon, etc.;
(7) Phenylpyrazole compounds
   acetoprole, ethiprole, fipronil, vaniliprole, pyriprole, pyrafluprole, etc.;
(8) Bt toxin insecticides
   Fresh spores derived from *Bacillus thuringiensis,* crystalline toxins generated therefrom, and the mixtures thereof;
(9) Hydrazine compounds
   chromafenozide, halofenozide, methoxyfenozide, tebufenozide, etc.;
(10) Organochlorine compounds
   aldrin, dieldrin, dienochlor, endosulfan, methoxychlor, etc.;
(11) Natural insecticides
   machine oil and nicotine-sulfate; and
(12) Other insecticides
   avermectin-B, bromopropylate, buprofezin, chlorphenapyr, cyromazine, D-D(1, 3-Dichloropropene), emamectin-benzoate, fenazaquin, flupyrazofos, hydroprene, methoprene, indoxacarb, metoxadiazone, milbemycin-A, pymetrozine, pyridalyl, pyriproxyfen, spinosad, sulfluramid, tolfenpyrad, triazamate, flubendiamide, lepimectin, Arsenic acid, benclothiaz, Calcium cyanamide, Calcium polysulfide, chlordane, DDT, DSP, flufenerim, flonicamid, flurimfen, formetanate, metamammonium, metam-sodium, Methyl bromide, nidinotefuran, Potassium oleate, protrifenbute, spiromesifen, Sulfur, metaflumizone, spirotetramat, pyrifluquinazone, spinetoram, and chlorantraniliprole.

Examples of other types of acaricides (acarcidal active ingredients) include acequinocyl, amitraz, benzoximate, bifenaate, bromopropylate, chinomethionat, chlorobenzilate, CPCBS (chlorfenson), clofentezine, cyflumetofen, kelthane (dicofol), etoxazole, fenbutatin oxide, fenothiocarb, fenpyroximate, fluacrypyrim, fluproxyfen, hexythiazox, propargite: BPPS, polynactins, pyridaben, Pyrimidifen, tebufenpyrad, tetradifon, spirodiclofen, spiromesifen, spirotetramat, amidoflumet, and cyenopyrafen.

Examples of such other types of nematicides (nematicidal active ingredients) include DCIP, fosthiazate, levamisol hydrochloride, methylisothiocyanate; moraltel tartarate, and imicyafos.

The weight ratio between the agricultural composition of the present invention and a disinfectant mixed with or used together with the aforementioned agricultural composition is generally between 1 : 0.01 and 1 : 100, and preferably between 1 : 0.1 and 1 : 10, in terms of active ingredient.

The weight ratio between the agricultural composition of the present invention and an insecticide mixed with or used together with the aforementioned agricultural composition is generally between 1 : 0.01 and 1 : 100, and preferably between 1 : 0.1 and 1 : 10, in terms of active ingredient.

The weight ratio between the agricultural composition of the present invention and an acaricide mixed with or used together with the aforementioned agricultural composition is generally between 1 : 0.01 and 1 : 100, and preferably between 1 : 0.1 and 1 : 10, in terms of active ingredient.

The weight ratio between the agricultural composition of the present invention and a nematicide mixed with or used together with the aforementioned agricultural composition is generally between 1 : 0.01 and 1 : 100, and preferably between 1 : 0.1 and 1 : 10, in terms of active ingredient.

The weight ratio between the agricultural composition of the present invention and a herbicide mixed with or used together with the aforementioned agricultural composition is generally between 1 : 0.01 and 1 : 100, and preferably between 1 : 0.1 and 1 : 10, in terms of active ingredient.

The weight ratio between the agricultural composition of the present invention and a plant growth regulator mixed with or used together with the aforementioned agricultural composition is generally between 1 : 0.00001 and 1 : 100, and preferably between 1 : 0.0001 and 1 : 1, in terms of active ingredient.

The weight ratio between the agricultural composition of the present invention and a fertilizer or soil improver mixed with or used together with the aforementioned agricultural composition is generally between 1 : 0.1 and 1 : 1000, and preferably between 1 : 1 and 1 : 200.

The type of a method of applying the agricultural composition of the present invention is not particularly limited, as long as the agricultural composition of the present invention can be substantially applied by the method. Examples of such an application method: include application of the present agricultural composition to the plant bodies of useful plants, such as foliar application; application of the present agricultural composition to a field in which useful plants are planted or are to be planted, such as a soil treatment; and application of the present agricultural composition to seeds, such as seed sterilization.

The applied amount of the agricultural composition of the present invention differs depending on a climatic condition, a dosage form, an application period, an application method, an application site, a target disease, target crops, etc. In terms of the weight of the present compound contained in the agricultural composition of the present invention, the applied amount is generally between 1 and 500 g, and preferably 2 and 200 g, per 10 ares. An emulsion, a wettable powder, a suspension, etc. are generally diluted with water for application. In such a case, the concentration of the present compound after dilution is generally between 0.0005% and 2 % by weight, and preferably between 0.005% and 1% by weight. On the other hand, a powder, a granule, etc. are directly applied without being diluted. When the agricultural composition of the present invention is applied to seeds, the present compound contained in the agricultural composition of the present invention is applied within a range generally between 0.001 and 100 g, and preferably between 0.01 and 50 g, per kg of seeds.

The agricultural composition of the present invention can be used as a reagent for controlling plant diseases caused by plant pathogenic microbes in agricultural lands such as fields, paddy fields, lawns, or orchards. The agricultural composition of the present invention is able to control or prevent plant diseases caused by plant pathogenic microbes in agricultural lands for cultivating the following "crops".

Crops: corn, rice, wheat, barley, rye, oat, sorghum, cotton, soybean, peanut, buckwheat, beet, rapeseed, sunflower, sugar cane, tobacco, etc.; vegetables: solanaceous vegetables (eggplant, tomato, pimento, pepper, potato, etc.), cucurbitaceous vegetables (cucumber, pumpkin, zucchini, water melon, melon, etc.), cruciferous vegetables (Japanese radish, white turnip, horseradish, kohlrabi, Chinese cabbage, cabbage, leaf mustard, broccoli, cauliflower, etc.), asteraceous vegetables (burdock, crown daisy, artichoke, lettuce, etc.), liliaceous vegetables (green onion, onion, garlic, and asparagus), ammiaceous vegetables (carrot, parsley, celery, parsnip, etc.), chenopodiaceous vegetables (spinach, Swiss chard, etc.), lamiaceous vegetables (Perilla frutescens, mint, basil, etc.), strawberry, sweet potato, Dioscorea japonica, colocasia, etc.,
flowers;
foliage plants;
fruits: pomaceous fruits (apple, pear, Japanese pear, Chinese quince, quince, etc.), stone fleshy fruits (peach, plum, nectarine, Prunus mume, cherry fruit, apricot, prune, etc.), citrus fruits (Citrus unshiu, orange, lemon, rime, grapefruit, etc.), nuts (chestnuts, walnuts, hazelnuts, almond, pistachio, cashew nuts, macadamia nuts, etc.), berries (blueberry, cranberry, blackberry, raspberry, etc.), grape, kaki fruit, olive, Japanese plum, banana, coffee, date palm, coconuts, etc., and
trees other than fruit trees; tea, mulberry, flowering plant, roadside trees (ash, birch, dogwood, Eucalyptus, Ginkgo biloba, lilac, maple, Quercus, poplar, Judas tree, Liquidambar formosana, plane tree, zelkova, Japanese arborvitae, fir wood, hemlock, juniper, Pinus, Picea, and Taxus cuspidate), etc.

The aforementioned "crops" include crops, to which resistance to HPPD inhibitors such as isoxaflutole, ALS inhibitors such as imazethapyr or thifensulfuron-methyl, EPSP synthetase inhibitors, glutamine synthetase inhibitors, and herbicides such as bromoxynil, has been conferred by a classical breeding method or genetic recombination.

Examples of "crops" to which resistance is conferred by a classical breeding method include Clearfield (registered trademark) canola that is resistant to imidazolinone herbicides such as imazethapyr, and STS soybean that is resistant to sulfonylurea ALS inhibitory herbicides such as thifensulfuron-methyl. In addition, examples of "crops" to which resistance is conferred by genetic recombination include corn varieties resistant to glyphosate or glufosinate. Such corn varieties have already been on the market with product names such as "RoundupReady (registered trademark)" and "LibertyLink (registered trademark)."

The aforementioned "crops" include genetically recombinant crops produced using such genetic recombination techniques, which, for example, are able to synthesize selective toxins as known in genus Bacillus.

Examples of toxins expressed in such genetically recombinant crops include: insecticidal proteins derived from *Bacillus cereus* or *Bacillus popilliae;* δ-endotoxins such as Cry1Ab, Cry1Ac, Cry1F, Cry1Fa2, Cry2Ab, Cry3A, Cry3Bb1 or Cry9C, derived from *Bacillus thuringiensis;* insecticidal proteins such as VIP1, VIP2, VIP3, or VIP3A; insecticidal proteins derived from nematodes; toxins generated by animals, such as scorpion toxin, spider toxin, bee toxin, or insect-specific neurotoxins; mold fungi toxins; plant lectin; agglutinin; protease inhibitors such as a trypsin inhibitor, a serine protease inhibitor, patatin, cystatin, or a papain inhibitor; ribosome-inactivating proteins (RIP) such as lycine, corn-RIP, abrin, luffin, saporin, or briodin; steroid-metabolizing enzymes such as 3-hydroxysteroid oxidase, ecdysteroid-UDP-glucosyl transferase, or cholesterol oxidase; an ecdysone inhibitor; HMG-COA reductase; ion channel inhibitors such as a sodium channel inhibitor or calcium channel inhibitor; juvenile hormone esterase; a diuretic hormone receptor; stilbene synthase; bibenzyl synthase; chitinase; and glucanase.

Moreover, toxins expressed in such genetically recombinant crops also include: hybrid toxins of δ-endotoxin proteins such as Cry1Ab, Cry1Ac, Cry1F, Cry1Fa2, Cry2Ab, Cry3A, Cry3Bb1 or Cry9C, and insecticidal proteins such as VIP1, VIP2, VIP3 or VIP3A; partially deleted toxins; and modified toxins. Such hybrid toxins are produced from a new combination of the different domains of such proteins, using a genetic recombination technique. As a partially deleted toxin, Cry1Ab comprising a deletion of a portion of an amino acid sequence has been known. A modified toxin is produced by substitution of one or multiple amino acids of natural toxins.

Examples of such toxins and recombinant plants capable of synthesizing such toxins are described in EP-A-0 374 753, WO 93/07278, WO 95/34656, EP-A-0 427 529, EP-A-451 878, WO 03/052073, etc.

Toxins contained in such recombinant plants are able to impart resistance particularly to insect pests belonging to Coleoptera, Diptera and Lepidoptera, to the plants.

Furthermore, genetically recombinant plants, which comprise one or multiple insecticidal pest-resistant genes and which express one or multiple toxins, have already been known, and some of such genetically recombinant plants have already been on the market. Examples of such genetically recombinant plants include YieldGard (registered trademark) (a corn variety for expressing Cry1Ab toxin), YieldGard Rootworm (registered trademark) (a corn variety for expressing Cry3Bb1 toxin), YieldGard Plus (registered trademark) (a corn variety for expressing Cry1Ab and Cry3Bb1 toxins), Herculex I (registered trademark) (a corn variety for expressing phosphinotricine N-acetyl transferase (PAT) so as to impart resistance to Cry1Fa2 toxin and gluphosinate), NuCOTN33B (a cotton variety for expressing Cry1Ac toxin), Bollgard I (registered trademark) (a cotton variety for expressing Cry1Ac toxin), Bollgard II (registered trademark) (a cotton variety for expressing Cry1Ac and Cry2Ab toxins), VIPCOT (registered trademark) (a cotton variety for expressing VIP toxin), NewLeaf (registered trademark) (a potato variety for expressing Cry3A toxin), NatureGard (registered trademark) Agrisure (registered trademark) GT Advantage (GA21 glyphosate-resistant trait), Agrisure (registered trademark) CB Advantage (Bt11 corn borer (CB) trait), and Protecta (registered trademark).

The aforementioned "crops" also include crops produced using a genetic recombinant technique, which have ability to generate antipathogenic substances having selective action.

A PR protein and the like have been known as such antipathogenic substances (PRPs, EP-A-0 392 225). Such antipathogenic substances and genetically recombinant crops that generate them are described in EP-A-0 392 225, WO 95/33818, EP-A-0 353 191, etc.

Examples of such antipathogenic substances expressed in genetically recombinant crops include: ion channel inhibitors such as a sodium channel inhibitor or a calcium channel inhibitor (KP1, KP4 and KP6 toxins, etc., which are produced by viruses, have been known); stilbene synthase; bibenzyl synthase; chitinase; glucanase; a PR protein; and antipathogenic substances generated by microorganisms, such as a peptide antibiotic, an antibiotic having a hetero ring, a protein factor associated with resistance to plant diseases (which is called a plant disease-resistant gene and is described in WO 03/000906).

Examples of plant diseases that can be controlled by the present invention include plant diseases caused by plant pathogenic microbes. More specific examples include the following plant diseases, but examples are not limited thereto.

Blast (Magnaporthe grisea), Helminthosporium leaf spot (Cochliobolus miyabeanus), sheath blight (Rhizoctonia solani), bakanae disease (Gibberella fujikuroi) and downy mildew (Sclerophthora macrospora) of rice; powdery mildew (Erysiphe graminis), Fusarium head blight (Fusarium graminearum, F. avenacerum, F. culmorum, Microdochium nivale), rust (Puccina striiformis, P. graminis, P. recondita, P. hordei), snow mold (Typhula sp., Micronectriella nivalis), loose smut (Ustilago tritici, U. nuda), bunt (Tilletia caries), eyespot (Pseudocercosporella herpotrichoides), scald (Rhynchosporium secalis), leaf blotch (Septoria tritici), glume blotch (Leptosphaeria nodorum), net blotch (Pyrenophora teres Drechsler), take-all (Gaeumannomyces graminis) and yellow spot (Pyrenophoratritici-repentis) of small grains; melanose (Diaporthe citri), scab (Elsinoe fawcetti) and penicillium rot (Penicillium digitatum, P. italicum) of citrus; blossom blight (Monilinia mali), canker (Valsa ceratosperma, powdery mildew (Podosphaera leucotricha), Alternaria leaf spot (Alternaria alternata apple pathotype), scab (Venturia inaequalis) and bitter rot (Glomerella cingulata) of apple; scab (Venturia nashicola, V. pirina), black spot (Alternaria alternata Japanese pear pathotype) and rust (Gymnosporangium haraeanum) of pear; brown rot (Monilinia fructicola), scab (Cladosporium carpophilum) and phomopsis rot (Phomopsis sp.) of peach; anthracnose (Elsinoe ampelina), ripe rot (Glomerella cingulata), powdery mildew (Uncinula necator), rust (Phakopsora ampelopsidis), black rot (Guignardia bidwellii) and downy mildew (Plasmopara viticola) of grape; anthracnose (Gloeosporium kaki) and leaf spot (Cercospora kaki, Mycosphaerella nawae) of Japanese persimmon; anthracnose (Colletotrichum lagenarium), powdery mildew (Sphaerotheca fuliginea), gummy stem blight (Mycosphaerella melonis), Fusarium wilt (Fusarium oxysporum), downy mildew (Pseudoperonospora cubensis), Phytophthora rot (Phytophthora sp.) and damping-off (Pythium sp.) of gourd; early blight (Alternaria solani), leaf mold (Cladosporium fulvum) and late blight (Phytophthora infestans) of tomato; brown spot (Phomopsis vexans) and powdery mildew (Erysiphe cichoracearum) of eggplant; Alternaria leaf spot (Alternaria japonica), white spot (Cercosporella brassicae), clubroot (Plasmodiophora brassicae) and downy mildew (Peronospora parasitica) of cruciferous vegetables; rust (Puccinia allii) of welsh onion; purple seed stain (Cercospora kikuchii), sphaceloma scad (Elsinoe glycines), pod and stem blight (Diaporthe phaseolorum var. sojae) and rust (Phakopsora pachyrhizi) of soybean; anthracnose (Colletotrichum lindemthianum) of kidney bean; leaf spot (Cercospora personata), brown leaf spot (Cercospora arachidicola) and southern blight (Sclerotium rolfsii of peanut; powdery mildew (Erysiphe pisi) of garden pea; early blight (Alternaria solani), late blight (Phytophthora infestans) and verticillium wilt (Verticillium albo-atrum, V. dahliae, V. nigrescens) of potato; powdery mildew (Sphaerotheca humuli) of strawberry; net blister blight (Exobasidium reticulatum), white scab (Elsinoe leucospila), gray blight (Pestalotiopsis sp.) and anthracnose (Colletotrichum theae-sinensis) of tea; brown spot (Alternaria longipes), powdery mildew (Erysiphe cichoracearum), anthracnose (Colletotrichum tabacum), downy mildew (Peronospora tabacina) and black shank (Phytophthora nicotianae) of tabacco; Cercospora leaf spot (Cercospora beticola), leaf blight (Thanatephorus cucumeris), root rot (Thanatephorus cucumeris) and Aphanomyces root rot (Aphanomyces sochlioides) of sugar beet; black spot (Diplocarpon rosae) and powdery mildew (Sphaerotheca pannosa) of rose; leaf blight (Septoria chrysanthemi-indici) and white rust (Puccinia horiana) of chrysanthemum; leaf blight (Botrytis cinerea, B. byssoidea, B. squamosa) and gray-mold neck rot (Botrytis alli) and small sclerotinial neck rot (Botrytis squamosa) of onion; gray mold (Botrytis cinerea) and Sclerotinia rot (Sclerotinia sclerotiorum) of various groups of crops; Alternaria leaf spot (Alternaria brassicicola) of Japanese radish; dollar spot (Sclerotinia homeocarpa) and brown patch and large patch (Rhizoctonia solani) of turfgrass; rust (Hemileia vastatrix) of coffee; and sigatoka (Mycosphaerella fijiensis, Mycosphaerella musicola) of banana.

### EXAMPLES

Next, the present invention will be illustrated further specifically by examples such as production examples, formulation examples, test examples and the like, but the present invention is not limited to these examples.

### Production Example 1

To a mixture of 0.16 g of 2-amino-3-(1H-pyrazol-4-yl)pyridine and 3 ml of dimethylformamide was added, under ice cool, 52 mg of sodium hydride, and the mixture was stirred at room temperature for 30 minutes. Then, 0.48 g of 2-fluoro-3-methoxyphenyl methyl methanesulfonate was added, and the mixture was stirred at room temperature for 1 hour. Thereafter, to the reaction mixture was added water, and the mixture was extracted with ethyl acetate, and washed sequentially with water and saturated saline, then, dried over anhydrous sodium sulfate, and concentrated under reduced pressure. The resultant residue was subjected to silica gel chromatography to obtain 0.21 g of 2-amino-3-{1-[(2-fluoro-3-methoxyphenyl)methyl]-1H-pyrazol-4-yl}pyridine (hereinafter, referred to as the present compound 1518). 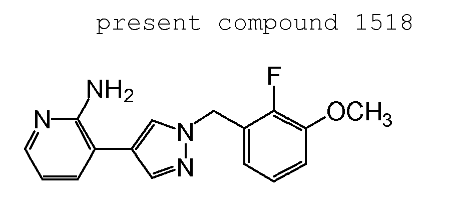 1H-NMR (CDCl3) δ: 3.89 (3H, s), 4.60 (2H, br s), 5.40 (2H, d, J = 1.5 Hz), 6.70 (1H, dd, J = 7.4, 5.0 Hz), 6.82-6.86 (1H, m), 6.93-6.98 (1H, m), 7.05-7.09 (1H, m), 7.40 (1H, dd, J = 7.3, 1.7 Hz), 7.67 (1H, s), 7.72 (1H, s), 8.00 (1H, dd, J = 4.9, 1.7 Hz).

### Production Example 2

To a mixture of 0.48 g of 2-amino-3-ethynylpyridine, 1.21 g of triethylamine and 8 ml of tetrahydrofuran was added, under ice cool, a mixture of 2.3 g of (4-phenoxyphenyl)acetohydroxamoyl chloride and 8 ml of tetrahydrofuran, and the mixture was stirred at room temperature for 1 hour, then, at 50°C for 2 hours. Thereafter, to the reaction mixture was added water, and the mixture was extracted with ethyl acetate, and washed sequentially with water and saturated saline, then, dried over anhydrous sodium sulfate, and concentrated under reduced pressure. The resultant residue was subjected to silica gel chromatography to obtain 0.31 g of 2-amino-3-{[3-(4-phenoxyphenyl)methyl]-isoxazol-5-yl}pyridine (hereinafter, referred to as the present compound 3). 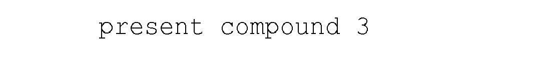 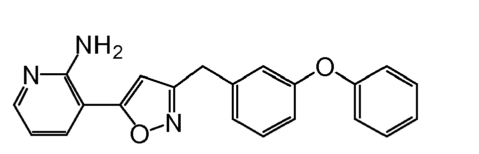 1H-NMR (CDCl3) δ: 4.04 (2H, s), 5.39 (2H, s), 6.28 (1H, s), 6.72 (1H, dd, J = 7.8, 4.9 Hz), 6.96-7.02 (4H, m), 7.08-7.12 (1H, m), 7.23-7.26 (2H, m), 7.31-7.35 (2H, m), 7.72 (1H, dd, J = 7.8, 1.7 Hz), 8.14 (1H, dd, J = 4.8, 1.8 Hz)

### Production Example 3

Zero point two zero grams (0.20 g) of 2-amino-N-hydroxy-nicotinamidine, 0.30 g of 4-phenoxyphenylacetic acid, 0.70 g of (benzotriazol-1-yloxy)tris(dimethylamino)phosphonium hexafluorophosphate, 0.64 ml of triethylamine and 5 ml of DMF were mixed, and mixed at 80°C for 3 hours. To the reaction mixture was added water, and the generated solid was filtrated, and dried under reduced pressure. The resultant solid and pyridine were mixed, and mixed at 130°C for 3 hours. The reaction mixture was cooled down to room temperature, then, concentrated under reduced pressure, and the resultant residue was subjected to silica gel chromatography to obtain 40 mg of 2-amino-3-{[5-(4-phenoxyphenyl)methyl]-oxadiazol-3-yl}pyridine (hereinafter, referred to as the present compound 5506). 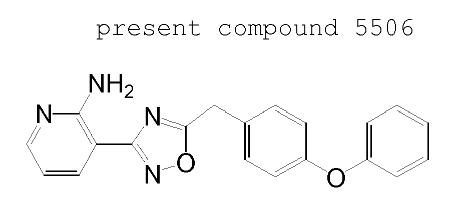 1H-NMR (CDCl3) δ: 4.27 (2H, s), 6.35 (2H, s), 6.74 (1H, dd, J = 7.7, 4.8 Hz), 6.95-7.36 (9H, m), 8.17 (1H, dd, J = 5.0, 1.8 Hz), 8.33 (1H, dd, J = 7.7, 1.7 Hz)

Next, reference production examples will be shown for illustrating production of a production intermediate of the present compound.

### Reference Production Example 1

To a mixture of 1.18 g of 2-amino-3-ethynyl-pyridine, 0.57 g of copper iodide and 20 ml of a mixed solvent composed of dimethylformamide and methanol (9:1) was added 1.18 g of trimethylsilyl azide, and the mixture was stirred at 100°C for 2 hours. The reaction mixture was cooled down too room temperature, filtrated through Cerite (registered trademark), and the filtrate was concentrated under reduced pressure. The resultant residue was subjected to silica gel chromatography to obtain 0.82 g of 2-amino-3-(1H-1,2,3-triazol-4-yl)pyridine.

### 2-amino-3-(1H-1,2,3-triazol-4-yl)pyridine

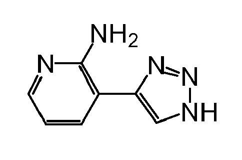

### Reference Production Example 2

To a mixture of 8.0 g of 2-amino-3-bromopyridine, 9.1 g of trimethylsilylacetylene, 0.88 g of copper iodide, 12.0 g of N-ethyldiisopropylamine and N-methyl-2-pyrrolidinone was added 2.7 g of tetrakis(triphenylphosphine)palladium, and the mixture was stirred at 100°C for 3 hours. Thereafter, to the reaction mixture was added water, and the mixture was extracted with ethyl acetate three times, and the organic layer was washed sequentially with water and saturated saline, then, dried over anhydrous sodium sulfate, and concentrated under reduced pressure. The resultant residue was subjected to silica gel chromatography to obtain 6.9 g of 2-amino-3-trimethylsilanylethynyl-pyridine.

### 2-amino-3-(2-trimethylsilylethynyl)pyridine

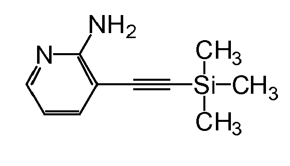

### Reference Production Example 3

To a mixture of 6.9 g of 2-amino-3-(2-trimethylsilylethynyl)pyridine and 40 ml of tetrahydrofuran was added 5 ml of a 1 mol/liter tetrahydrofuran solution of tetrabutylammonium fluoride, and the mixture was stirred at room temperature for 1 hour. Thereafter, to the reaction mixture was added water, and the mixture was extracted with ethyl acetate three times, and the organic layer was washed sequentially with water and saturated saline, then, dried over anhydrous sodium sulfate, and concentrated under reduced pressure. The resultant residue was subjected to silica gel chromatography to obtain 2.2 g of 2-amino-3-ethynylpyridine.

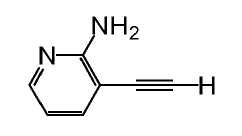

Next, formulation examples are shown. Parts are by weight.

### Formulation Example 1

Fifty parts (50 parts) of any one compound among present compounds 1 to 454, 501 to 954, 1001 to 1454, 1501 to 1954, 2001 to 2454, 2501 to 2954, 3001 to 3454, 3501 to 3954, 4001 to 4454, 4501 to 4954, 5001 to 5454, 5501 to 5953 and 5764, 3 parts of calcium ligninsulfonate, 2 parts of magnesium laurylsulfate and 45 parts of synthetic hydrated silicon oxide are thoroughly pulverized and mixed, to obtain wettable powders.

### Formulation Example 2

Twenty parts (20 parts) of any one compound among present compounds 1 to 454, 501 to 954, 1001 to 1454, 1501 to 1954, 2001 to 2454, 2501 to 2954, 3001 to 3454, 3501 to 3954, 4001 to 4454, 4501 to 4954, 5001 to 5454, 5501 to 5953 and 5764 and 1.5 parts of sorbitan trioleate are mixed with 28.5 parts of an aqueous solution containing 2 parts of polyvinyl alcohol, and the mixture is finely pulverized by a wet pulverization method, then, into this is added 40 parts of an aqueous solution containing 0.05 parts of xanthan gum and 0.1 part of aluminum magnesium silicate, further, 10 parts of propylene glycol is added and mixed with stirring, to obtain flowable formulations.

### Formulation Example 3

Two parts (2 parts) of any one compound among present compounds 1 to 454, 501 to 954, 1001 to 1454, 1501 to 1954, 2001 to 2454, 2501 to 2954, 3001 to 3454, 3501 to 3954, 4001 to 4454, 4501 to 4954, 5001 to 5454, 5501 to 5953 and 5764, 88 parts of kaolin clay and 10 parts of talc are thoroughly pulverized and mixed, to obtain powders.

### Formulation Example 4

Five parts (5 parts) of any one compound among present compounds 1 to 454, 501 to 954, 1001 to 1454, 1501 to 1954, 2001 to 2454, 2501 to 2954, 3001 to 3454, 3501 to 3954, 4001 to 4454, 4501 to 4954, 5001 to 5454, 5501 to 5953 and 5764, 14 parts of polyoxyethylene styryl phenyl ether, 6 parts of calcium dodecylbenzenesulfonate and 75 parts of xylene are thoroughly mixed, to obtain emulsifiable concentrates.

### Formulation Example 5

Two parts (2 parts) of any one compound among present compounds 1 to 454, 501 to 954, 1001 to 1454, 1501 to 1954, 2001 to 2454, 2501 to 2954, 3001 to 3454, 3501 to 3954, 4001 to 4454, 4501 to 4954, 5001 to 5454, 5501 to 5953 and 5764, 1 part of synthetic hydrated silicon oxide, 2 parts of calcium ligninsulfonate, 30 parts of bentonite and 65 parts of kaolin clay are thoroughly pulverized and mixed, then, water is added to this and the mixture is thoroughly kneaded, and granulated and dried, to obtain granules.

### Formulation Example 6

Ten parts (10 parts) of any one compound among present compounds 1 to 454, 501 to 954, 1001 to 1454, 1501 to 1954, 2001 to 2454, 2501 to 2954, 3001 to 3454, 3501 to 3954, 4001 to 4454, 4501 to 4954, 5001 to 5454, 5501 to 5953 and 5764; 35 parts of white carbon containing 50 parts of polyoxyethylene alkyl ether sulfate ammonium salt; and 55 parts of water are mixed, and finely pulverized by a wet pulverization method, to obtain formulations.

The following test examples will show that the present invention is useful for control of a plant disease.

The controlling effect was evaluated by visually observing the area of disease spots on a test plant in checking and by comparing the area of disease spots on a plant treated with a controlling agent of the present invention with the area of disease spots on a non-treated plant.

### Test Example 1

### Test of effect in preventing infection by cucumber powdery mildew (Sphaerotheca fuliginea)

A plastic pot was stuffed with sandy soil, and cucumber (variety: Sagami-Hanjiro) was seeded on this and allowed to grow in a greenhouse for 12 days. Thereafter, the present compounds 1503 and 2503 were processed into flowable formulations according to Formulation Example 6, then, the formulations were diluted with water to attain a given concentration (500 ppm), and then subjected to foliar application so as to fully adhere to the leaf surfaces of the cucumber. After completion of the foliar application, the plant was air-dried, and inoculated with spores of the pathogen by sprinkling. After inoculation, the plant was placed in a greenhouse of 23°C for 10 days, then, the infected area was checked. As a result, the infected area on the plant treated with the present compounds 1503 and 2503 was 30% or less of the infected area on a non-treated plant.

### Test Example 2

### Test of effect in preventing infection by wheat leaf blotch (Septoria tritici)

A plastic pot was stuffed with sandy soil, and wheat (variety: Apogee) was seeded on this and allowed to grow in a greenhouse for 10 days. The present compounds 778, 1503, 1518 and 2503 were processed into flowable formulations according to Formulation Example 6, then, the formulations were diluted with water to attain a given concentration (500 ppm), and then subjected to foliar application so at to fully adhere to the leaf surfaces of the wheat. After completion of the foliar application, the plant was air-dried, and two days after, inoculated with an aqueous suspension of Septoria tritici spores by spraying. After inoculation, the plant was first allowed to stand under humid condition at 18°C for 3 days, further, allowed to stand for 14 to 18 days under illumination, then, the lesion area was checked. As a result, the lesion area on the plant treated with the present compounds 778, 1503, 1518 and 2503 was 30% or less of the affected area on a non-treated plant.

### Test Example 3

### Test of effect in preventing infection by cucumber gray mold (Botrytis cinerea)

A plastic pot was stuffed with sandy soil, and cucumber (plant variety: Sagami-Hanjiro) was seeded on this and allowed to grow in a greenhouse for 12 days. The present compounds 3, 6, 251, 778, 1506, 1518, 1751, 2003 and 5751 were processed into flowable formulations according to Formulation Example 6, then, the formulations were diluted with water to attain a given concentration (500 ppm), and then subjected to foliar application so as to fully adhere to the leaf surfaces of the cucumber. After completion of the foliar application, the plant was air-dried, and a Botrytis cinerea spore-containing PDA medium was placed on the leaf surfaces of the cucumber. After inoculation, the plant was allowed to stand under humid condition at 12°C for 4 days, then, the lesion area was checked. As a result, the affected area on the plant treated with the present compounds 3, 6, 251, 778, 1506, 1518, 1751, 2003 and 5751 was 30% or less of the lesion area on a non-treated plant.

### Test Example 4

### Test of effect in preventing infection by cucumber Sclerotinia rot (Sclerotinia sclerotiorum)

A plastic pot was stuffed with sandy soil, and cucumber (variety: Sagami-Hanjiro) was seeded on this and allowed to grow in a greenhouse for 12 days. The present compounds 3, 6, 251, 1503, 1506, 1518, 1751, 2003, 2503, 2506, 2251, 2751 and 5751 were processed into flowable formulations according to Formulation Example 6, then, the formulations were diluted with water to attain a given concentration (500 ppm), and then subjected to foliar application so as to fully adhere to the leaf surfaces of the cucumber. After completion of the foliar application, the plant was air-dried, and a Sclerotinia sclerotiorum micelium-containing PDA medium was placed on the leaf surfaces of the cucumber. After the inoculation, the plant was allowed to stand under humid condition at 18°C for 4 days, then, the lesion area was checked. As a result, the lesion area on the plant treated with the present compounds 3, 6, 251, 1503, 1506, 1518, 1751, 2003, 2503, 2506, 2251, 2751 and 5751 was 30% or less of the lesion area on a non-treated plant.

### Test Example 5

### Test of effect in preventing infection by Japanese radish Alternaria leaf spot (Alternaria brassicicola)

A plastic pot was stuffed with sandy soil, and Japanese radish (variety: Wase-Shijunichi) was seeded on this and allowed to grow in a greenhouse for 5 days. The present compounds 2751 was processed into a flowable formulation according to Formulation Example 6, then, the formulation was diluted with water to attain a given concentration (500 ppm), and then subjected to foliar application so as to fully adhere to the leaf surfaces of the radish. After completion of the foliar application, the plant was air-dried, and inoculated with an aqueous suspension of Alternaria brassicicola spores by spraying. After inoculation, the plant was first allowed to stand under humid condition at 24°C for 1 day, further, allowed to stand in a greenhouse for 3 days, then, the affected area was checked. As a result, the lesion area on the plant treated with the present compound 2751 was 30% or less of the lesion area on a non-treated plant.

### Test Example 6

Test of effect in preventing infection by rice blast

### (Magnaporthe grisea)

A plastic pot was stuffed with a soil, and rice (variety: Nihonbare) was seeded on this and allowed to grow in a greenhouse for 12 days. The present compounds 3, 6, 251, 778, 1503, 1506, 1518, 1751, 2003, 2503, 2506 and 5506 were processed into flowable formulations according to Formulation Example 6, then, the formulations were diluted with water to attain a given concentration (500 ppm), and then subjected to foliar application so as to fully adhere to the leaf surfaces of the rice. After completion of the foliar application, the plant was air-dried, and pots which contained rice seedlings which were infected by rice blast were allowed to stand still around the applied plant. All the rice plants were allowed to stand under humid condition only in night, and 5 days after inoculation, the lesion area was checked. As a result, the lesion area on the plant treated with the present compounds 3, 6, 251, 778, 1503, 1506, 1518, 1751, 2003, 2503, 2506 and 5506 was 30% or less of the lesion area on a non-treated plant.

### Test Example 7

### Test of effect in preventing infection by kidney bean stem rot (Sclerotinia sclerotiorum)

A plastic pot was stuffed with sandy soil, and kidney bean (variety: Nagauzura) was seeded on this and allowed to grow in a greenhouse for 8 days. The present compound 778 was processed into a flowable formulation according to Formulation Example 6, then, the formulation was diluted with water to attain a given concentration (500 ppm), and then subjected to foliar application so as to fully adhere to the leaf surfaces of the common bean. After completion of the foliar application, the plant was air-dried, a Sclerotinia sclerotiorum micelium-containing PDA medium was placed on the leaf surfaces of the kidney bean. After the inoculation, all the kidney bean plants were allowed to stand under humid condition only in night, and 5 days after the inoculation, the lesion area was checked. As a result, the lesion area on the plant treated with the present compound 778 was 30% or less of the lesion area on a non-treated plant.

### Test Example 8

### Test of soil treatment (drenching) for tomato wilt

A plastic pot is stuffed with soil contaminated with tomato wilt (*Fusarium oxysporum*), and tomato (variety: Patio) is seeded on this. The present compound is processed into a flowable formulation according to Formulation Example 6, then, the formulation is diluted with water, and the soil is drenched with the above water-diluted liquid with an amount of 10 mg of the present compound per plastic pot. After cultivation for one month in a greenhouse, the growing condition of the plant is checked. As a result, it can be confirmed that the plant cultivated in the soil which has been drenched with the present compound (treated group) has less disease symptoms of tomato wilt, as compared with a plant cultivated, in the same manner as in the treated group, in a soil which has not been drenched with the present compound (non-treated group).

### Test Example 9

### Test of soil treatment (drenching) for potato Verticillium wilt

A plastic pot is stuffed with soil contaminated with potato Verticillium wilt (Verticillium albo-atrum, v. dahliae, v. nigrescens), and potato (variety: Danshaku) is planted on this. The present compound is processed into a flowable formulation according to Formulation Example 6, then, the formulation is diluted with water, and the soil is drenched with the above water-diluted liquid with an amount of 10 mg of the present compound per plastic pot. After cultivation for 2 months in a greenhouse, the growing condition of the plant is checked. As a result, it can be confirmed that the plant cultivated in the soil which has been drenched with the present compound (treated group) has less disease symptoms of potato Verticillium wilt as compared with a plant cultivated, in the same manner as in the treated group, in a soil which has not been drenched with the present compound (non-treated group).

### Test Example 10

### Test of seed treatment for rice bakanae disease

The present compound is processed into a flowable formulation according to Formulation Example 6, then, the formulation is diluted to 2000 ppm with water, and rough rice contaminated with rice bakanae disease (Gibberella fujikuroi) is seed-treated by an immersion method. Thereafter, the above seed-treated rough rice is seeded on a nursery box stuffed with a soil, and cultivated in a greenhouse for one month, then, growing condition of the plant is checked. As a result, it can be confirmed that the cultivated plant which has been seed-treated with the present compound (treated group) has less disease symptoms of rice bakanae disease as compared with a plant cultivated, in the same manner as in the treated district, without seed treatment (non-treated group).

### Test Example 11

### Test of seed treatment for wheat take-all

The present compound is processed into a flowable formulation according to Formulation Example 6, then, seeds contaminatetd with wheat take-all (Gaeumannomyces graminis) are treated, according to a smearing method by a seed dresser, with the above flowable formulation with an amount of 200 g of the present compound per 100 kg of seeds. Thereafter, the above contaminated seeds that have been seed-treated are seeded on a plastic pot stuffed with sandy soil, and cultivated in a greenhouse for one month, then, growing condition of the plants is checked. As a result, it can be confirmed that the cultivated plant which has been seed-treated with the present compound (treated group) has less disease symptoms of wheat take-all as compared with a plant cultivated, in the same manner as in the treated district, without seed treatment (non-treated group).

### Industrial Applicability

The present invention is capable of controlling or preventing a plant disease due to a plant pathogen (excluding Aspergillus).

## Claims

1. An agricultural composition comprising a compound represented by the formula (1) or a salt of the compound or a hydrate thereof, for controlling a plant disease due to a plant pathogen (excluding Aspergillus): 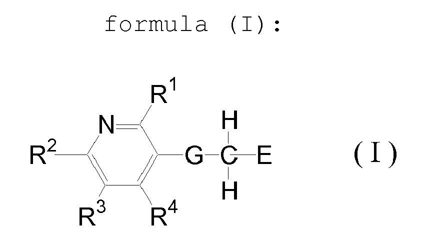 [wherein,
G represents a di-valent aromatic hetero 5-membered ring optionally having a halogen atom or a C1-3 alkyl group (with the proviso that a hetero atom of said aromatic hetero 5-membered ring is a nitrogen atom, an oxygen atom or a sulfur atom);
R¹ represents a hydrogen atom or an amino group;
R², R³ and R⁴ represent each independently a hydrogen atom or a member selected from the following substituent group a-1 and substituent group a-2; and
E represents a 5 to 10-membered heterocyclic group optionally having 1 to 5 members selected from the following substituent group a-1 and substituent group a-2 or a C6 to 10 aryl group optionally having 1 to 5 members selected from the following substituent group a-1 and substituent group a-2.] [Substituent Group a-1]
halogen atom, amino group, hydroxyl group, mercapto group, cyano group, formyl group, carboxyl group, C1-8 alkyl group, C2-9 alkenyl group, C2-9 alkynyl group, C3-8 cycloalkyl group, C6-10 aryl group, 5 to 10-membered heterocyclic group, C3-8 cycloalkyl C1-6 alkyl group, C3-8 cycloalkylidene C1-6 alkyl group, C6-10 aryl C1-6 alkyl group, C6-10 aryl C2-6 alkenyl group, 5 to 10-membered heterocyclic C1-6 alkyl group, C1-8 alkoxy group, C3-9 alkenyloxy group, C3-9 alkynyloxy group, C3-8 cycloalkoxy group, C6-10 aryloxy group, 5 to 10-membered heterocyclic oxy group, C3-8 cycloalkyl C1-6 alkoxy group, C6-10 aryl C1-6 alkoxy group, 5 to 10-membered heterocyclic C1-6 alkoxy group, C1-8 alkylthio group, C3-9 alkenylthio group, C3-9 alkynylthio group, C3-8 cycloalkylthio group, C6-10 arylthio group, C3-8 cycloalkyl C1-6 alkylthio group , C6-10 aryl C1-6 alkylthio group, 5 to 10-membered heterocyclic C1-6 alkylthio group, mono-C1-8 alkylamino group, mono-C3-9 alkenylamino group, mono-C3-9 alkynylamino group, mono-C3-8 cycloalkylamino group, mono-C6-10 arylamino group, mono-C3-8 cycloalkyl C1-6 alkylamino group, mono-C6-10 aryl C1-6 alkylamino group, mono-5 to 10-membered heterocyclic C1-6 alkylamino group, di-C1-6 alkylamino group, N-C3-9 alkenyl-N-C1-8 alkylamino group, N-C3-9 alkynyl-N-C1-8 alkylamino group, N-C3-8 cycloalkyl-N-C1-8 alkylamino group, N-C6-10 aryl-N-C1-8 alkylamino group, N-C3-8 cycloalkyl C1-6 alkyl-N-C1-8 alkylamino group, N-C6-10 aryl C1-6 alkyl-N-C1-8 alkylamino group, N-5 to 10-membered heterocyclic C1-6 alkyl-N-C1-8 alkylamino group, C1-8 alkylcarbonyl group, C6-10 arylcarbonyl group, C1-8 alkylsulfonyl group, C1-8 alkylsulfinyl group, C6-10 aryloxy C1-6 alkyl group, 5 to 10-membered heterocyclic oxy C1-6 alkyl group and C1-3 trialkylsilyl C2-9 alkynyl group; and
[Substituent Group a-2]
C1-8 alkyl group, C2-9 alkenyl group, C2-9 alkynyl group, C3-8 cycloalkyl group, C6-10 aryl group, 5 to 10-membered heterocyclic group, C3-8 cycloalkyl C1-6 alkyl group, C6-10 aryl C1-6 alkyl group, 5 to 10-membered heterocyclic C1-6 alkyl group, C1-8 alkoxy group, C3-9 alkenyloxy group, C3-9 alkynyloxy group, C3-8 cycloalkoxy group, C6-10 aryloxy group, 5 to 10-membered heterocyclic oxy group, C3-8 cycloalkyl C1-6 alkoxy group, C6-10 aryl C1-6 alkoxy group, 5 to 10-membered heterocyclic C1-6 alkoxy group, C1-8 alkylthio group, C3-9 alkenylthio group, C3-9 alkynylthio group, C3-8 cycloalkylthio group, C6-10 arylthio group, C3-8 cycloalkyl C1-6 alkylthio group , C6-10 aryl C1-6 alkylthio group, 5 to 10-membered heterocyclic C1-6 alkylthio group, mono-C1-8 alkylamino group, mono-C3-9 alkenylamino group, mono-C3-9 alkynylamino group, mono-C3-8 cycloalkylamino group, mono-C6-10 arylamino group, mono-C3-8 cycloalkyl C1-6 alkylamino group, mono-C6-10 aryl C1-6 alkylamino group, mono-5 to 10-membered heterocyclic C1-6 alkylamino group, di-C1-6 alkylamino group, N-C3-9 alkenyl-N-C1-8 alkylamino group, N-C3-9 alkynyl-N-C1-8 alkylamino group, N-C3-8 cycloalkyl-N-C1-8 alkylamino group, N-C6-10 aryl-N-C1-8 alkylamino group, N-C3-8 cycloalkyl C1-6 alkyl-N-C1-8 alkylamino group, N-C6-10 aryl C1-6 alkyl-N-C1-8 alkylamino group, N-5 to 10-membered heterocyclic C1-6 alkyl-N-C1-8 alkylamino group, C6-10 aryloxy C1-6 alkyl group and 5 to 10-membered heterocyclic oxy C1-6 alkyl group
(with the proviso that each group in the substituent group a-2 has 1 to 3 members selected from the following substituent group b-1):
[Substituent Group b-1]
halogen atom, hydroxyl group, mercapto group, cyano group, amino group, nitro group, C1-6 alkyl group, C3-8 cycloalkyl group, C6-10 aryl group, 5 to 10-membered heterocyclic group, C1-6 alkoxy group, C3-8 cycloalkoxy group, C6-10 aryloxy group, C6-10 arylthio group, C6-10 arylamino group, 5 to 10-membered heterocyclic oxy group, C1-6 alkylthio group, C1-6 alkylsulfonyl group, monofluoromethyl group, difluoromethyl group, trifluoromethyl group, monofluoromethoxy group, difluoromethoxy group, trifluoromethoxy group, mono-C1-6 alkylamino group, di-C1-6 alkylamino group; mono-C6-10 arylamino group optionally having one amino group or aminosulfonyl group and N-C6-10 aryl C1-6 alkyl-N-C1-6 alkylamino group optionally having one amino group.

2. The agricultural composition according to Claim 1 wherein, in the formula (I),
R¹ is a hydrogen atom or amino group;
R² is a hydrogen atom, halogen atom, amino group, C1 to 6 alkyl group, C1 to 6 alkoxy group, C1 to 6 alkoxy C1 to 6 alkyl group, hydroxyl C1 to 6 alkyl group, cyano C1 to 6 alkyl group, R⁵-NH- (R⁵ means a C1 to 6 alkyl group, hydroxyl C1 to 6 alkyl group, C1 to 6 alkoxy C1 to 6 alkyl group or C1 to 6 alkoxy carbonyl C1 to 6 alkyl group) or R⁶-(C=O)NH (R⁶ means a C1 to 6 alkyl group or C1 to 6 alkoxy C1 to 6 alkyl group);
R³ is a hydrogen atom or halogen atom;
R⁴ is a hydrogen atom or halogen atom;
G is any of G¹ to G¹² optionally substituted by one or two halogen atoms or C1 to 3 alkyl groups (for G¹ to G¹¹ and G¹², its left connecting bond is connected to a pyridine ring and its right connecting bond is connected to a methylene group.); 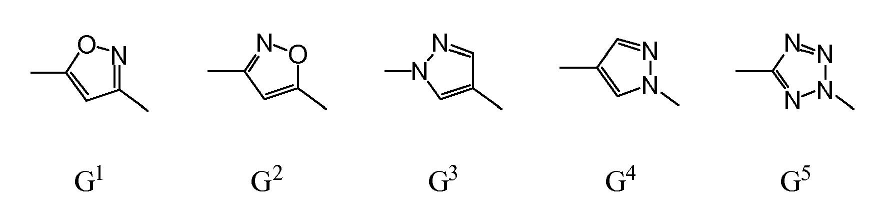 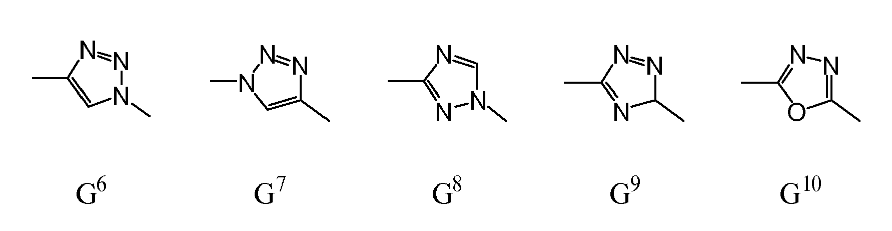 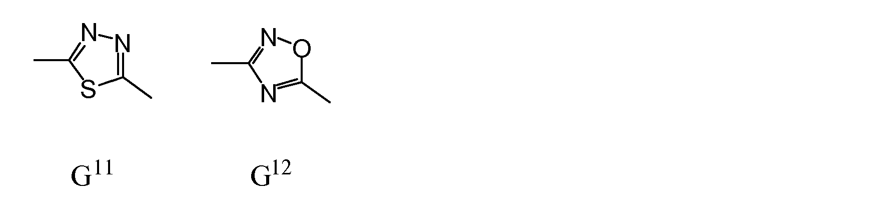 E is a furyl group optionally having 1 or 2 identical or mutually different members selected from the following substituent group a-3 and substituent group a-4, a thienyl group optionally having 1 or 2 identical or mutually different members selected from the substituent group a-3 and substituent group a-4, a pyrrolyl group optionally having 1 or 2 identical or mutually different members selected from the substituent group a-3 and substituent group a-4, a tetrazolyl group optionally having one identical or mutually different member selected from the substituent group a-3 and substituent group a-4, a thiazolyl group optionally having 1 or 2 identical or mutually different members selected from the substituent group a-3 and substituent group a-4, a pyrazolyl group optionally having 1 or 2 identical or mutually different members selected from the substituent group a-3 and substituent group a-4, a pyridyl group optionally having 1 or 4 identical or mutually different members selected from the substituent group a-3 and substituent group a-4 or a phenyl group optionally having 1 or 5 identical or mutually different members selected from the substituent group a-3 and substituent group a-4:
[Substituent Group a-3]
halogen atom, amino group, hydroxyl group, mercapto group, cyano group, formyl group, carboxyl group, C1-8 alkyl group, C2-9 alkenyl group, C2-9 alkynyl group, C3-8 cycloalkyl group, C6-10 aryl group, 5 to 10-membered heterocyclic group, C3-8 cycloalkyl C1-6 alkyl group, C3-8 cycloalkylidene C1-6 alkyl group, C6-10 aryl C1-6 alkyl group, C6-10 aryl C2-6 alkenyl group, 5 to 10-membered heterocyclic C1-6 alkyl group, C1-8 alkoxy group, C3-9 alkenyloxy group, C3-9 alkynyloxy group, C3-8 cycloalkoxy group, C6-10 aryloxy group, 5 to 10-membered heterocyclic oxy group, C3-8 cycloalkyl C1-6 alkoxy group, C6-10 aryl C1-6 alkoxy group, 5 to 10-membered heterocyclic C1-6 alkoxy group, C1-8 alkylthio group, C3-9 alkenylthio group, C3-9 alkynylthio group, C3-8 cycloalkylthio group, C6-10 arylthio group, C3-8 cycloalkyl C1-6 alkylthio group , C6-10 aryl C1-6 alkylthio group, 5 to 10-membered heterocyclic C1-6 alkylthio group, mono-C1-8 alkylamino group, mono-C3-9 alkenylamino group, mono-C3-9 alkynylamino group, mono-C3-8 cycloalkylamino group, mono-C6-10 arylamino group, mono-C3-8 cycloalkyl C1-6 alkylamino group, mono-C6-10 aryl C1-6 alkylamino group, mono-5 to 10-membered heterocyclic C1-6 alkylamino group, di-C1-6 alkylamino group, N-C3-9 alkenyl-N-C1-8 alkylamino group, N-C3-9 alkynyl-N-C1-8 alkylamino group, N-C3-8 cycloalkyl-N-C1-8 alkylamino group, N-C6-10 aryl-N-C1-8 alkylamino group, N-C3-8 cycloalkyl C1-6 alkyl-N-C1-8 alkylamino group, N-C6-10 aryl C1-6 alkyl-N-C1-8 alkylamino group, N-5 to 10-membered heterocyclic C1-6 alkyl-N-C1-8 alkylamino group, C1-8 alkylcarbonyl group, C6-10 aryl Carbonyl group, C1-8 alkylsulfonyl group, C1-8 alkylsulfinyl group, C6-10 aryloxy C1-6 alkyl group, 5 to 10-membered heterocyclic oxy C1-6 alkyl group and C1-3 trialkylsilyl C2-9 alkynyl group; and
[Substituent Group a-4]
C1-8 alkyl group, C2-9 alkenyl group, C2-9 alkynyl group, C3-8 cycloalkyl group, C6-10 aryl group, 5 to 10-membered heterocyclic group, C3-8 cycloalkyl C1-6 alkyl group, C6-10 aryl C1-6 alkyl group, 5 to 10-membered heterocyclic C1-6 alkyl group, C1-8 alkoxy group, C3-9 alkenyloxy group, C3-9 alkynyloxy group, C3-8 cycloalkoxy group, C6-10 aryloxy group, 5 to 10-membered heterocyclic oxy group, C3-8 cycloalkyl C1-6 alkoxy group, C6-10 aryl C1-6 alkoxy group, 5 to 10-membered heterocyclic C1-6 alkoxy group, C1-8 alkylthio group, C3-9 alkenylthio group, C3-9 alkynylthio group, C3-8 cycloalkylthio group, C6-10 arylthio group, C3-8 cycloalkyl C1-6 alkylthio group , C6-10 aryl C1-6 alkylthio group, 5 to 10-membered heterocyclic C1-6 alkylthio group, mono-C1-8 alkylamino group, mono-C3-9 alkenylamino group, mono-C3-9 alkynylamino group, mono-C3-8 cycloalkylamino group, mono-C6-10 arylamino group, mono-C3-8 cycloalkyl C1-6 alkylamino group, mono-C6-10 aryl C1-6 alkylamino group, mono-5 to 10-membered heterocyclic C1-6 alkylamino group, di-C1-6 alkylamino group, N-C3-9 alkenyl-N-C1-8 alkylamino group, N-C3-9 alkynyl-N-C1-8 alkylamino group, N-C3-8 cycloalkyl-N-C1-8 alkylamino group, N-C6-10 aryl-N-C1-8 alkylamino group, N-C3-8 cycloalkyl C1-6 alkyl-N-C1-8 alkylamino group, N-C6-10 aryl C1-6 alkyl-N-C1-8 alkylamino group, N-5 to 10-membered heterocyclic C1-6 alkyl-N-C1-8 alkylamino group, C6-10 aryloxy C1-6 alkyl group and 5 to 10-membered heterocyclic oxy C1-6 alkyl group
(with the proviso that each group in the substituent group a-2 has 1 to 3 members selected from the following substituent group b-2):
[Substituent Group b-2]
halogen atom, hydroxyl group, mercapto group, cyano group, amino group, nitro group, C1-6 alkyl group, C3-8 cycloalkyl group, C6-10 aryl group, 5 to 10-membered heterocyclic group, C1-6 alkoxy group, C6-10 aryloxy group, 5 to 10-membered heterocyclic oxy group, C1-6 alkylthio group, C1-6 alkylsulfonyl group, monofluoromethyl group, difluoromethyl group, trifluoromethyl group, monofluoromethoxy group, difluoromethoxy group, trifluoromethoxy group, mono-C1-6 alkylamino group, di-C1-6 alkylamino group; mono-C6-10 arylamino group optionally having one amino group or aminosulfonyl group and N-C6-10 aryl C1-6 alkyl-N-C1-6 alkylamino group optionally having one amino group.

3. The agricultural composition according to Claim 1 or 2 wherein, in the formula (I), G is any of G¹ to G⁴.

4. The agricultural composition according to Claim 1 or 2 wherein, in the formula (I), G is any of G⁵ to G¹².

5. The agricultural composition according to any one of Claims 1 to 4 wherein, in the formula (I),
R¹ is an amino group;
R² is a hydrogen atom, halogen atom, amino group, C1 to 6 alkyl group, C1 to 6 alkoxy group, C1 to 6 alkoxy C1 to 6 alkyl group, hydroxyl C1 to 6 alkyl group, cyano C1 to 6 alkyl group, R⁵-NH- (R⁵ means a C1 to 6 alkyl group, hydroxyl C1 to 6 alkyl group, C1 to 6 alkoxy C1 to 6 alkyl group or C1 to 6 alkoxycarbonyl C1 to 6 alkyl group) or R⁶-(C=O)NH (R⁶ means a C1 to 6 alkyl group or C1 to 6 alkoxy C1 to 6 alkyl group); and
each of R³ and R⁴ is a hydrogen atom.

6. The agricultural composition according to any one of Claims 1 to 4 wherein, in the formula (I),
R¹ is an amino group; and
each of R², R³ and R⁴ is a hydrogen atom.

7. The agricultural composition according to any one of Claims 1 to 6 wherein, in the formula (I),
E is a furyl group optionally having 1 or 2 identical or mutually different members selected from the following substituent group a-5, a thienyl group optionally having 1 or 2 identical or mutually different members selected from the substituent group a-5, a pyridyl group optionally having 1 or 4 identical or mutually different members selected from the substituent group a-5 or a phenyl group optionally having 1 or 5 identical or mutually different members selected from the substituent group a-5:
[Substituent Group a-5]
halogen atom; cyano group; C1-8 alkyl group; C2-9 alkenyl group; C2-9 alkynyl group; C3-8 cycloalkyl group; phenyl group; 5 to 10-membered heterocyclic group; C3-8 cycloalkyl C1-6 alkyl group; C3-8 cycloalkylidene C1-6 alkyl group; phenyl C1-6 alkyl group; 5 to 10-membered heterocyclic C1-6 alkyl group; phenyl C2-6 alkenyl group; 5 to 10-membered heterocyclic C2-6 alkenyl group; C1-8 alkoxy group; C3-9 alkenyloxy group; C3-9 alkynyloxy group; C3-8 cycloalkoxy group; phenoxy group; 5 to 10-membered heterocyclic oxy group; C3-8 cycloalkyl C1-6 alkoxy group; C3-8 cycloalkylidene C1-6 alkoxy group; phenyl C1-6 alkoxy group; 5 to 10-membered heterocyclic C1-6 alkoxy group; phenoxy C1-6 alkyl group; 5 to 10-membered heterocyclic oxy C1-6 alkyl group; C1-3 trialkylsilyl C2-9 alkynyl group; C1-8 alkyl group substituted by a member selected from the group consisting of halogen atom, cyano group, C1-6 alkoxy group, monofluoromethyl group, difluoromethyl group, trifluoromethyl group, monofluoromethoxy group, difluoromethoxy group and trifluoromethoxy group; C2-9 alkenyl group substituted by a member selected from the group consisting of halogen atom, cyano group, C1-6 alkoxy group, monofluoromethyl group, difluoromethyl group, trifluoromethyl group, monofluoromethoxy group, difluoromethoxy group and trifluoromethoxy group; C2-9 alkynyl group substituted by a member selected from the group consisting of halogen atom, cyano group, C1-6 alkoxy group, monofluoromethyl group, difluoromethyl group, trifluoromethyl group, monofluoromethoxy group, difluoromethoxy group and trifluoromethoxy group; C3-8 cycloalkyl group substituted by a member selected from the group consisting of halogen atom, cyano group, C1-6 alkoxy group, monofluoromethyl group, difluoromethyl group, trifluoromethyl group, monofluoromethoxy group, difluoromethoxy group and trifluoromethoxy group; phenyl group substituted by a selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; 5 to 10-membered heterocyclic group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; C3-8 cycloalkyl C1-6 alkyl group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; C3-8 cycloalkylidene C1-6 alkyl group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; phenyl C1-6 alkyl group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; 5 to 10-membered heterocyclic C1-6 alkyl group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; phenyl C2-6 alkenyl group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; 5 to 10-membered heterocyclic C2-6 alkenyl group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; C1-8 alkoxy group substituted by a member selected from the group consisting of halogen atom, cyano group, C1-6 alkoxy group, monofluoromethyl group, difluoromethyl group, trifluoromethyl group, monofluoromethoxy group, difluoromethoxy group and trifluoromethoxy group; C3-9 alkenyloxy group substituted by a member selected from the group consisting of halogen atom, cyano group, C1-6 alkoxy group, monofluoromethyl group, difluoromethyl group, trifluoromethyl group, monofluoromethoxy group, difluoromethoxy group and trifluoromethoxy group; C3-9 alkynyloxy group substituted by a member selected from the group consisting of halogen atom, cyano group, C1-6 alkoxy group, monofluoromethyl group, difluoromethyl group, trifluoromethyl group, monofluoromethoxy group, difluoromethoxy group and trifluoromethoxy group; C3-8 cycloalkoxy group substituted by a member selected from the group consisting of halogen atom, cyano group, C1-6 alkoxy group, monofluoromethyl group, difluoromethyl group, trifluoromethyl group, monofluoromethoxy group, difluoromethoxy group and trifluoromethoxy group; phenoxy group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; 5 to 10-membered heterocyclic oxy group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; C3-8 cycloalkyl C1-6 alkoxy group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; C3-8 cycloalkylidene C1-6 alkoxy group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; phenyl C1-6 alkoxy group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; 5 to 10-membered heterocyclic C1-6 alkoxy group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; phenoxy C1-6 alkyl group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group; and 5 to 10-membered heterocyclic oxy C1-6 alkyl group substituted by a member selected from the group consisting of halogen atom, cyano group, C1-6 alkyl group, C1-6 alkoxy group, monofluoromethyl group, difluoromethyl group, trifluoromethyl group, monofluoromethoxy group, difluoromethoxy group and trifluoromethoxy group.

8. The agricultural composition according to any one of Claims 1 to 7 wherein the plant pathogen is a plant pathogenic filamentous fungus (excluding Aspergillus).

9. A method of controlling or preventing a disease due to a plant pathogen (excluding Aspergillus) comprising treating a useful crop with an effective amount of the agricultural composition according to any one of Claims 1 to 7.

10. The method according to Claim 9 wherein the treatment method is a foliar treatment, soil treatment or seed treatment.

11. Use of a compound represented by the formula (I) or a salt of the compound or a hydrate thereof, for producing the agricultural composition according to Claim 1.
